# EUROPEAN PATENT APPLICATION

(11) **EP 0 696 593 A2**
(43) Date of publication of application: **14.02.1996**
(21) Application number: 95112535.0
(22) Date of filing: 09.08.1995
(51) Int. Cl.: C07K 5/08, C07K 5/06, C07D 207/10, C07D 401/12, C07C 321/22

(54) **Inhibitors of farnesyl protein transferase**

(30) Priority: 11.08.1994 US 289476
(71) Applicant: BRISTOL-MYERS SQUIBB COMPANY, Princeton, NJ 08543-4000 (US)
(72) Inventor: Leftheris, Katerina, Skillman, NJ (US)
(74) Representative: VOSSIUS & PARTNER

(57) **Abstract**

Inhibition of farnesyl transferase, which is an enzyme involved in ras oncogene expression, is effected by compounds of the formula 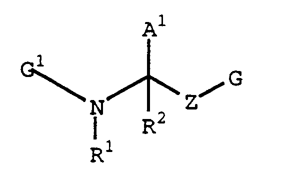
their enantiomers, diastereomers, and pharmaceutically acceptable salts, prodrugs, and solvates, wherein:
G is 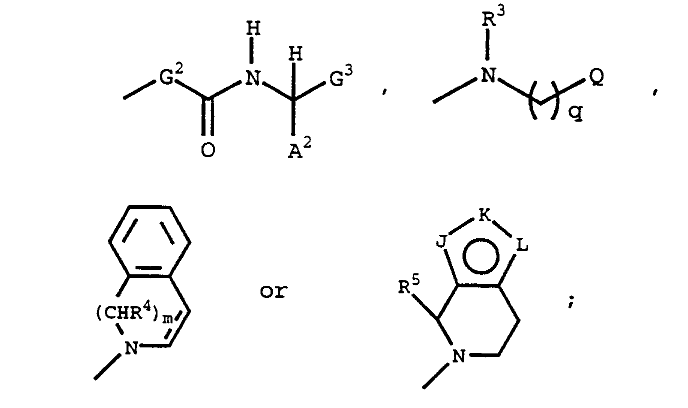
when G is 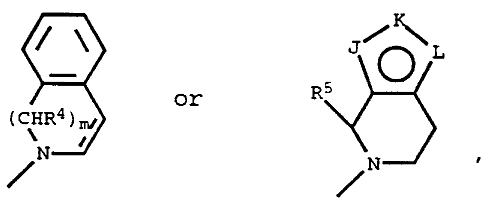
it is optionally substituted, at any available position or positions, with halo, alkyl or substituted alkyl having 1 to 20 carbon atoms, alkoxy, or a combination of these groups;
G¹ is 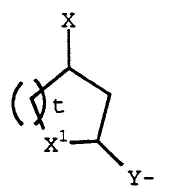
optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combination of these groups;
G² is 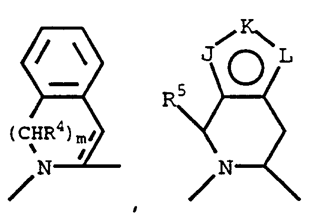
or -NR⁶-CH(Q¹)-;
J, K and L are each, independently, N, NR⁷, O, S or CR⁶ with the provisos that only one of the groups J, K and L can be O or S, and at least one of the groups J or L must be N, NR⁷, O or S to form a fused five-membered heteroring; the bond between J and K or K and L may also form one side of a phenyl ring fused to the fused five-membered heteroring;
Q is is alkyl, cycloalkyl, substituted alkyl, aryl, pyrrolyl, furyl, thiophenyl, imidazolyl, oxazolyl, thiazolyl, pyrazolyl, pyrrolidyl or pyridyl;
Q¹, A¹ and A² are each, independently, H, alkyl, substituted alkyl, phenyl or substituted phenyl;
G³ is R⁸, -C(O)OR⁸, -C(O)NR⁸R⁹, -C(O)N(R¹⁰)OR⁸, -C(O)NHSO₂R¹¹ or -CH₂OR⁸;
X is -SH, -OH or -NHR¹²;
X¹ is -NR¹³-, -CH₂- or -CH(NHR¹⁴)-;
Y and Z are each, independently, -CH₂- or -C(O)-;
R¹ - R¹⁴ are each, independently, H or alkyl having 1 to 20 carbon atoms;
R³ may also be substituted alkyl or cycloalkyl; R⁴, R⁵ and R¹¹ may also be aryl or aralkyl; R⁷, R⁸, R⁹ and R¹⁰ may also be aralkyl; and R¹², R¹³ and R¹⁴ may also be substituted alkyl or aralkyl;
m is 0 or an integer from 1 to 2;
q is 0 or an integer from 1 to 3;
t is an integer from 1 to 2; and
the dotted line represents an optional double bond.

## Description

This invention relates to compounds that inhibit farnesyl-protein transferase and Ras protein farnesylation, thereby making them useful as anti-cancer agents. The compounds are also useful in the treatment of diseases, other than cancer, associated with signal transduction pathways operating through Ras and those associated with CAAX-containing proteins other than Ras that are also post-translationally modified by the enzyme farnesyl protein transferase. The compounds may also act as inhibitors of other prenyl transferases, and thus be effective in the treatment of diseases associated with other prenyl modifications of proteins.

The mammalian ras gene family comprises three genes: H-ras, K-ras and N-ras. The Ras proteins are a family of GTP-binding and hydrolyzing proteins that regulate cell growth and differentiation. Overproduction of normal Ras proteins or mutations that inhibit their GTPase activity can lead to uncontrolled cell division.

The transforming activity of Ras is dependent upon localization of the protein to plasma membranes. This membrane binding occurs via a series of post-translational modifications of the cytosolic Ras proteins. The first and mandatory step in this sequence of events is the farnesylation of these proteins. The reaction is catalyzed by the enzyme farnesyl protein transferase (FPT), and farnesyl pyrophosphate (FPP) serves as the farnesyl group donor in this reaction. The Ras C-terminus contains a sequence motif termed a "Cys-Aaa₁-Aaa₂-Xaa" box (CAAX box), wherein Cys is cysteine, Aaa is an aliphatic amino acid, and Xaa is a serine or methionine. Farnesylation occurs on the cysteinyl residue of the CAAX box (Cys-186), thereby attaching the prenyl group on the protein via a thio-ether linkage.

In accordance with the present invention, a compound of the formula 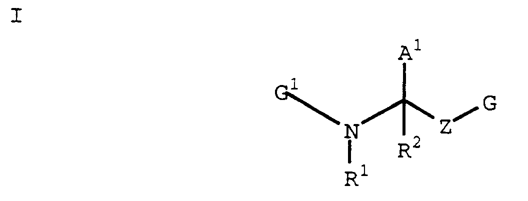
its enantiomers and diastereomers, and pharmaceutically acceptable salts, prodrugs and solvates thereof inhibit S-farnesyl protein transferase, which is an enzyme involved in Ras oncogene function. In formula I and throughout this specification, unless otherwise specified, the above symbols are defined as follows:
G is 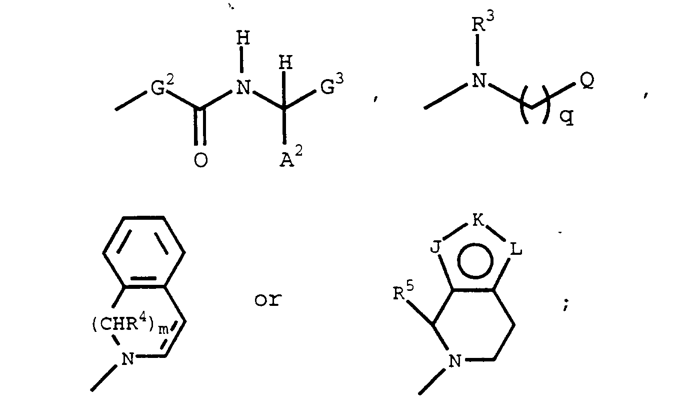
when G is 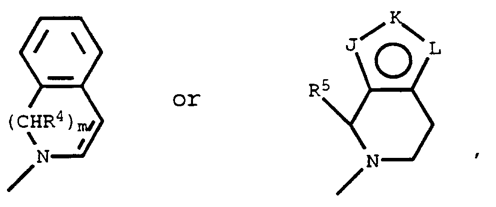
it is optionally substituted, at any available position or positions, with halo, alkyl or substituted alkyl having 1 to 20 carbon atoms, alkoxy, or a combination of these groups;
G¹ is 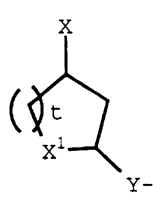
optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, alkoxycarbonyl, phenyl, substituted phenyl, or a combination of these groups;
G² is 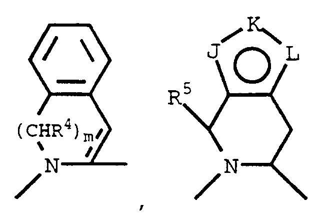
or -NR⁶-CH(Q¹)-;
J, K and L are each, independently, N, NR⁷, O, S or CR⁶ with the provisos that only one of the groups J, K and L can be O or S, and at least one of the groups J or L must be N, NR⁷, O or S to form a fused five-membered heteroring; the bond between J and K or K and L may also form one side of a phenyl ring fused to the fused five-membered heteroring;
Q is alkyl, cycloalkyl, substituted alkyl, aryl, pyrrolyl, furyl, thiophenyl, imidazolyl, oxazolyl, thiazolyl, pyrazolyl, pyrrolidyl or pyridyl;
Q¹, A¹ and A² are each, independently, H, alkyl, substituted alkyl, phenyl or substituted phenyl;
G³ is R⁸, -C(O)OR⁸, -C(O)NR⁸R⁹, -C(O)N(R¹⁰)OR⁸, -C(O)NHSO₂R¹¹ or -CH₂OR⁸;
X is -SH, -OH or -NHR¹²;
X¹ is -NR¹³-, -CH₂- or -CH(NHR¹⁴)-;
Y and Z are each, independently, -CH₂- or -C(O)-;
R¹ - R¹⁴ are each, independently, H or alkyl having 1 to 20 carbon atoms;
R³ may also be substituted alkyl or cycloalkyl; R⁴, R⁵ and R¹¹ may also be aryl or aralkyl; R⁷, R⁸, R⁹ and R¹⁰ may also be aralkyl; and R¹², R¹³ and R¹⁴ may also be substituted alkyl or aralkyl;
m is 0 or an integer from 1 to 2;
q is 0 or an integer from 1 to 3;
t is an integer from 1 to 2; and
the dotted line represents an optional double bond.

Listed below are definitions of various terms used to describe this invention. These definitions apply to the terms as they are used throughout this specification, unless otherwise limited in specific instances, either individually or as part of a larger group.

The term "alkyl" refers to straight or branched chain unsubstituted hydrocarbon groups of 1 to 20 carbon atoms, preferably 1 to 7 carbon atoms. The expression "lower alkyl" refers to unsubstituted alkyl groups of 1 to 4 carbon atoms.

The expression "substituted alkyl" refers to an alkyl group substituted by, for example, one to four substituents such as halo, hydroxy, alkoxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, alkoxycarbonyl, phenyl, substituted phenyl, guanidino, indolyl, imidazolyl, furyl, thienyl, thiazolyl, pyrrolidyl, pyridyl, pyrimidyl and the like.

The term "cycloalkyl" refers to cyclic hydrocarbon groups of 3 to 8 carbon atoms.

The term "halogen" or "halo" refers to fluorine, chlorine, bromine and iodine.

The term "alkoxy" refers to alkyl-O-.

The term "alkanoyl" refers to alkyl-C(O)-.

The term "alkanoyloxy" refers to alkyl-C(O)-O-.

The terms "alkylamino" and "dialkylamino" refer to (alkyl)NH- and (alkyl)₂N-, respectively.

The term "alkanoylamino" refers to alkyl-C(O)-NH-.

The term "alkylthio" refers to alkyl-S-.

The term "alkylthiono" refers to alkyl-S(O)-.

The term "alkylsulfonyl" refers to alkyl-S(O)₂-.

The term "carbamyl" refers to -C(O)NH₂.

The term "alkoxycarbonyl" refers to alkyl-O-C(O)-.

The term "aryl" refers to monocyclic or bicyclic aromatic hydrocarbon groups having 6 to 12 carbon atoms in the ring portion, such as phenyl, naphthyl, tetrahydronapthyl, biphenyl and diphenyl groups, each of which may be substituted.

The term "aralkyl" refers to an aryl group bonded directly through an alkyl group, such as benzyl.

The term "substituted phenyl" refers to a phenyl group substituted by, for example, one to four substituents such as alkyl, cycloalkyl, halo, trifluoromethyl, hydroxy, alkoxy, alkanoyl, alkanoyloxy, amino, alkylamino, dialkylamino, alkanoylamino, thiol, alkylthio, nitro, cyano, carboxy, carbamyl, alkoxycarbonyl, alkylthiono, alkylsulfonyl, sulfonamido and the like.

The expression "substituted carbamyl" refers to a carbamyl group N-substituted by, for example, one to two substituents such as hydroxy, alkyl, phenyl and the like, and when the substitutions are alkyl, they may be taken together to form a four-to 8-membered saturated, unsaturated or aromatic ring with the nitrogen atom to which they are attached.

Throughout this specification, groups and substituents thereof may be chosen to provide stable moieties and compounds.

The compounds of formula I form salts which are also within the scope of this invention. Pharmaceutically acceptable (i.e., non-toxic, physiologically acceptable) salts are preferred, although other salts are also useful, e.g., in isolating or purifying the compounds of this invention.

The compounds of formula I may form salts with alkali metals such as sodium, potassium and lithium, with alkaline earth metals such as calcium and magnesium, with organic bases such as dicyclohexylamine, tributylamine, pyridine and amino acids such as arginine, lysine and the like. Such salts may be obtained, for example, by exchanging the carboxylic acid protons, if they contain a carboxylic acid, in compound I with the desired ion in a medium in which the salt precipitates or in an aqueous medium followed by evaporation. Other salts can be formed as known to those skilled in the art.

The compounds of formula I may form salts with a variety of organic and inorganic acids. Such salts include those formed with hydrogen chloride, hydrogen bromide, methanesulfonic acid, sulfuric acid, acetic acid, trifluoroacetic acid, maleic acid, benzenesulfonic acid, toluenesulfonic acid and various others (e.g., nitrates, phosphates, borates, tartrates, citrates, succinates, benzoates, ascorbates, salicylates and the like). Such salts may be formed by reacting compound I in an equivalent amount of the acid in a medium in which the salt precipitates or in an aqueous medium followed by evaporation.

In addition, zwitterions ("inner salts") may be formed.

A compound of the formula I may also have prodrug forms. Any compound that will be converted in vivo to provide the bioactive agent (i.e., the compound of formula I) is a prodrug within the scope and spirit of the invention. For example, compound I may be in the form of a prodrug having the formula 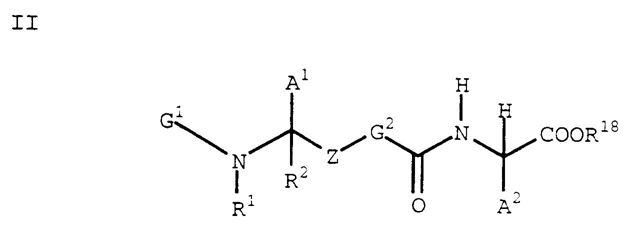
wherein R¹⁵ is:
lower alkyl, such as methyl, ethyl and the like;
substituted lower alkyl, such as 2-(N-morpholine)ethyl and the like;
lower aralkyl, such as benzyl, biphenylmethyl and the like;
(acyloxy)alkyl, such as (pivalyloxy)methyl, 1-(propanoyloxy)-2-methyl-1-propyl and the like;
(aminoacyloxy)aroyloxyalkyl, such as para-glycyloxybenzoyloxymethyl and the like;
(aminoalkoxy)aroyloxyalkyl, such as para-2-[(N-morpholine)ethoxy]benzoyloxymethyl and the like;
substituted amides, such as N,N-di(2-hydroxyethyl)acetamido, 4-methylpiperazine-1-acetyl, 4-(2-hydroxyethyl)piperazine-1-acetyl and the like; or
a dioxolanemethyl, such as (5-methyl-2-oxo-1,3-dioxolan-4-yl)methyl and the like.

Further, for a compound of formula I where A² is substituted alkyl of the formula -(CH₂)_{w}OH (where w is 2 or 3), and G³ is -C(O)OH A² may be joined with the carboxyl group to form a lactone ring which can be opened in vivo to give a compound of formula I where A² is -(CH₂)_{w}OH (again where w is 2 or 3).

Various forms of prodrugs are well known in the art. For examples of such prodrug derivatives, see:
a) Design of Prodrugs, edited by H. Bundgaard, (Elsevier, 1985) and Methods in Enzymology, Vol. 42, p. 309-396, edited by K. Widder, *et al*. (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, *et al*., Journal of Pharmaceutical Sciences, 77, 285 (1988); and
e) N. Kakeya, *et al*., Chem Pharm Bull, 32, 692 (1984).

It should further be understood that solvates (e.g., hydrates) of the compounds of formula I are also within the scope of the present invention. Methods of solvation are generally known in the art. Similarly, enantiomers and diastereomers of the compounds of formula I are within the scope of the present invention.

### Preferred Moieties

For compounds of the formula I, the following moieties are preferred:
G¹ is 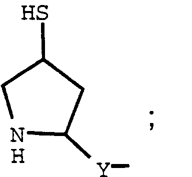
G² is 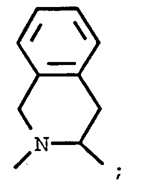
A¹ and A² are each, independently, H or D-, L- or D,L- -CH₃, -CH(CH₃)₂, -CH₂CH(CH₃)₂, -CH(CH₃)CH₂CH₃, -C(CH₃)₃, -CH₂OH, -CH₂CH₂OH, -CH₂CH₂CH₂OH, -CH(OH)CH₃, 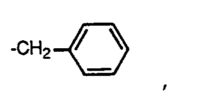 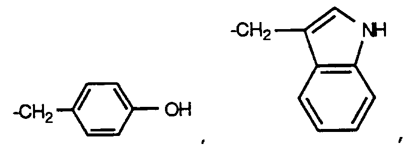
-CH₂CH₂CH₂CH₂NH₂, 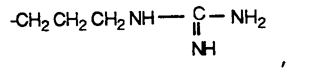
-CH₂C(O)OH, -CH₂CH₂C(O)OH, -CH₂C(O)NH₂, -CH₂CH₂C(O)NH₂, -CH₂CH₂OCH₃, -CH₂CH₂CONR¹⁵R¹⁶, -CH₂SH, -CH₂CH₂SH, -CH₂CH₂SOCH₃, CH₂CH₂SO₂CH₃ or -CH₂CH₂SCH₃, where R¹⁵ and R¹⁶ are each, independently, hydrogen or alkyl, or R¹⁵ and R¹⁶, taken together, form a 5- to 7-membered saturated ring with the N-atom to which they are attached; and
G³ is -C(O)OH, -C(O)OR⁸ or -C(O)NHSO₂R¹¹.

When G² is 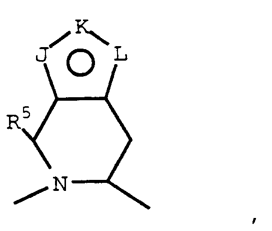 the fused five-membered(substituted) heteroring is 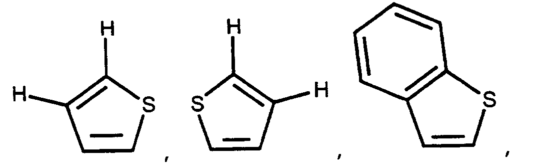
preferably 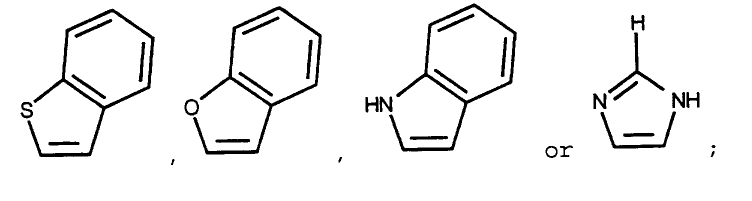
and when G² is -NR⁶-CH(Q¹)-, Q¹ is preferably D-, L- or D,L- 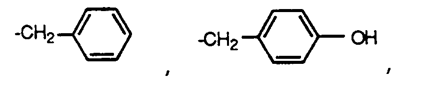
or

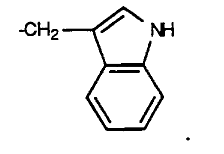

The following moieties are particularly preferred:
G¹ is 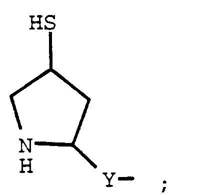
G² is 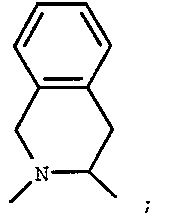
G³ is -C(O)OR⁸ or -C(O)NHSO₂R¹¹; A¹ is L- -CH₃, -CH(CH₃)₂, -CH₂CH(CH₃)₂, -C(CH₃)₃, 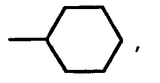
-CH(CH₃)CH₂CH₃, -CH₂OH or -CH(OH)CH₃;
A² is L- -CH₂CH₂SCH₃, -CH₂CH₂OH, -CH₂CH₂CH₂OH, -CH₂CH₂OCH₃, -CH₂CH₂CONR¹²R¹³, -CH₂CH₂SH, -CH₂CH₂SOCH₃ or -CH₂CH₂SO₂CH₃; and
R¹ and R² are H.

### Use and Utility

The compounds of formula I are inhibitors of S-farnesyl protein transferase. They are thus useful in the treatment of a variety of cancers, including (but not limited to) the following:
- carcinoma, including that of the bladder, breast, colon, kidney, liver, lung, ovary, pancreas, stomach, cervix, thyroid and skin;
- hematopoietic tumors of lymphoid lineage, including acute lymphocytic leukemia, B-cell lymphoma and Burketts lymphoma;
- hematopoietic tumors of myeloid lineage, including acute and chronic myelogenous leukemias and promyelocytic leukemia;
- tumors of mesenchymal origin, including fibrosarcoma and rhabdomyosarcoma; and
- other tumors, including melanoma, seminoma, tetratocarcinoma, neuroblastoma and glioma.

The compounds of formula I are especially useful in treatment of tumors having a high incidence of Ras involvement, such as colon, lung, and pancreatic tumors. By the administration of a composition having one (or a combination) of the compounds of this invention, development of tumors in a mammalian host is reduced.

Compounds of formula I may also be useful in the treatment of diseases other than cancer that may be associated with signal transduction pathways operating through Ras, e.g., neuro-fibromatosis.

Compounds of formula I may also be useful in the treatment of diseases associated with CAAX-containing proteins other than Ras (e.g., nuclear lamins and transducin) that are also post-translationally modified by the enzyme farnesyl protein transferase.

Compounds of formula I may also act as inhibitors of other prenyl transferases (e.g., geranylgeranyl transferase), and thus be effective in the treatment of diseases associated with other prenyl modifications (e.g., geranylgeranylation) of proteins (e.g., the rap, rab, rac and rho gene products and the like). For example, they may find use as drugs against Hepatitis delta virus (HDV) infections, as suggested by the recent finding that geranylgeranylation of the large isoform of the delta antigen of HDV is a requirement for productive viral infection [J. S. Glenn, *et al*., Science, 256, 1331 (1992)].

The compounds of this invention may also be useful in combination with known anti-cancer and cytotoxic agents. If formulated as a fixed dose, such combination products employ the compounds of this invention within the dosage range described below and the other pharmaceutically active agent within its approved dosage range. Compounds of formula I may be used sequentially with known anticancer or cytotoxic agents when a combination formulation is inappropriate.

The compounds of this invention may be formulated with a pharmaceutical vehicle or diluent for oral, intravenous or subcutaneous administration. The pharmaceutical composition can be formulated in a classical manner using solid or liquid vehicles, diluents and additives appropriate to the desired mode of administration. Orally, the compounds can be administered in the form of tablets, capsules, granules, powders and the like. These compounds may be administered in a dosage range of about 0.05 to 50 mg/kg/day, preferably less than 50 mg/kg/day, in a single dose or in 2 to 4 divided doses.

### Process of Preparation

A compound of formula III below, wherein Prot¹ is an amine protecting group (e.g., *t*-butyloxycarbonyl (Boc), benzyloxycarbonyl (Cbz) and the like): 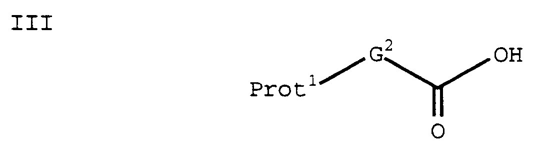
can be coupled with a carboxylic acid protected derivative of an amino acid of formula IV: 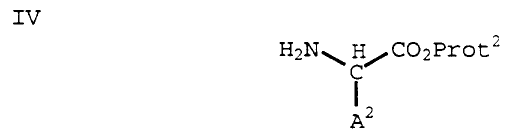
to form a compound of formula V below, wherein Prot² is a carboxylic acid protecting group (e.g., alkyl, benzyl, p-methoxybenzyl and the like): 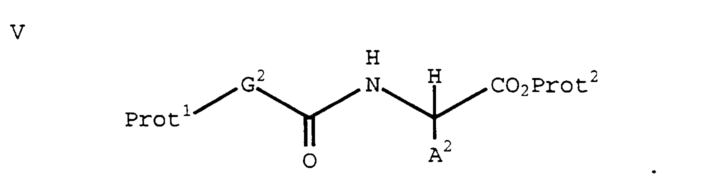
For additional examples of amino and carboxylic acid protecting groups (as well as means of formation and eventual deprotection), see T.W. Greene and P.G.M. Wuts, "Protective Groups in Organic Synthesis", Second Edition, John Wiley & Sons, New York, 1991.

A variety of coupling agents may be used for this coupling, including 1-(3-dimethylaminopropyl)-3-ethylcarbodiimide hydrochloride (EDCI) with 1-hydroxybenzotriazole (HOBT), dicyclohexyl-carbodiimide (DCC) with HOBT, benzotriazol-1-yloxytris(dimethylamino)phosphonium hexafluoro-phosphate (BOP) with or without HOBT, carbonyldiimidazole (CDI), bis(2-oxo-3-oxazolidinyl)phosphinic chloride (BOP chloride), isopropylchloroformate (IPCF) and the like.

Compounds of the formula IV are known in the art. See, for example, R. M. Williams, "Synthesis of Optically Active α-Amino Acids," Pergamon Press, Oxford, 1989.

Compounds of formula III can be prepared by methods known in the art. For example, see J. P. Maffrand, U.S. Patent No. 4,147,787 issued April 3, 1979; H. Kawakubo, *et al*., J. Med. Chem., 33, 3110 (1990); D. G. Harvey, *et al*., J. Chem Soc., 153 (1941); A. Brossi, *et al*., J. Med. Chem. 16, 418, (1973); M. Cain, *et al*., Heterocycles, 19, 1003 (1982); S. Ueki, *et al*., U.S. Patent No. 5,126,448 issued June 30, 1992; J. W. Skiles, *et al*., J. Med. Chem., 29, 784 (1986); V. Schollkopf, *et al*., Angew. Chem. Int. Ed. Engl., 26, 143 (1987); I. Huber and D. Seebach, Helvitica Chim Acta, 70, 1944 (1987); J. L. Stanton, *et al*., J. Med. Chem., 26, 1267 (1983); and M. Okada, *et al*., JP 02,193,971, 31 July 1990.

A compound of formula V can be treated with a suitable N-deprotecting agent to provide the corresponding free amine of formula VI: 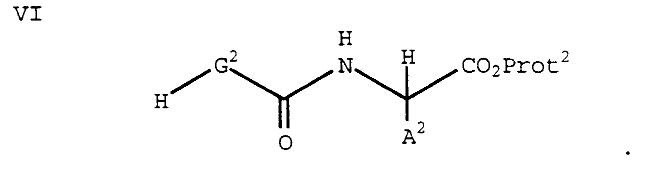
(The Prot¹ and Prot² protecting groups are chosen so that Prot¹ can be selectively removed in the presence of Prot²).

An amine of formula VI can be coupled with a suitable amine-protected amino acid of formula VII (wherein Prot³ is an amine protecting group): 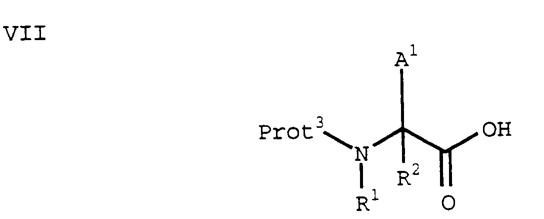
using an appropriate coupling reagent (e.g., BOP-Cl) to form a compound of formula VIII: 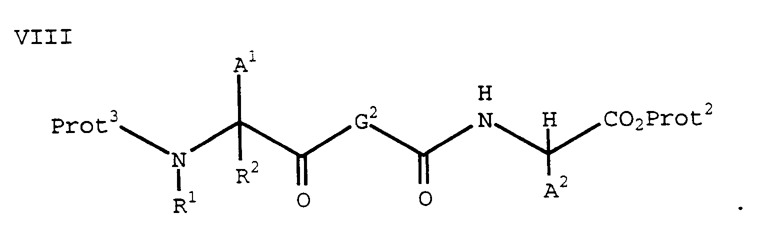
(Compounds of formula VII are known in the art. See, for example, R.M. Williams, "Synthesis of Optically Active α-Amino Acids," Pergamon Press, Oxford, 1989).

Compounds of formula VIII can also be prepared by coupling a compound of the formula IX (wherein Prot⁴ is a carboxylic acid protecting group): 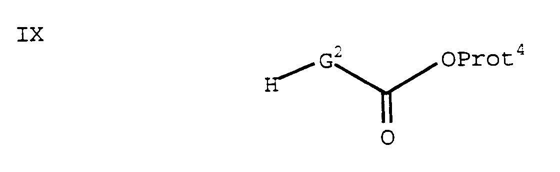
with an amine-protected amino acid of formula VII above to provide a compound of formula X: 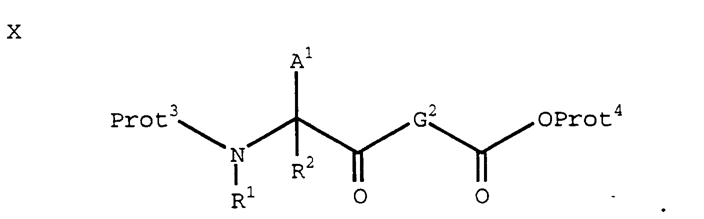
The Prot⁴ protecting group of compound X can be selectively removed by methods known in the art to provide a compound of formula XI below: 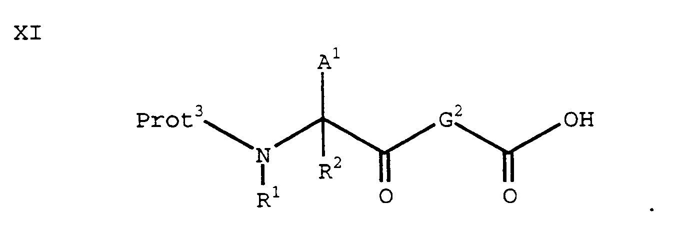
(The Prot³ and Prot⁴ protecting groups are chosen so that Prot⁴ can be selectively removed in the presence of Prot³).

Coupling of a compound of formula XI with an amino ester of formula IV would then provide compound VIII.

A compound of formula VIII can be selectively N-deprotected by methods known in the art to provide an amine of formula XII: 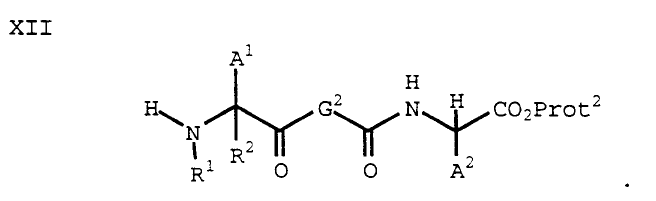
(The Prot² and Prot³ protecting groups are chosen so that Prot³ can be selectively removed in the presence of Prot²).

Additionally, an amine of the formula VI'(a), VI'(b) or VI'(c): 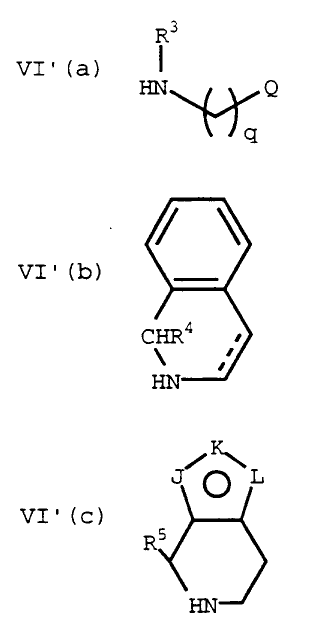
can be coupled with a suitable amino protected amino acid of the formula VII to provide a compound of the formula VIII'(a), VIII'(b) or VIII'(c), respectively: 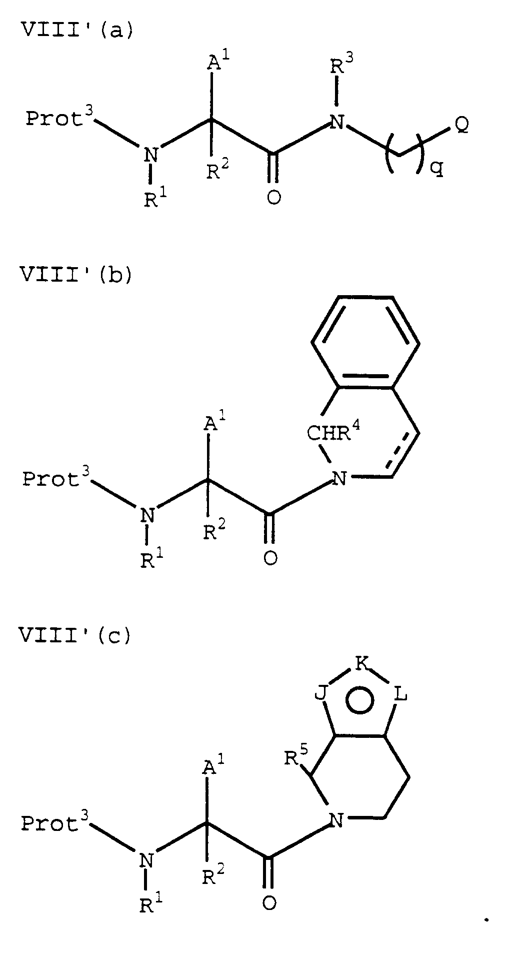
The Prot³ protecting group can then be removed by methods known in the art to provide a compound of the formula XII'(a), XII'(b) or XII'(c), respectively:

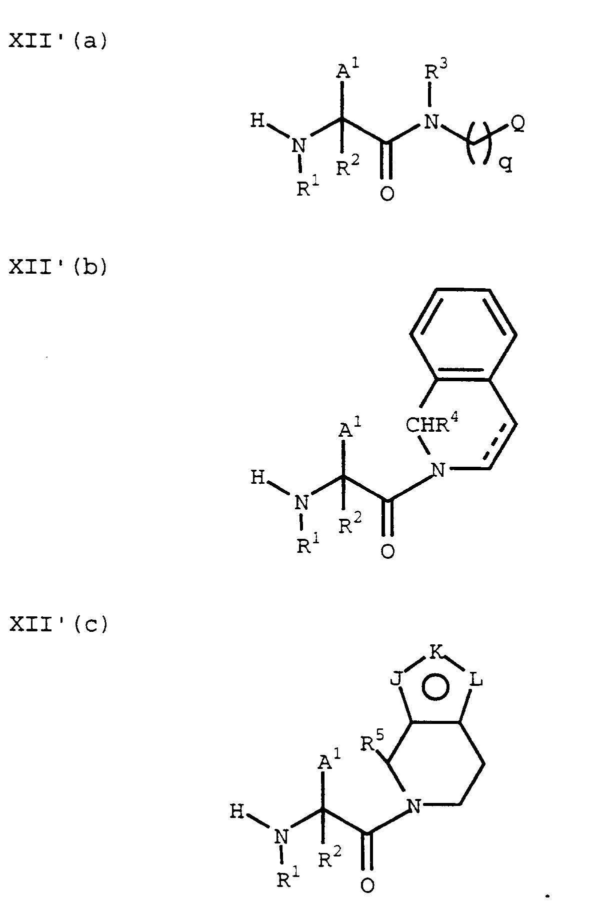

A compound of formula XII (or XII' (a-c)) can be condensed with a compound of formula XIII, in which groups X and X¹ are optionally protected (for example, when X is -SH, a thiol protecting group such as trityl may be used, and when X¹ is -NH, an amine protecting group such as Boc may be used): 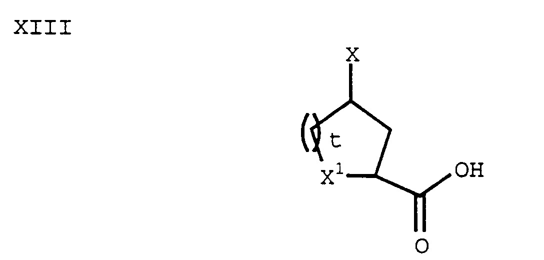
to give, following removal of protecting groups, a compound of formula I where Y is -(CO)-, Z is -(CO)- and G³, if present, is -CO₂H.

Compound of formula XIII can be prepared by methods known in the art. See, for example, V. Eskwarakrishnan and L. Field, J. Org. Chem., 46, 4182 (1981); D. Papaioannou, et al., Acta Chemica Scandinavia, 44, 243 (1990); A. Meyers, Org. Syn., 51, 103 (1971); T. Rosen, et al., Synthesis, 40 (1988); C. Agami, et al., Tetrahedron, 48, 431 (1992); and Y. Ueda and V. Vinet, Can. J. Chem., 64, 2184 (1986).

Further, a compound of formula I where Y and Z are -C(O)- can be prepared by automated solid phase peptide synthesis using methods that are well known in the art. See, for example:
a) M. Bodansky and A. Bodansky, "The Practice of Peptide Synthesis", Springer-Verlag, Berlin/Heidelberg/New York/Tokyo, 1984; and
b) J. M. Stewart and J. D. Young, "Solid Phase Peptide Synthesis", Pierce Chemical Co., Rockport, Illinois, 1984.

In another process, an amino acid of formula VII (wherein Prot³ is a suitable amino protecting group) can be converted to the N-methoxy-N-methylamide of formula XIV by methods known in the art:

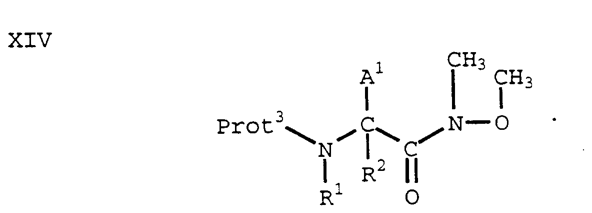

A compound of formula XIV can be reduced to an aldehyde of the formula XV by methods known in the art. (See, e.g., Fehrentz, *et al*., Synthesis, 676 (1983)):

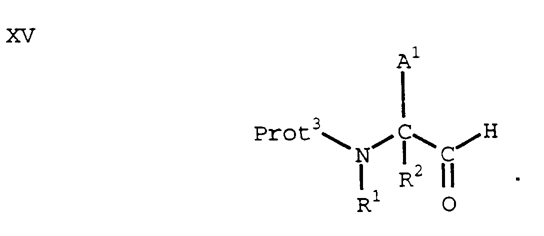

Alternatively, a compound of formula XV can be prepared by reduction of a compound of formula VII with a reducing agent such as borane, followed by oxidation of the resulting alcohol using, for example, the Swern method of oxidation. (See, e.g., Luly, *et al*., J. Org. Chem., 52, 1487 (1987); or Stanfield, *et al*., J. Org. Chem., 46, 4797 (1981)). In yet another alternative, a compound of the formula XV can be prepared by reduction of an ester of a compound of formula VII with a reducing agent such as diisobutylaluminum hydride. See, e.g., Rich, *et al*., J. Org. Chem., 43, 3624 (1978).

An N-protected aldehyde of formula XV can be reductively aminated with an amine of the formula VI to form a compound of the formula XVI using methods known in the art (e.g., Borch, *et al*., J. Am. Chem. Soc., 93, 2897 (1971)):

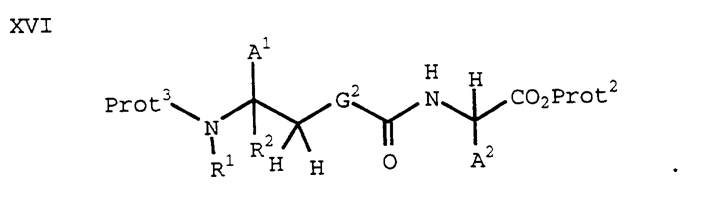

A compound of the formula XVI can be treated with a suitable N-deprotecting agent to provide the corresponding free amine of the formula XVII:

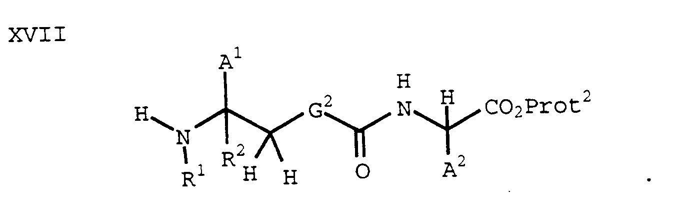

Similarly, an N-protected aldehyde of formula XV can be reductively aminated by the same methods with an amine of the formula VI' (a-c) to form a compound of the formula XVI'(a), XVI'(b) or XVI'(c), respectively:

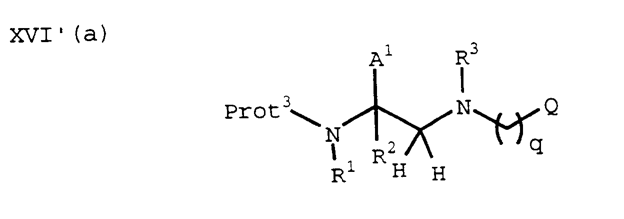

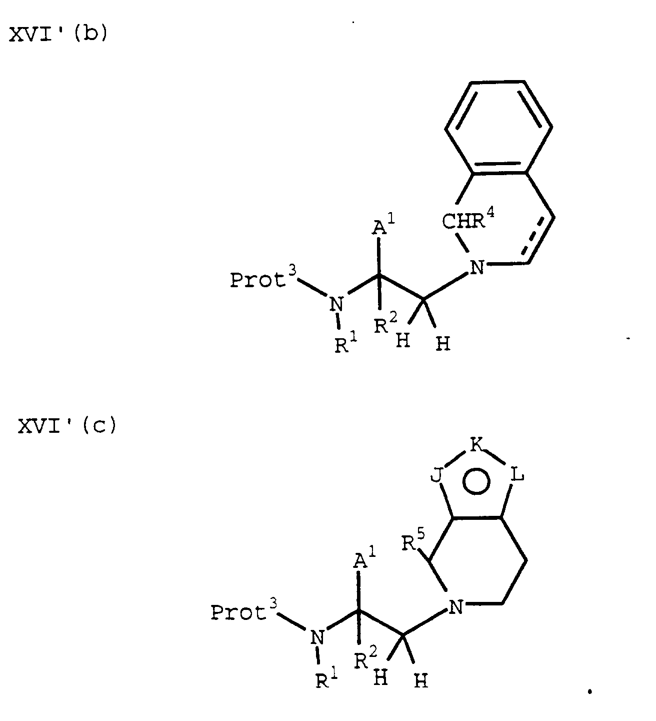

A compound of the formula XVI'(a - c) can be treated with a suitable N-deprotecting agent to provide the corresponding free amine of the formula XVII'(a), XVII'(b) or XVII'(c), respectively:

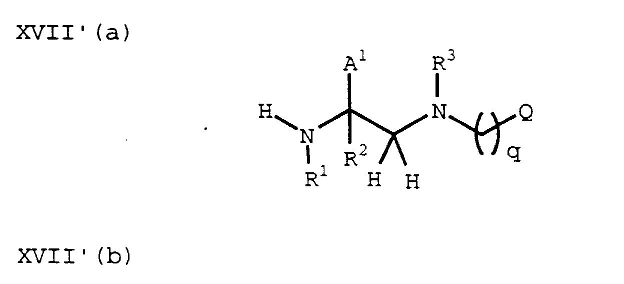

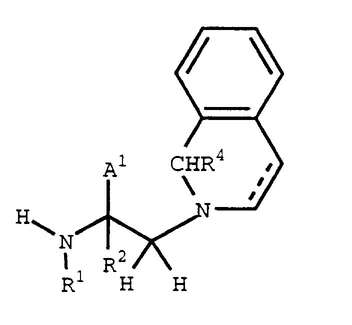

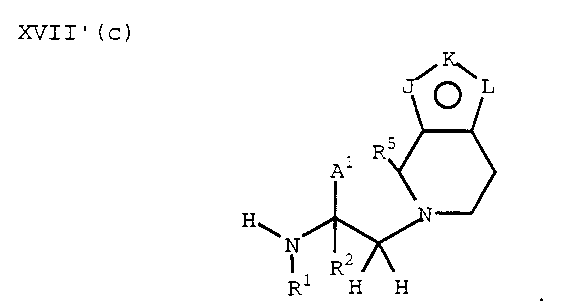

(The Prot² and Prot³ protecting groups are chosen so that Prot³ can be selectively removed in the presence of Prot²).

A compound of formula XVII (or XVII'(a-c)) can be coupled with a compound of formula XIII to give, following protecting group removal, a compound of formula I where Y is -(CO)-, Z is -CH₂- and G³, if present, is -CO₂H.

In another process, a compound of formula XVIII: 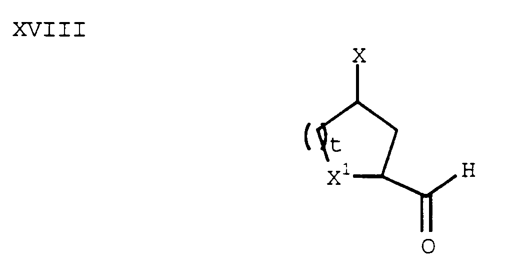
can be prepared from a compound of formula XIII using methods described for the preparation of a compound of formula XV. Then, a compound of formula XVIII can be reductively aminated with a compound of formula XII using an acid and a reducing agent (for example, NaCNBH₃) to give a compound of formula XIX: 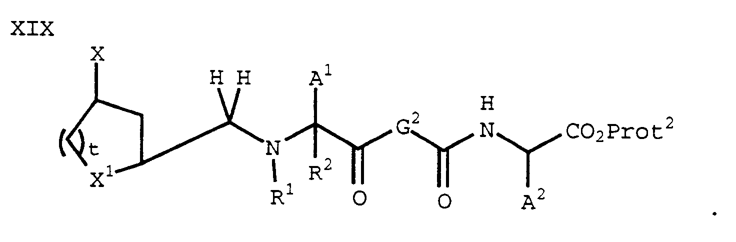
Removal of protecting groups from a compound of formula XIX provides a compound of formula I where Y is -CH₂-, Z is -(CO)- and G³ is -CO₂H.

An analogous process may be used with a compound of formula XII' (a-c) to form a compound of formula I where Y is -CH₂-, Z is -(CO)- and G is 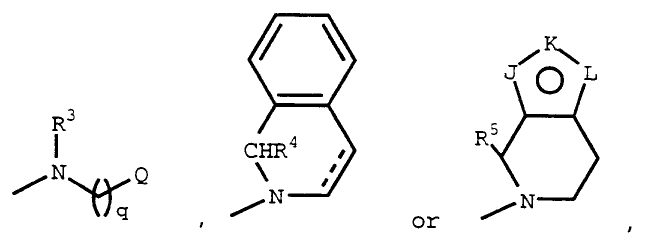
respectively.

In an alternate process, a compound of formula XVIII and a compound of formula XX: 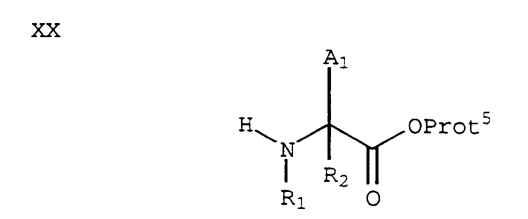
can be combined using methods described above to give a compound of formula XXI: 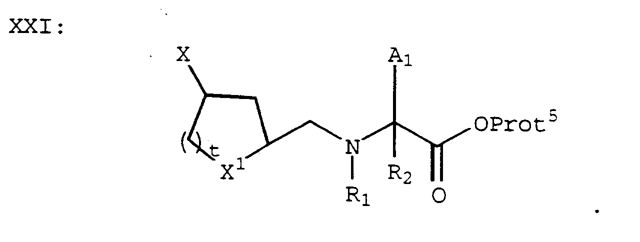
The protecting group can be removed by methods known in the art to provide a compound of formula XXII: 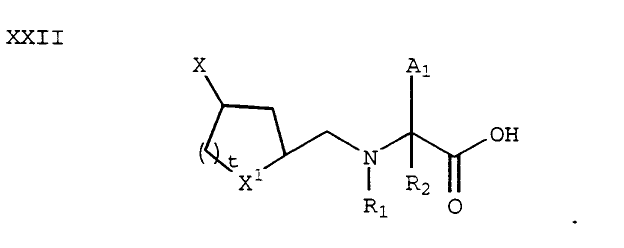
A compound of formula XXII can then be coupled with a compound of formula VI (or VI'a-c) to give, following removal of protecting groups, if any, a compound of formula I where Y is -CH₂- and Z is -(CO)-.

By combining the appropriate steps above, one skilled in the art can also prepare a compound of formula I where both Y and Z are -CH₂-.

Side-chain protecting groups may be used in these processes with amino acids, for example, having reactive functionalities such as hydroxyl, carboxyl, amino, mercapto, guanidino, imidazolyl, indolyl and the like. The particular protecting groups used for any amino acid residues depend upon the sidechains to be protected and are generally known in the art. Exemplary sidechain protecting groups include acetyl, benzoyl, benzyl, t-butyl and the like for hydroxyl; cyclohexyl, benzyl, methyl, ethyl, t-butyl and the like for carboxyl; benzyl, 4-methylbenzyl, 4-methoxybenzyl, acetyl, acetamidomethyl, triphenylmethyl (trityl) and the like for mercapto; t-butoxycarbonyl (Boc), benzyloxylcarbonyl (Cbz), N-[(9H-Fluoren-9-ylmethoxy)carbonyl] (Fmoc), phthaloyl (Pht), p-toluenesulfonyl (Tos), trifluoroacetyl, 2-(trimethylsilyl)ethoxycarbonyl (Teoc) and the like for amino; 2,4-dinitrophenyl, benzyloxymethyl, Tos, Boc, trityl and the like for imidazolyl; formyl, Cbz, Teoc, 2,2,2-trichloroethyl carbamate (TROC) and the like for indolyl; and tosyl, nitro, bis(1-adamantyloxycarbonyl) and the like for guanidino.

Side-chain protecting groups may be removed, if desired, by, for example, treatment with one or more deprotecting agents in an inert solvent or solvent mixture. For examples of protecting groups and suitable deprotecting agents, see M. Bodansky and A. Bodansky, "The Practice of Peptide Synthesis", Springer-Verlag, Inc. (1984); and T. W. Greene and P.G.M. Wuts, "Protective Groups in Organic Synthesis", Second Edition, John Wiley & Sons, New York, 1991.

The invention will now be further described by the following working example(s), which are preferred embodiments of the invention. All temperatures are in degrees Celsius (°C) unless otherwise indicated. These examples are illustrative rather than limiting.

### Example 1

### N-[[(S)-1,2,3,4-Tetrahydro-2-[N-[[(2S-trans)-4-mercapto-2-pyrrolidinyl]methyl]-L-valyl]-3-isoquinolinyl]carbonyl]-L-methionine, trifluoroacetate (1:3) salt

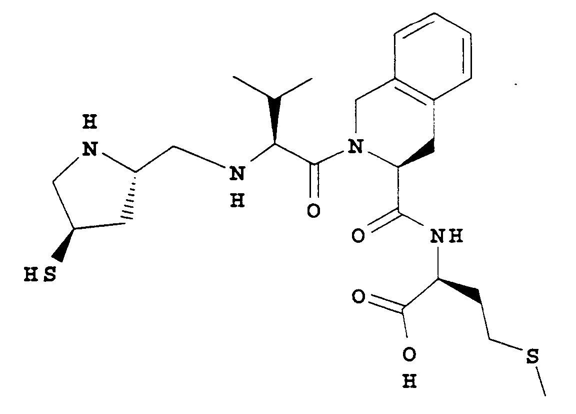

### A. [S-(R*,R*)]-4-Hydroxy-1,2-pyrrolidinedicarboxylic acid, 1-(1,1-dimethylethyl) ester

[R-(R*,S*)]-4-Hydroxy-1,2-pyrrolidinedicarboxylic acid, 1-(1,1-dimethylethyl) ester (10 g, 43 mmol) was combined with diisopropyl-azodicarboxylate (9.1 g, 45 mmol) in dry tetrahydrofuran (THF) (20 mL) under nitrogen. A solution of triphenylphosphine (12 g, 45 mmol) in dry THF (20 mL) was added dropwise under nitrogen at room temperature. The reaction was stirred for 20 hours and concentrated under vacuum followed by the addition of methanol (30 mL). A solution of 2N sodium hydroxide (NaOH) was added to bring the pH to 12 (0.4 mL), and the reaction was allowed to proceed until saponification was complete (2 hours). The solution was concentrated under vacuum and the residue was dissolved in brine/ethyl acetate. The layers were separated and the organic layer was dried with magnesium sulfate (MgSO₄), filtered and concentrated. The product crystallized on standing. The crystals were washed with ethyl ether and the ether extracts were concentrated to give additional product in crystalline form. The combined yield was 9.1 g.
MS (M+H)⁺ 232⁺, R_{f} 0.25 (chloroform:methanol:acetic acid/85:10:5, PMA).

### B. [S-(R*,R*)]-4-Hydroxy-1,2-pyrrolidine-dicarboxylic acid, 1-(1,1-dimethylethyl) 2-methyl ester

To a solution of Compound A (4.7 g, 20 mmol) in ethyl ether (10 mL) was slowly added diazomethane (25 mmol) dissolved in ethyl ether (approximately 80 mL). The reaction mixture was stirred for 1 hour at which time the yellow color disappeared. This was followed by concentration under vacuum to afford 5.0 g of Compound B. MS (M+H)⁺ 246⁺, R_{f} 0.57 (chloroform:methanol:acetic acid/85:10:5, PMA).

### C. [R-(R*,S*)1-4-[(Triphenylmethyl)thio]-1,2-pyrrolidinedicarboxylic acid, 1-(1,1-dimethylethyl) 2-methyl ester

To a solution of Compound B (2 g, 8.2 mmol) and pyridine (0.60 mL, 8.4 mmol) in methylene chloride (5 mL) at 0°C under nitrogen, was added triflic anhydride (1.5 mL, 8.9 mmol) in methylene chloride (5 mL) over 1 hour. The solution was stirred for an additional 15 minutes at 0°C. This was added directly to a stirred solution of triphenylmethylmercaptan (5.5 g, 20 mmol) and lithium hexamethyldisilazide (3.3 g, 20 mmol) in dry THF (30 mL) at 0°C. The mixture was warmed to room temperature and stirred for 16 hours. The solution was concentrated and the residue was chromatographed (260-400 mesh silica gel, 7.1 X 20 cm, 1:9-1:3/ethyl acetate:hexane). Fractions containing the desired product were collected and concentrated to yield 0.95 g of Compound C. MS (M+H)⁺ 504⁺.

### D. [R-(R*,S*)]-4-[(Triphenylmethyl)thio]-1,2-pyrrolidinedicarboxylic acid, 1-(1,1-dimethylethyl) ester

A solution of Compound C (0.8 g, 1.6 mmol) in THF (8 mL) and methanol (5 ml) was adjusted to pH 12 with 1N NaOH. This was stirred for 16 hours at room temperature. Additional 1N NaOH (0.3 mL) was added and stirring continued until saponification was complete by tlc (8 hr). Ethyl ether (50 mL) was added along with water (30 mL) and the layers were separated. The aqueous layer was washed with ethyl ether (2 x 40 mL), acidified to pH 3-4 using 1N potassium bisulfate (KHSO₄) and extracted again with ethyl ether (3 x 50 mL). The ether extracts from the acidic aqueous solution were dried (MgSO₄), pooled and concentrated to give 0.69 g of Compound D. MS (M+H)⁺ 489⁺, R_{f} 0.55 (chloroform: methanol:acetic acid/85:10:5; UV, PMA).

### E. [R-(R*,S*)]-2-[(N-methoxy-N-methylamino)-carbonyl]-4-[(triphenylmethyl)thio]-1-pyrrolidinecarboxylic acid, 1,1-dimethylethyl ester

Compound D (0.69 g, 1.4 mmol) was combined with N,O-dimethylhydroxylamine hydrochloride salt (140 mg, 1.5 mmol), bromo-tris-pyrrolidino-phosphonium hexafluorophosphate (680 mg, 1.5 mmol) and diisopropylethylamine (480 µL, 2.9 mmol) in methylene chloride (30 mL) under argon at room temperature. This was stirred for 2 hours and concentrated under vacuum. The residue was chromatographed (silica gel, 7.1 X 20 cm, 1:3-2:1/ethyl acetate:hexane) and fractions containing the desired compound were collected and concentrated to yield 432 mg of Compound E as a glass. MS (M+H)⁺ 533⁺.R_{f} 0.25 (chloroform:methanol:acetic acid/85:10:5, UV, PMA).

### F. [R-(R*,S*)]-2-Formyl-4-[(triphenyl-methyl)thio]-1-pyrrolidinecarboxylic acid, 1,1-dimethylethyl ester

Compound E (0.30 g, 0.56 mmol) was dissolved in dry THF (25 mL) and the solution was cooled to 0°C. 1M lithium aluminum hydride (LiAlH₄) in THF (1.1 mL, 1.1 mmol) was added dropwise over 15 minutes. The reaction mixture was stirred for 30 minutes at 0°C under nitrogen. Diethyl ether (70 mL) was added and the reaction mixture was quenched with dropwise addition of 1M KHSO₄ (10 mL) at 0°C. The mixture was stirred for 1 hour at 0°C and the layers were separated. The organic layer was washed with 1M KHSO₄ (4 x 50 ml), saturated sodium bicarbonate (NaHCO₃) (50 mL) and brine (50 mL), dried (MgSO₄), and concentrated to yield Compound F as a white glass (260 mg) which was used immediately in the next step without further purification. R_{f} 0.25 (ethyl acetate:hexane/1:1, UV, PMA).

### G. (R*)-N-[[2-[(1,1-Dimethylethoxy)-carbonyl]-1,2,3,4-tetrahydro-3-isoquinolinyl]carbonyl]-L-methionine. methyl ester

A solution of (S)-3,4-dihydro-2,3(1H)-isoquinolinedicarboxylic acid, 2-(1,1-dimethylethyl) ester (2.0 g, 7.21 mmol) and L-methionine methyl ester HCl (1.44 g, 7.21 mmol) in 5:15 N-methylpyrolidinone-methylene chloride was stirred at 4^{o}C. N,N-Diisopropylethylamine (1.23 mL, 7.21 mmol) was added, followed by N-hydroxybenzotriazole (974 mg, 7.21 mmol). The reaction mixture was stirred for 5 minutes, then 1-ethyl-3-(3-dimethyl-aminopropyl)carbodiimide·HCl (1.38 g, 7.21 mmol) was added. The reaction mixture was allowed to come to room temperature, stirred overnight and partitioned between methylene chloride and saturated sodium chloride (NaCl). The organic phase was washed successively with citric acid, NaHCO₃ and NaCl, dried over MgSO₄, and concentrated *in vacuo* to give 2.64 g of Compound G. This was used in the next step without further purification.

### H. (R*)-N-[(1,2,3,4-Tetrahydro-3-isoquinolinyl)carbonyl]-L-methionine, methyl ester

A solution of Compound G (2.00 g, 6.22 mmol) in 10 mL methylene chloride was treated at room temperature with 10 mL trifluoroacetic acid (TFA) and 0.5 mL dimethyl sulfide, and stirred for 0.5 hour. The reaction mixture was concentrated *in vacuo*, dissolved in methylene chloride and concentrated. This procedure was repeated five times to yield Compound H (99%) as a clear glass. This was used in the next step without further purification.

### I. (R*)-N-[[2-[N-[(1,1-Dimethylethoxy)-carbonyl]-L-valyl]-1,2,3,4-tetrahydro-3-isoquinolinyl]carbonyl]-L-methionine, methyl ester

Compound H (4.73 mmol) was dissolved in methylene chloride (20 mL) and cooled to 0°C. N-t-Butyloxycarbonyl-L-valine (2.06 g, 9.46 mmol), bis(2-oxo-3-oxazolidinyl)phosphinic chloride (1.2 g, 4.7 mmol) and N,N-diisopropylethylamine (1.2 g, 1.6 mL, 9.4 mmol) were added. The reaction mixture was stirred for 24 hours at 0°C. Additional bis(2-oxo-3-oxazolidinyl)phosphinic chloride (1.2 g, 4.7 mmol) and N,N-diisopropylethylamine (590 mg, 0.8 mL, 4.7 mmol) were added and the reaction mixture was stirred for an additional 12 hours at 0°C. The reaction mixture was concentrated and chromatographed (silica gel, eluting with 50% ethyl acetate, 50% hexane). The appropriate fractions were collected and concentrated to yield Compound I as a clear oil (2.4 g).

### J. (R*)-N-[[2-(L-Valyl)-1,2,3,4-tetrahydro-3-isoquinolinyl]carbonyl]-L-methionine, methyl

Compound I (190 mg, 0.45 mmol) was stirred in methyl sulfide (0.3 mL) and 4N hydrochloric acid in dioxane (10mL) for 30 minutes at room temperature. The reaction mixture was concentrated *in vacuo*, dissolved in methylene chloride (30 mL) and concentrated. This latter procedure was repeated five times to yield Compound J (99%) as a clear glass. This was used in the next coupling step without further purification.

### K. N-[[(S)-2-[N-[[(2S-trans)-1-[(1,1-Dimethylethoxy)carbonyl]-4-[(triphenyl methyl)thio]-2-pyrrolidinyl]methyl]-L-valyl]-1,2,3,4-tetrahydro-3-isoquinolinyl]carbonyl-L-methionine, methyl ester

A solution of Compound F (0.26 g, 0.56 mmol), Compound J (0.26 g, 0.56 mmol) and glacial acetic acid (0.4 mL) in dry methanol (15 mL) was stirred for 10 minutes followed by the addition of sodium cyanoborohydride (NaBH₃CN) (35 mg, 0.56 mmol) in portions over 1 hour. Stirring under nitrogen continued for 16 hours. The solution was cooled to 0°C, and saturated aqueous sodium bicarbonate solution was added (10 mL) along with ethyl acetate (60 mL) and water (50 mL). The layers were separated, and the organic layer was washed with water, dried (MgSO₄) and concentrated to a residue. This residue was chromatographed on a flash silica gel column (5 x 20 cm) eluting with ethyl acetate:hexanes/1:2-2:1 to afford 310 mg of Compound K. MS (M+H)⁺ 879⁺, R_{f} 0.2 (ethyl acetate:hexane/1:1, UV, ninhydrin, PMA).

### L. N-[[(S)-2-[N-[[(2S-trans)-1-[(1,1-Dimethylethoxy)carbonyl]-4-[(triphenyl-methyl)thio]-2-pyrrolidinyl]methyl]-L-valyl]-1,2,3,4-tetrahydro-3-isoquinolinyl]carbonyl-L-methionine

A solution of Compound K (45 mg, 0.05 mmol) in 10 ml of methanol was adjusted to pH 12 (0.5 mL) with 2N NaOH. This was stirred until there was quantitative disappearance of starting material (2.5 hours) as detected by tlc (chloroform, methanol, acetic acid/85:10:5). The pH was adjusted to 3-4 using 1N KHSO₄, and ethyl acetate (30 mL) was added. The layers were separated and the aqueous layer was washed with ethyl acetate (3 x 10 mL). The organic extracts were pooled, dried (MgSO₄) and concentrated to give 39 mg of Compound L. MS; (M+H)⁺ 835⁺.

### M. N-[[(S)-1,2,3,4-Tetrahydro-2-[N-[[(2S-trans)-4-mercapto-2-pyrrolidinyl]methyl]-L-valyl]-3-isoquinolinyl]carbonyl]-L-methionine, trifluoroacetate (1:3) salt

Compound L (20 mg, 0.023 mmol) was combined with TFA (7 mL), methylene chloride (7 mL) and triethylsilane (38 µL, 0.24 mmol). The solution was stirred for 1 hour and then concentrated under vacuum. Methylene chloride was added and the solution was concentrated and thoroughly dried under vacuum. The residue was dissolved in methanol (2.5 mL), filtered and applied to a YMC C18 column (S-10, ODS 30 x 500 mm) with monitoring at 220 nm. HPLC purification was performed under the following conditions: Solvent A- 0.1% TFA in 90% water, 10% methanol; Solvent B- 0.1% TFA in 90% methanol, 10% water; 20-50% B in A over 40 minutes. Fractions containing the major peak were pooled and lyophilized to yield 7.4 mg of the title compound as a white powder. MS (M+H)⁺ 523⁺, ¹H-NMR(CD₃OD, 400 MHz) δ (ppm) 7.26 (4H, m), 4.72 (2H, m), 4.35 (1H, m), 4.25 (1H, m), 4.20 (2H, m), 3.50 (2H, m), 3.28 (4H, m), 3.19 (1H, dd), 2.51 (3H, m), 2.22 (4H, m), 2.07 (1.5H, s), 1.95 (1.5H, s), 1.20 (6H, m).
Elemental Analysis for C₂₅H₃₈N₄O₄S₂·3C₂HF₃O₂, 2H₂O:
Calculated: C 41.32, H 5.03, N 6.21;
Found: C 41.27, H 5.09, N 6.56.

### Example 2

### N-[[(S)-1,2,3,4-Tetrahydro-2-[N-[[(2S-cis)-4-mercapto-2-pyrrolidinyl]methyl]-L-valyl]-3-isoquinolinyl]carbonyl]-L-methionine. trifluoroacetate (2:3) salt

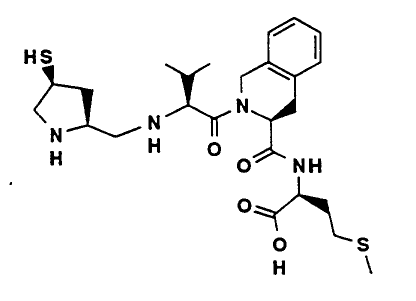

### A. [S-(R*,R*)]-4-[(Triphenylmethyl)thio]-1,2-pyrrolidinedicarboxylic acid, 1-(1,1-dimethylethyl) 2-methyl ester

To a solution of [R-(R*,S*)]-4-Hydroxy-1,2-pyrrolidinedicarboxylic acid, 1-(1,1-dimethylethyl) 2-methyl ester (2.0 g, 8.2 mmol) and 2,6-di-tert-butyl-4-methylpyridine (1.7 g, 8.2 mmol) in methylene chloride (30 mL) at -40°C under nitrogen, was added triflic anhydride (1.5 mL, 8.9 mmol) dropwise via syringe over 15 minutes. The solution was stirred at -40°C for 0.5 hours and at 0°C for 1 hour. This solution was added slowly (over 0.5 hours) to a solution of triphenyl-methylmercaptan (5.5 g, 20 mmol) and lithium hexamethyldisilazide (1 Molar solution in THF, 20 mL, 20 mmol) in THF (10 mL) at 0°C. The resulting solution was stirred for 16 hours at room temperature.

The solution was concentrated and the residue was chromatographed (260-400 mesh silica gel, 7.1 X 20 cm, 1:9-1:3/ethyl acetate:hexane). Fractions containing the desired product were collected and concentrated to yield 2.5 g of Compound A. MS (M+H)⁺ 504⁺.

### B. [S-R*,R*)]-4-[Triphenylmethyl)thio]-1,2-pyrrolidinedicarboxylic acid, 1-(1,1-dimethylethyl) ester

To a solution of Compound A (1.0 g, 1.9 mmol) in THF (20 mL) and methanol (10 ml) was added 1M lithium hydroxide (LiOH) (3 mL, 3 mmol). The solution was stirred for 3 hours until saponification was complete. To this solution was added 1N KHSO₄ (3.0 mL, 3.0 mmol). The solution was concentrated under vacuum followed by addition of methylene chloride (50 mL). The organic layer was washed with 1M KHSO₄ (3 x 15 mL), dried (MgSO₄) and concentrated to give 0.97 g of Compound B. MS (M-H)⁻ 488⁻, R_{f} 0.55 (chloroform: methanol:acetic acid/85:10:5; UV, PMA).

### C. [S-(R*,R*)]-2-[(N-Methoxy-N-methyl-amino)carbonyl]-4-[(triphenylmethyl)thio]-1-pyrrolidinecarboxylic acid, 1,1-dimethyl ethyl ester

Compound B (0.74 g, 1.5 mmol) was treated using the procedure described in Example 1E to yield Compound C as a glass. Yield: 640 mg, MS (M+H)⁺ 533⁺.R_{f} 0.25 (chloroform:methanol:acetic acid/85:10:5, UV, PMA).

### D. [S-(R*,R*)]-2-Formyl-4-[(triphenyl-methyl)thio]-1-pyrrolidinecarboxylic acid, 1,1-dimethylethyl ester

Compound C (0.35 g, 0.66 mmol) was treated using the procedure described in Example 1F to yield Compound D as a white glass (310 mg). R_{f} 0.25 (ethyl acetate:hexane/1:1, UV, PMA).

### E. N-[[(S)-2-[N-[[(2S-cis)-1-[(1,1-Dimethylethoxy)carbonyl]-4-[(triphenyl-methyl)thio]-2-pyrrolidinyl]methyl]-L-valyl]-1,2,3,4-tetrahydro-3-isoquinolinyl]carbonyl-L-methionine, methyl ester

Compound D (0.310 g, 0.66 mmol), and Compound 1J (0.616 mg, 1.3 mmol), were treated using the procedure described in Example 1K to afford 310 mg of Compound E. MS (M+H)⁺ 879⁺, R_{f} 0.2 (ethyl acetate:hexane/1:1, UV, ninhydrin, PMA).

### F. N-[[(S)-2-[N-[[(2S-cis)-1-[(1,1-Dimethylethoxy)carbonyl]-4-[(triphenylmethyl)thio]-2-pyrrolidinyl]methyl]-L-valyl]-1,2,3,4-tetrahydro-3-isoquinolinyl]carbony]-L-methionine

Compound E (270 mg, 0.05 mmol) was treated using the procedure described in Example 1L to give 210 mg of Compound F. MS; (M+H)⁺ 835⁺.

### G. N-[[(S)-1,2,3,4-Tetrahydro-2-[N-[[(2S-cis)-4-mercapto-2-pyrrolidinyl]methyl]-L-valyl]-3-isoquinolinyl]carbonyl]-L-methionine, trifluoroacetate (2:3) salt

Compound F (210 mg, 0.24 mmol) was combined with TFA (7 mL), methylene chloride (30 mL) and triethylsilane (80 µL, 0.48 mmol). The solution was stirred for 2 hours and concentrated under vacuum. Methylene chloride (20 ml) was added and the solution was concentrated. The residue was dissolved in methanol (3 mL), filtered and applied to a YMC C18 column (S-10, ODS 30 x 500 mm) with monitoring at 220 nm. HPLC purification was performed under the following conditions: Solvent A- 0.1% TFA in 90% water, 10% methanol; Solvent B-0.1% TFA in 90% methanol, 10% water; 20-50% B in A over 40 minutes. Pooling of appropriate fractions afforded 36 mg of the title compound as a white powder. MS (M+H)⁺ 523⁺, ¹H-NMR(CD₃OD, 400 MHz) δ (ppm) 7.26 (4H, m), 4.72 (4H, m), 3.92 (2H, m), 3.41 (3H, m), 3.19 (4H, m), 2.62 (1H, m), 2.43 (1H, m), 2.19 (2H, m), 1.95 (3H, s), 1.77 (4H, m) 1.20 (6H, m).
Elemental Analysis for C₂₅H₃₈N₄O₄S₂·1.5C₂HF₃O₂, 2.5H₂O:
Calculated: C 41.52, H 6.07, N 7.58, F 11.57;
Found: C 41.54, H 5.52, N 7.51, F 11.52.
Rotation: [α]_{D} -33.7^{o} (c=0.79, CH₃OD)

### Example 3

### N²-[[(S)-1,2,3,4-Tetrahydro-2-[N-[[2S-cis)-4-mercapto-2-pyrrolidinyl]methyl]-3-methyl-L-valyl]-3-isoquinolinyl]carbonyl]-L-glutamine. trifluoroacetate

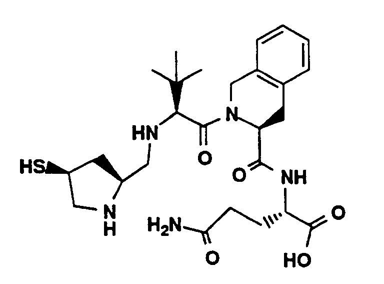

### A. 3-Methyl-N-[(phenylmethoxy)carbonyl]-L-valine

To a solution of L-tert-leucine (10.2 g, 77.6 mmol) in aqueous NaOH (2N, 40 mL) at 0°C was added benzyl chloroformate (12.1 mL, 85.4 mmol) and NaOH (2N, 40 mL) alternatively in 5 portions over 30 minutes. The cold bath was removed and after 1 hour the pH of the mixture was adjusted to 10. The mixture was extracted with ether (2 x 50 mL). The aqueous layer was cooled to 0°C and acidified (pH 2) with 5 N HCl and extracted with ether (3 x 70 mL). The organic layer was dried (magnesium sulfate) and concentrated *in vacuo* to afford a thick oil Compound A (18.2 g, 88%). MS (CI) : (M+H)⁺ = 266⁺

### B. (S)-1,2,3,4-Tetrahydro-2-[3-methyl-N-[(phenylmethoxy)carbonyl]-L-valyl]-3-isoquinolinecarboxylic acid, methyl ester

To a solution of Compound A (5.31 g, 20 mmol) in methylene chloride (80 mL) at 0°C under argon were sequentially added diisopropylethylamine (10.6 mL, 60 mmol), benzotriazol-1-yloxytris-(di-methylamino)phophonium hexafluorophosphate (BOP) (5.08 g, 20 mmol) and (S)-1,2,3,4-Tetrahydroisoquinoline-3-carboxylic acid, methyl ester, hydrochloride (5.68 g, 25 mmol). The reaction mixture was allowed to warm to 5°C over 2 hours, and then stirred overnight (16 hours). The mixture was washed with 1N HCl, saturated NaHCO₃ and brine (50 mL each). The organic layer was dried (MgSO₄), filtered and concentrated to afford an oil. Purification by flash silica gel column chromatography eluting with 20% ethyl acetate in hexanes afforded Compound B (4.0 g, 45%).
MS: (CI) (M+H)⁺ = 439⁺

### C. (S)-1,2,3,4-Tetrahydro-2-[3-methyl-N-[(phenylmethoxy)carbonyl]-L-valyl]-3-isoquinolinecarboxylic acid

To a solution of Compound B (830 mg, 1.9 mmol) in THF/methanol (4 mL/5 mL) at room temperature was added LiOH (1N, 1.9 mL ). After 1 hour, additional LiOH (1N, 1.5 mL) was added. After 2 hours, NaOH (1N, 1.9 mL) was added. After another hour, solvent was removed and the residue was partitioned between 1N HCl and ethyl acetate (10 mL each). The organic layer was dried (MgSO₄) and concentrated *in vacuo* to afford Compound C (820 mg).

### D. (R*)-N²-[[1,2,3,4-Tetrahydro-2-[3-methyl-N-[(phenylmethoxy)carbonyl]-L-valyl]-3-isoquinolinyl]carbonyl]-L-glutamine, 1,1-dimethylethyl ester

Diisopropylethylamine (1.1 ml, 6.3 mmol) was added to a solution of Compound C (0.9 g, 2.1 mmol), L-glutamine t-butyl ester hydrochloride (0.501 g, 2.1 mmol), and BOP (0.93 g, 2.1 mmol) in 3:1 acetonitrile/dimethylformamide (26 ml) and stirred at room temperature for 16 hours. The reaction was quenched with HCl (1N, 100 ml) and extracted with ethyl acetate (4 x 75 mL). The combined organic extracts were washed with 10% lithium chloride (3 x 150 mL). The washed extracts were dried (MgSO₄), filtered and concentrated under vacuum. The residue was purified by flash chromatography (eluting with 1/1 hexane/acetone) to afford 1.1 g of Compound D as a white solid.
mp: 60-68°C
TLC: R_{f} = 0.62 (2/1 acetone/hexane, visualization by UV)
MS: (M+H)⁺ 609.

### E. (R*)-N²-[[1,2,3,4-Tetrahydro-2-(3-methyl-L-valyl)-3-isoquinolinyl]-carbonyl)-L-glutamine, 1,1-dimethylethyl ester, hydrochloride

Palladium hydroxide on carbon ( 10%, 0.091 g) was added to a solution of Compound D (0.914 g, 1.5 mmol) in THF (9.1 mL) with 1N HCl (1.5 ml). A hydrogen balloon was attached to the reaction and the mixture was stirred at room temperature for 3 hours. The reaction mixture was filtered through Celite® and concentrated under vacuum to afford 0.767 g of Compound E which was used without further purification.
TLC: R_{f} = 0.77 (6/3/1 n-propanol/ammonium hydroxide/water, visualization by ninhydrin)
MS: (M+H)⁺ 475.

### F. N²-[[(S)-2-[N-[[2S-cis)-1-[(1,1-Dimethylethoxy)carbonyl]-4-[(triphenylmethyl)thio]-2-pyrroli dinyl]methyl]-3-methyl-L-valyl]1,2,3,4-tetrahydro-3-isoquinolinyl]carbonyl]-L-glutamine, 1,1-dimethyl ester

Compound 2D (0.140 g, 0.3 mmol), and Compound E (0.150 g, 0.3 mmol) were treated as in Example 1K, except that the residue was chromatographed on a flash silica gel column (5 x 20cm) eluting with methylene chloride:methanol/9:1 to afford 161 mg of Compound F. MS (M+H)⁺ 932⁺, R_{f} 0.1 (methylene chloride:methanol/9:1, UV, ninhydrin, PMA).

### G. N²-[[(S)-1,2,3,4-Tetrahydro-2-[N-[[(2S-cis)-4-mercapto-2-pyrrolidinyl]methyl]-3-methyl-L-valyl]-3-isoquinolinyl]carbonyl]-L-glutamine, trifluoroacetate

Compound F (160 mg, 0.17 mmol) was treated as in Example 2G, except 15 equivalents of triethylsilane was used. 80 mg of the title compound was prepared as a white powder. MS (M+H)⁺ 534⁺, mp 133-135^{o}C,
Analysis calculated for C₂₆H₃₉N₅O₅S·2.1C₂HF₃O₂, 0.85H₂O:
Calculated: C 46.01, H 5.47, N 8.88, F 15.18,
Found: C 46.02, H 5.30, N 8.87, F 15.21.
¹H-NMR,(CD₃OD, 400 MHz) δ (ppm) 7.34-7.18 (4H, m), 5.02 (1H, m), 4.79 (1H, m), 5.02 (1H, m), 4.60-4.51 (1H, m), 4.25 (1H, m), 3.90 (1H, m), 3.67 (1H, m), 3.41 (1H, m), 3.19 (2H,m), 2.61 (1H,m), 2.50 (1H,m), 2.32 (1H, m), 2.04 (1H, m), 1.97 (2H, m), 1.89 (1H, m), 1.70 (1H, m), 1.13 (9H, d).
¹³C-NMR(100 MHz); 176.5, 173.4, 170.8, 134.4, 131.6, 128.6, 127.6, 127.5, 126.7, 125.8, 66.4, 58.6, 56.6, 53.4, 52.51, 45.1, 39.2, 35.6, 34.3, 32.4, 31.8, 31.7, 31.0, 26.7.
IR (KBr) 1676, 1427, 1204, 1136 cm⁻¹, [α]_{D} -18.6 (c = 0.45, methanol)

### Example 4

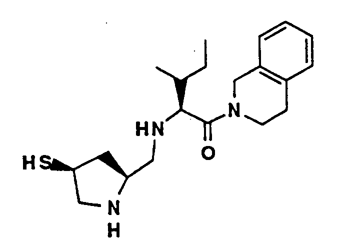

### 2-[2(S)-[[(4(S)-Mercapto-2(S)-pyrrolidinyl)-methyl]amino]-3(S)-methyl-1-oxopentyl]-1,2,3,4-tetrahydroisoquinoline, trifluoroacetate (1:2)

### A. 2-[N-[(1,1-Dimethylethoxy)carbonyl]-L-isoleucyl)-1,2,3,4-tetrahydroisoquinoline

A solution of t-butoxycarbonyl-isoleucine hemihydrate (4.8 g, 20 mmol), 1,2,3,4-tetrahydroisoquinoline (3.8 mL, 30 mmol), 1-(3-dimethyl-aminopropyl)-3-ethylcarbodiimide hydrochloride (4.2 g, 22 mmol) and hydroxybenzotriazole (3.0 g, 22 mmol) in 20 mL of dimethylformamide (DMF) was stirred for 62 hours. Most of the DMF was removed under vacuum with gentle warming, water was added and the mixture was extracted twice with ether. The combined organic phases were washed three times with aqueous 5% KHSO₄, twice with 5% aqueous NaHCO₃ and once with brine, dried (MgSO₄) and evaporated to afford 6.9 g of yellow gum. Flash chromatography on silica with 25% ethyl acetate/hexanes provided 6.9 g (100%) of Compound A as a clear gum.
¹³C (CDCl₃) 12.32, 16.70, 16.76, 24.83, 29.22, 29.33, 30.55, 39.11, 39.23, 41.16, 44.33, 45.49, 48.43, 55.52, 55.64, 80.33, 156.71, 172.11, 172.34 ppm.

### B. 2-(L-isoleucyl)-1,2,3,4-tetrahydroisoquinoline, hydrochloride

To Compound A (1.60 g, 4.62 mmol) was added 6 mL of 4N HCl in dioxane. The solution was stirred for 2.25 hours, evaporated and chased with ether twice to afford 1.29 g (98%) of Compound B as a foamy white solid.

### C. 2-[2(S)[[1-[(1,1-Dimethylethoxy)carbonyl]-4-(S)-[(triphenylmethyl)thio]-2(S)-pyrrolidinyl]methyl]amino]-3(S)-methyl-1-oxopentyl]-1,2,3,4-tetrahydroisoquinoline

Compound 2D (0.14 g, 0.3 mmol) and Compound B (0.085 g, 0.3 mmol) were treated as in Example 1K to provide 160 mg of Compound C. MS (M+H)⁺ 704⁺, R_{f} 0.5 (ethyl acetate:hexanes/1:1, UV, ninhydrin, PMA).

### D. 2-[2(S)-[[(4(S)-Mercapto-2(S)-pyrrolidinyl)-methyl]amino]-3(S)-methyl-1-oxopentyl]-1,2,3,4-tetrahydroisoquinoline, trifluoroacetate (1:2)

Compound C (150 mg, 0.21 mmol) was treated as in Example 3G to provide 78 mg (59%) of the title compound as a white powder. MS (M+H)⁺ 362⁺, M.P. 65-67^{o}C,
Analysis calculated for C₂₀H₃₁N₃OS·2.1C₂HF₃O₂, 0.45H₂O:
Calculated: C 47.47, H 5.58, N 6.83, F 19.93,
Found: C 47.48, H 5.50, N 6.91, F 19.93
¹H-NMR(CDCl₃, 400 MHz) δ (ppm) 7.34-7.18 (4H, m), 4.87 (1H, m), 4.69(2H, m), 3.9 (1H, m), 3.79-3.31 (5H, m), 3.2 (2H, m), 2.95(2H, m), 2.61(1H, q, J=6.4Hz),1.92 (1H, m), 1.65(2H, m), 1.22(1H, m), 1.12-0.85(6H, m).
¹³C-NMR(100 MHz); 134.0, 131.7, 128.9, 128.5, 127.6, 127.1, 126.9, 126.5, 126.1,63.6, 56.8, 53.4, 47.8, 45.0, 43.8, 41.4, 39.6, 37.2, 34.0, 29.3, 28.1, 24.6, 14.7,11.6.
IR (KBr) 1678, 1454, 1204, 1134 cm⁻¹, [α]_{D} -14.3 (c = 0.96, methanol)

### Example 5

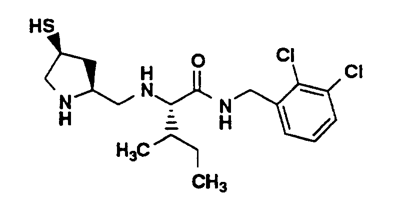

### [S-(R*,R*)]-N-[(2,3-Dichlorophenyl)methyl]-N²-[(4-mercapto-2-pyrrolidinyl)methyl]-L-isoleucinamide, trifluoroacetate

### A. N-[(1,1-Dimethylethoxy)carbonyl]-L-isoleucine, 2,3-dichlorobenzylamide

A suspension of Boc-isoleucine hemihydrate (0.61 g, 2.5 mmol), 2,3-dichlorobenzylamine hydrochloride (0.36 g, 1.75 mmol), N-methylmorpholine (0.22 mL, 2.0 mmol), 1-(3-dimethylaminopropyl)-3-ethylcarbodiimide hydrochloride (0.48 g, 2.5 mmol) and hydroxybenzotriazole (0.34 g, 2.5 mmol) in 10 mL of 9:1 THF:DMF was stirred 13 hours. Half saturated NaHCO₃ and ethyl acetate were added, the mixture was partitioned, the organic phase was washed again with aqueous NaHCO₃, twice with aqueous 5% KHSO₄ and once with brine, dried (MgSO₄) and evaporated to afford 0.90 g of foamy gum. Flash chromatography on silica with 25% ethyl acetate/hexanes provided 0.61 g (92%) of Compound A as a white solid.
¹³C (CDCl₃) 10.95, 15.45, 24.71, 28.17, 36.68, 41.41, 59.26, 79.57, 126.50, 126.90, 128.92, 131.00, 132.81, 137.69, 156.03, 172.53 ppm.

### B. N-[(2,3-Dichlorophenyl)methyl]-L-isoleucinamide, hydrochloride

Compound A (0.60 g, 1.5 mmol) was treated as in Example 4B to provide 0.47 g (94%) of Compound B as a white solid.

### C. N-[(2,3-Dichlorophenyl)methyl]-N²-[[1-[(1,1-Dimethylethoxy)carbonyl]-4-[(triphenylmethyl)thio]-2-pyrrolidinyl]methyl]-L-isoleucinamide

Compound 2D (270 mg, 0.57 mmol) and Compound B (140 mg) were treated as in Example 1K to provide 240 mg of Compound C as a clear oil. MS (M+H)⁺ 746⁺ , ¹H-NMR (CD₃OD, 400 MHz) δ (ppm) 7.45-7.20 (18H, m), 4.52 (2H, d, J=6.0Hz), 3.73 (1H, m), 3.52 (1H, m), 2.85 (1H, m), 2.71 (1H, m), 2.67 (1H, m), 2.48 (1H, m), 2.29 (1H, m), 1.82 (1H, m), 1.64 (1H, m), 1.35 (9H, m), 1.12 (1H, m), 0.90-0.81 (6H, m).

### D. [S-(R*,R*)]-N-[(2,3-Dichlorophenyl)methyl]-N²-[(4-mercapto-2-pyrrolidinyl)methyl]-L-isoleucinamide, trifluoroacetate

Compound C (200 mg, 0.268 mmol) was treated as in Example 2G to provide 92 mg of the title compound. Purity >99.4 % (YMC S3 ODS column, 6 x 150 mm, 220 nm, 1.5 mL/min, 0-100% B in A over 30 minutes (A, 0.2% phosphoric acid in 90% water, 10% methanol; B, 0.2% phosphoric acid in 90% methanol, 10% water, RT 19.83 minutes HI>99.4 %), [α]_{D}25° = - 9.0° (c=0.1, methanol).
MP 134-135°C, MS (M+H)⁺ 404⁺, Analysis: Calculated for C₁₈H₂₇N₃Cl₂OS·2.60CF₃CO₂H : C, 39.76; H, 4.26; N, 6.00; F, 21.14; Found: C, 40.08; H, 4.05; N, 5.90; F, 21.18. ¹H-NMR (CD₃OD, 400 MHz) δ (ppm) 7.49 (1H,m), 7.37 (1H, m), 7.28 (1H, t), 4.63-4.49 (2H, d of d), 3.88 (1H, m), 3.68 (1H, m), 3.53 (2H, m), 3.23 (1H, m), 3.15 (2H, m), 2.68 (1H, m), 1.84 (1H, m), 1.74 (1H, m), 1.61 (1H, m), 1.19 (1H, m), 0.98-0.91 (6H, m).

### Example 6

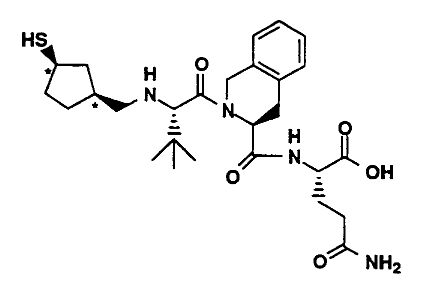

### N²-[[(S)-1,2,3,4-Tetrahydro-2-[N-[[(cis)-3-mercabtocyclopentyl]methyl]-3-methyl-L-valyl]-3-isoquinolinyl]carbonyl]-L-glutamine. trifluoroacetate

### A. Cis-N-Methoxy-N-methyl-3-[triphenyl methyl)thio]cyclopentanecarboxamide

To a mixture of 950 mg (2.45 mmol) of (±)-cis-3-[(triphenylmethyl)thio]cyclopentanecarboxylic acid [Ueda, Y., Vinet, V. Can. J. Chem. **64**, 2184, 1986], 286 mg (2.93 mmol) of N,O-dimethyl-hydroxylamine hydrochloride, 448 mg (2.93 mmol) of N-hydroxybenzotriazole and 0.51 mL (2.93 mmol) of diisopropylethylamine in 5 mL of N,N-dimethylformamide at 0°C was added 562 mg (2.93 mmol) of 1-(3-dimethylaminopropyl)-3-ethyl-carbodiimide hydrochlroide. The mixture was warmed to room temperature and stirred for 8 hours. The mixture was diluted with 50 mL of ethyl acetate and washed with 150 mL each of water, 1M HCl, saturated NaHCO₃ and brine. The organic layer was dried over sodium sulfate (Na₂SO₄), filtered and concentrated in vacuo to give 978 mg of Compound A.

### B. Cis-3-[Triphenylmethyl)thio]cyclopentanecarboxaldehyde

To a solution of 938 mg (2.17 mmol) of Compound A in 20 mL of diethyl ether at -15°C was added 2.6 mL of LiAH₄ (1M in THF). The mixture was stirred at -15°C for 30 minutes, and then quenched by the addition of 30 mL of 10% KHSO₄. The mixture was stirred for 30 minutes at room temperature and then extracted with 50 mL of diethyl ether. The organic extract was washed with 1M HCl (2 x 50 mL), saturated sodium bicarbonate (50 mL) and brine (50 mL). The organic layer was dried over Na₂SO₄, filtered and concentrated *in vacuo* to give 758 mg of Compound B.

### C. N²-[[(S)-1,2,3,4-Tetrahydro-2-N-[[(cis)-3-[triphenylmethyl)thio]-cyclopentyl]methyl]-3-methyl-L-valyl]-3-isoquinolinyl]carbonyl]-L-glutamine, 1,1-dimethylethyl ester

To a solution of 327 mg (1.03 mmol) of Compound B in 10 mL of 5% acetic acid-methanol at room temperature was added 350 mg (0.686 mmol) of Compound 3E. The mixture was stirred for 5 minutes and then treated with 70 mg (1.03 mmol) of NaBH₃CN which was added in 3 portions over 1 hour. Additional aldehyde 200 mg) and NaBH₃CN (25 mg) were added to the reaction mixture and stirring was continued for 20 minutes. Water (2 mL) was added and the reaction mixture was concentrated *in vacuo*. Flash chromatography (25 x 150 mm silica), eluting with 50% ethyl acetate-hexane, 100% ethyl acetate, then 5% acetone-ethyl acetate, provided 231 mg of Compound C. MS 831⁺ (M+H)⁺.

### D. N²-[[(S)-1,2,3,4-Tetrahydro-2-[N-[[(cis)-3-mercaptocyclopentyl]methyl]-3-methyl-L-valyl]-3-isoquinolinyl]carbonyl]-L-glutamine, trifluoroacetate

To a solution of 208 mg of Compound C in 5 mL of methylene chloride at room temperature was added 150 µL of triethylsilane and 0.5 mL of TFA. The mixture was stirred for a total of 9 hours at room temperature and then concentrated. The residue was triturated with hexane (2 x 3 mL). Preparative HPLC (YMC S-10 ODS column, 30 X 500 mm; solvent A, 0.1% TFA in 90% water, 10 % methanol; solvent B, 0.1% TFA in 10% water, 90% methanol: 30-100% B in 60 minutes, flow rate 25 mL/min; UV monitored at 220 nm), followed by lyophyllization of appropriate fractions provided 107 mg of the title compound as a white solid. mp 90-103°C. MS 533⁺ (M+H)⁺.

### Example 7

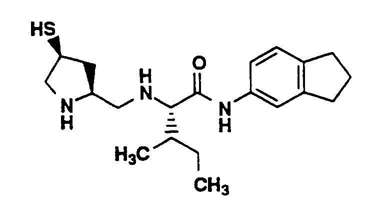

### [S-(R*,R*)]-N-(2,3-Dihydro-1H-inden-5-yl)-N²-[(4-mercapto-2-pyrrolidinyl)methyl]-L-isoleucinamide, trifluoroacetate

### A. [S-(R*, R*)]-N-[[1-[(1,1-Dimethylethoxy)carbonyl]-4-[(triphenylmethyl)thio]-2-pyrrolidinyl]methyl]-L-isoleucine, methyl ester

Compound 2D (2.6 g, 5.5 mmol) and L-isoleucine methyl ester hydrochloride (1.1g, 6.1 mmol), 3A molecular sieves (3g) and glacial acetic acid (0.2 mL) were stirred in methanol (60 mL). NaBH₃CN (350 mg, 5.5 mmol) was added portionwise over 30 minutes. The reaction mixture was stirred for 16 hours. The mixture was cooled to 0°C and saturated NaHCO₃ (50 mL) was slowly added. The mixture was concentrated and the product was extracted into ethyl acetate (2 x 100 mL). The combined ethyl acetate layer was washed with water (2 x 100 mL) and brine (100 mL), dried (MgSO₄), concentrated and chromatographed (silica gel, 5.0 X 15 cm, 40% ethyl acetate-hexanes). Fractions containing the desired product were combined and concentrated to yield Compound A as a clear oil (2.5 g). MS (M+H)⁺ 603⁺.

### B. [S-(R*, R*)]-N-[[1-[(1,1-Dimethylethoxy)carbonyl]-4-[(triphenylmethyl)thio]-2-pyrrolidinyl]methyl]-L-isoleucine

Compound A (1.0 g, 1.7 mmol) was dissolved in a mixture of methanol (8 mL) and THF (16 mL). 1M LiOH (10 mL) was added and the reaction mixture was stirred for 64 hours. The mixture was neutralized to pH 6 using 1M KHSO₄ (9.8 mL) and concentrated. The residue was dissolved in ethyl acetate (200 mL) and washed with water (100 mL). The layers were separated. The aqueous layer was acidified to pH 2 using 5N HCl and extracted with ethyl acetate (100 mL). The combined organic layer was washed with brine (100 mL), dried (MgSO₄) and concentrated to yield a white solid (900 mg). MS (M+H)⁺ 589⁺.

### C. [S-(R*, R*)]-N-(2,3-Dihydro-1H-inden-5-yl)-N²-[[1-[(1,1-dimethylethoxy)carbonyl]-4-[(triphenylmethyl)thio]-2-pyrrolidinyl] methyl]-L-isoleucinamide

Compound B (100 mg, 0.17 mmol), 1-ethyl-3-(3-dimethylaminopropyl)carbodiimide·HCl (39 mg, 0.20 mmol) and 1-hydroxybenzotriazole (27 mg, 0.20 mmol) were dissolved in DMF (2 mL). 5-Aminoindan (50 mg, 0.050 mL, 0.37 mmol) was added. The reaction mixture was stirred for 16 hours at room temperature, poured into water (10 mL) and extracted with ethyl acetate (60 mL). The organic layer was washed with water (3 x 70 mL) and brine (50 mL). The organic layer was dried (MgSO₄) and concentrated. Flash chromatography (silica gel, 1.5 x 15 cm, 40% ethyl acetate, 60% hexane) of the residue provided compound C as a clear oil (100 mg). MS (M+H)⁺ 704⁺.

### D. [S-(R*,R*)]-N-(2,3-Dihydro-1H-inden-5-yl)-N²-[(4-mercapto-2-pyrrolidinyl)methyl]-L-isoleucinamide, trifluoroacetate

Compound C (100 mg, 0.14 mmol) was dissolved in methylene chloride (5 mL), triethylsilane (0.075 mL, 55 mg, 0.47 mmol) and TFA (5 mL). The mixture was stirred for 40 minutes at room temperature. The mixture was concentrated, dissolved in methylene chloride (50 mL) and concentrated again. This latter procedure was repeated 3 times to yield the crude product as a white sticky solid. The crude solid was purified by preparative HPLC (YMC S-10 ODS column, 30 X 500 mm; solvent A, 0.1% TFA in 90% water, 10 % methanol; solvent B, 0.1% TFA in 10% water, 90% methanol: 20-70% B in 40 minutes, flow rate 20 mL/min; UV monitored at 220 nm). Fractions containing the desired product were combined and lyophilized to provide the title compound (35 mg). Purity > 99.9 % (YMC S3 ODS column, 6 X 150 mm, 220 nm, 1.5 mL/min, 0-100% B in A over 30 minutes (A, 0.2% phosphoric acid (H₃PO₄) in 90% H₂O, 10% methanol; B, 0.2% H₃PO₄ in 90% methanol, 10% H₂O, room temperature 22.14 min. HI>99.9 %), [α]_{D}25° = - 14.0° (c=0.1, methanol). MP 68-69°C, MS (M+H)⁺ 362⁺, ¹H-NMR (CD₃OD, 400 MHz) δ (ppm) 7.50 (1H, s), 7.27 (1H, d, J=6.0Hz), 7.19 (1H, d, J=7.7Hz)), 4.06 (1H, m), 4.03 (1H, m), 3.86 (1H, m), 3.45 (1H, m), 3.19 (2H, m), 2.89 (4H, m), 2.58 (2H, m), 2.08 (2H, quintet), 1.68 (2H, m), 1.27 (1H, m), 1.05 (3H, d, J=6.8Hz), 0.96 (3H, t).

### Example 8

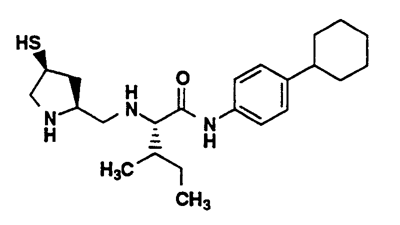

### [S-(R*,R*)]-N-(4-Cyclohexylphenyl)-N²-[(4-mercapto-2-pyrrolidinyl)methyl]-L-isoleucinamide, trifluoroacetate

### A. [S-(R*, R*)]-N-(4-Cyclohexylphenyl)-N²-[[1-[(1,1-dimethylethoxy)carbonyl]-4-[(triphenylmethyl)thio]-2-pyrrolidinyl]methyl]-L-isoleucinamide

Compound 7B (140 mg, 0.24 mmol) and 4-Cyclohexylaniline (84 mg, 48 mmol) were treated as in Example 7C except that the product was used without purification. Compound A was provided as a clear oil (150 mg). MS (M+H)⁺ 746⁺.

### B. [S-(R*,R*)]-N-(4-Cyclohexylphenyl)-N²-[(4-mercapto-2-pyrrolidinyl)methyl]-L-isoleucinamide, trifluoroacetate

Compound A (150 mg, 0.20 mmol ) was dissolved in methylene chloride (9 mL), triethylsilane (0.10 mL, 73 mg, 0.63 mmol) and TFA (1 mL), and was stirred for 4 hours at room temperature. The mixture was concentrated, dissolved in methylene chloride (50 mL) and concentrated again. This latter procedure was repeated 3 times to yield the crude product as a white sticky solid in quantitative yield. The crude solid was purified by preparative HPLC (YMC S-10 ODS column, 30 X 500 mm; solvent A, 0.1% TFA in 90% water, 10% acetonitrile; solvent B, 0.1% TFA in 10% water, 90% acetonitrile: 20-70% B in 40 minutes, flow rate 20 mL/min; UV monitored at 220 nm). Fractions containing the desired product were combined, concentrated and lyophilized to provide the title compound (35 mg), Purity >97 % (YMC S3 ODS column, 6 X 150 mm, 220 nm, 1.5 mL/min, 0-100% B in A over 30 minutes (A, 0.2% H₃PO₄ in 90% H₂O, 10% methanol; B, 0.2% H₃PO₄ in 90% methanol, 10% H₂O), room temperature 28.3 minutes. HI>97 %), MP 115-116°C, MS (M+H)⁺ 404⁺, ¹H-NMR (CD₃OD, 400 MHz) d (ppm) 7.40 (2H, d, J=8.6 Hz), 7.15 (2H, d, J=8.6 Hz), 4.19 (1H, m), 4.03 (1H, m), 3.67 (2H, m), 3.38 (2H, m), 3.17 (1H, m), 2.46 (2H, m), 2.06 (1H, m), 1.90-1.71 (4H, m), 1.63 (1H, m), 1.50-1.17 (6H, m), 1.04 (3H, d, J=6.4 Hz), 0.94 (3H, t).

### Example 9

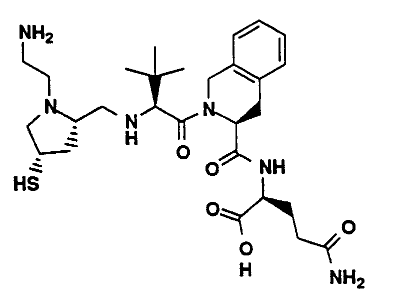

### N²[[(S)-2-[N-[[(2S-cis)-1-(2-Aminoethyl)-4-mercapto-2-pyrrolidinyl]methyl]-3-methyl-L-valyl]-1,2,3,4-tetrahydro-3-isoquinolinyl]carbonyl]-L-glutamine, trifluoroacetate

### A. N²-[[(S)-2-[N-[[(2S-cis)-1-[2-[[(1,1-Dimethylethoxy)carbonyl]amino]ethyl]-4-[(triphenylmethyl)thio]-2-pyrrolidinyl]-methyl]-3-methyl-L-valyl]-1,2,3,4-tetrahydro-3-isoquinolinyl]carbonyl]-L-glutamine, 1,1-dimethylethyl ester

Compound 3F (100 mg, 0.11 mmol) was dissolved in methylene chloride (10 mL) and TFA (10 mL), and stirred for 90 minutes at room temperature. The mixture was concentrated, dissolved in methylene chloride (30 mL) and concentrated again. This latter procedure was repeated three times to yield the amine as a clear glass in quantitative yield. This amine and N-Boc-glycinal (22 mg, 0.14 mmol) were dissolved in methanol (10 mL) and glacial acetic acid (0.1 mL). NaBH₃CN (6.7 mg, 0.11 mmol) was added portionwise over 15 minutes. The reaction mixture was stirred for 16 hours. The mixture was cooled to 0°C and saturated NaHCO₃ (10 mL) was slowly added. The mixture was concentrated and the product was extracted into ethyl acetate (70 mL). The ethyl acetate layer was washed with water (50 mL) and brine (50 mL). Then, the organic layer was dried (MgSO₄) and concentrated. Flash chromatography (silica gel, 4.1 x 15 cm, 95% methylene chloride, 5% methanol) of the residue provided Compound A as a clear oil (65 mg).
MS (M+H)⁺ 919⁺.

### B. N²[[(S)-2-[N-[[(2S-cis)-1-(2-Aminoethyl)-4-mercapto-2-pyrrolidinyl]methyl]-3-methyl-L-valyl]-1,2,3,4-tetrahydro-3-isoquinolinyl] carbonyl]-L-glutamine, trifluoroacetate

Compound A (65 mg, 0.071 mmol) was treated as in Example 1M to provide the title compound (30 mg), Purity > 97.9% (YMC S3 ODS column, 6 x 150 mm, 220 nm, 1.5 mL/min, 65°C; 0-100% B in A over 30 minutes (A, 0.2% H₃PO₄ in 90% H₂O, 10% methanol; B, 0.2% H₃PO₄ in 90% methanol, 10% water, RT 10.15 minutes. HI>67.3 % 30.6% exists as disulfide RT 13.40), [α]_{D}25° = - 46.0° (c=0.1, methanol), MP 119-120°C, MS (M+H)⁺ 577⁺, Analysis: Calculated for C₂₆H₄₄N₆O₅S· 3.95CF₃CO₂H,3.75H₂O: C, 39.38; H, 5.10; N, 7.67; Found: C, 39.10; H, 4.68; N, 7.75. ¹H-NMR (CD₃OD, 400 MHz) δ (ppm) 7.40-7.12 (4H, m), 5.12-4.94 (2H, m), 4.89-4.67 (2H, m), 4.58 (1H, m), 4.47 (1H, m), 4.12 (1H, m), 3.60-3.41 (1H, m), 3.37 (2H, m), 3.35-3.25(1H, m), 3.24-2.92 (4H, m), 2.86-2.63 (2H, m), 2.60-2.43 (2H, m), 2.40-2.17 (2H, m), 2.10-1.90 (2H, m), 1.23-1.16 (9H, m).

### Example 10

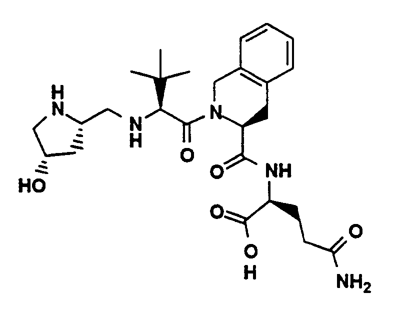

### N-2-[[(S)-1,2,3,4-Tetrahydro-2-[N-[(2-cis)-4-hydroxy-2-pyrrolidinyl]methyl]-3-methyl-L-valyl]-3-isoquinolinyl]carbonyl]-L-glutamine, trifluoroacetate

### A. (2S-cis)-2-[N-Methoxy-N-methyl amino]carbonyl-4-phenylmethoxy-1-pyrrolidinecarboxylic acid, 1,1-dimethylethyl ester

[S-(R*,R*)]-4-Phenylmethoxy-1,2-pyrrolidinedicarboxylic acid, 1,1-dimethylethyl ester (2.0 g, 6.2 mmol), N,O-Dimethylhydroxylamine hydrochloride (670 mg, 6.9 mmol), bromo-tris-pyrrolidinophosphonium hexafluorophosphate (3.2 g, 6.9 mmol) and 4-dimethylaminopyridine (300 mg, 2.5 mmol) were dissolved in methylene chloride (10 mL). N,N-Diisopropylethylamine (2.3 g, 3.0 mL, 17 mmol) was added dropwise over 15 minutes. The reaction mixture was stirred for 2 hours at room temperature, and then concentrated. Flash chromatography (silica gel, 5.0 X 15 cm, 40% ethyl acetate, 60% hexane) of the residue provided Compound A (1.9 g), as a clear oil.
MS (M+Na)⁺ 387⁺.

### B. (2S-cis)-2-Formyl-4-phenylmethoxy-1-pyrrolidinecarboxylic acid, 1,1-dimethylethyl ester

Compound A (1.0 g, 2.7 mmol) was dissolved in THF (10 mL) and the solution was cooled to 0°C. 1M LiAlH₄ in THF (2.7 mL, 2.7 mmol) was added dropwise over 15 minutes. The reaction mixture was stirred for 30 minutes at 0°C under nitrogen. Diethyl ether (200 mL) was added and the reaction mixture was quenched with dropwise addition of 1M KHSO₄ (60 mL) at 0°C. The mixture was stirred for an additional 30 minutes at 0°C and the layers were separated. The organic layer was washed with saturated NaHCO₃ (50 mL), 1M KHSO₄ (50 mL) and brine (50 mL). The organic layer was dried (MgSO₄) and concentrated to provide Compound B as a clear oil (830 mg) which was used immediately in the next step without further purification. MS (M+H)⁺ 306⁺.

### C. N²-[[(S)-2-[N-[[(2S-cis)-1-[(1,1-Dimethylethoxy)carbonyl]-4-(phenylmethoxy)-2-pyrrolidinyl]methyl]-3-methyl-L-valyl]-1,2,3,4-tetrahydro-3-isoquinolinyl]-carbonyl]-L-glutamine, 1,1-dimethylethyl

Compound B (1.0 g, 2.7 mmol) and Compound 3E (200 mg, 0.39 mmol) were dissolved in methanol (10 mL) and glacial acetic acid (0.2 mL). NaBH₃CN (25 mg, 0.39 mmol) was added portionwise over 15 minutes. The reaction mixture was stirred for 16 hours. The mixture was cooled to 0°C and saturated NaHCO₃ (30 mL) was slowly added. The mixture was concentrated and the product was extracted into ethyl acetate (100 mL). The ethyl acetate layer was washed with water (100 mL) and brine (100 mL). Then, the organic layer was dried (MgSO₄) and concentrated. Flash chromatography (silica gel, 4.1 X 15 cm, 95% methylene chloride, 5% methanol) of the residue provided Compound C (140 mg) as a clear oil. MS (M+H)⁺ 764⁺.

### D. N-2-[[(S)-1,2,3,4-Tetrahydro-2-[N-[(2-cis)-4-hydroxy-2-pyrrolidinyl]methyl]-3-methyl-L-valyl]-3-isoquinolinyl]carbonyl]-L-glutamine, tri-fluoroacetate

Compound C (140 mg, 0.18 mmol) was dissolved in absolute ethanol (5 mL) and then added to suspension of 10% palladium on carbon (14 mg) in absolute ethanol (5 mL). The mixture was stirred under hydrogen atmosphere (balloon) for 64 hours, filtered through a pad of Celite®, and concentrated to yield the free hydroxy compound as a clear oil (100 mg) [MS (M+H)⁺ 674⁺]. This clear oil was dissolved in methylene chloride (10 mL), TFA (10 mL) and triethylsilane (0.1 mL), and was stirred for 40 minutes at room temperature. The mixture was concentrated, dissolved in methylene chloride (30 mL) and concentrated. This latter procedure was repeated five times to yield the crude product as a white sticky solid in quantitative yield. The crude solid was purified by preparative HPLC (YMC S-10 ODS column, 30 X 500 mm; solvent A, 0.1% TFA in water; solvent B, 0.1% TFA in methanol: 30-50% B in 30 minutes, flow rate 20 mL/minute; UV monitored at 220 nm). Fractions containing the desired product were combined and lyophilized to provide the title compound (55 mg). M.P. 115-116°C, Purity > 95.7% (YMC S3 ODS column, 6 X 150 mm, 220 pm, 1.5 mL/min, 65 °C; 0-100% B in A over 30 minutes (A, 0.2% H₃PO₄ in 90% H₂O, 10% methanol; B, 0.2% H₃PO₄ in 90% methanol, 10% H₂O), RT 11.73 min. HI>95.7%), [α]_{D}²⁵ = - 17 ° (c=0.1, methanol), MS (M+H)⁺ 518⁺, Analysis Calculated for 4.10 CF₃CO₂H· 1.10 H₂O: C, 40.43; H, 5.66; N, 7.44; Found: C, 40.46; H, 4.68; N, 7.36. H-NMR (CD₃OD, 400 MHz) δ (ppm) 7.32-7.26 (4H, m), 5.05-4.92 (2H, m), 4.79-4.60 (2H, m), 4.51 (1H, m), 4.47 (1H, m), 4.22 (1H, m), 3.97 (1H, m), 3.48-3.33 (2H, m), 3.27-3.08 (2H, m), 2.96-2.80 (1H, m), 2.33 (1H, t), 2.25-2.00 (3H, m), 1.90-1.68 (2H, m), 1.17-1.12 (9H, 2s).

### Example 11

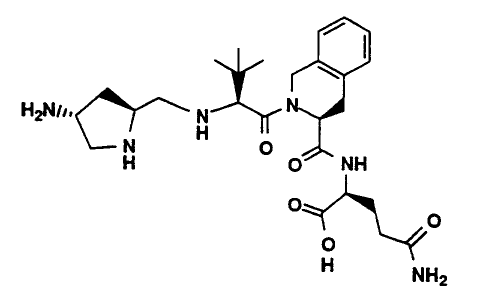

### N²-[[(S)-2-[N-[[(2S-trans)-4-Amino-2-pyrrolidinyl]methyl]-3-methyl-L-valyl]-1,2,3,4-tetrahydro-3-isoquinolinyl]carbonyl]-L-glutamine trifluoroacetate (1:3)

### A. (2S-trans)-4-[[(9-Fluorenylmethoxy)-carbonyl]amino]-2-[(methoxymethylamino)carbonyl]-1-pyrrolidinecarboxylic acid 1,1-dimethylethyl ester

To a solution of (2S-trans)-4-[[(9-Fluorenylmethoxy)carbonyl]amino]-1,2-pyrrolidinedicarboxylic acid 1-(1,1-dimethylethyl) ester (0.70 g, 1.5 mmol) (prepared analogously to the method described in Rosen, T., et al., Synthesis 40 (1988)), bromo-tris-pyrrolidinophosphonium hexafluorophosphate (700 mg, 1.5 mmol) and N,O-dimethylhydroxylamine hydrochloride dissolved in methylene chloride (20 mL) were added dimethylaminopyridine (20 mg, 0.16 mmol) and diisopropylethylamine (790 µL, 0.16 mmol). The solution was stirred for 6 hours and concentrated. The residue was chromatographed on a flash silica gel column (5 x 20 cm) eluting with 1:1/ethyl acetate/hexanes to afford 620 mg (84%) of compound A. MS: (M+H)⁺ 496⁺.

### B. (2S-trans)-4-[[(1,1-Dimethylethoxy)carbonyl]amino]-2-[(methoxymethylamino)carbonyl]-1-pyrrolidinecarboxylic acid 1,1-dimethylethyl ester

To a solution of compound A (550 mg, 1.1 mmol) in DMF (10 mL) under nitrogen was added potassium fluoride (450 mg, 7.8 mmol) followed by triethylamine (340 µL, 2.4 mmol) and t-butyloxycarbonyl anhydride (360 mg, 1.2 mmol). The solution was stirred for 16 hours under nitrogen. Water (50 mL) and ethyl acetate(50 mL) were added and the layers were separated. The organic layer was washed with brine, dried (MgSO₄) and concentrated. The resulting residue was chromatographed on a flash silica gel column (5 x 20cm) eluting with 1:1/ethyl acetate/hexanes to afford 418 mg (99%) of compound B. MS:(M+H)⁺ 374⁺.

### C. (2S-trans)-4-[[(1,1-Dimethylethoxy)-carbonyl]amino]-2-formyl-1-pyrrolidinecarboxylic acid 1,1-dimethylethyl ester

To a cooled (0^{o}C) solution of compound B (0.2 g, 0.54 mmol) in THF (20 mL) under nitrogen was slowly added 0.54 mL (0.54 mmol) of a 1M solution of lithium aluminum hydride in THF. Stirring was continued for 0.5 hours at 0^{o}C followed by addition of 1M potassium bisulfate (5 mL) and ethyl ether (10 mL). The solution was stirred at 0^{o}C for 1 hour under nitrogen. The layers were separated and the aqueous layer was washed with ethyl ether (20 mL). The pooled organic layer was dried (MgSO₄) and concentrated to give 170 mg (95%) of compound C.

### D. N²-[[(S)-2-[N-[[(2S-trans)-1-[(1,1-Dimethylethoxy)carbonyl]-4-[[1,1-dimethylethoxy)carbonyl]amino]-2-pyrrolidinyl]methyl]-3-methyl-L-valyl]-1,2,3,4-tetrahydro-3-isoquinolinyl]-carbonyl]-L-glutamine 1,1-dimethylethyl ester

A solution of the compound of Example 3D (0.17 g, 0.54 mmol) and glacial acetic acid (0.3 mL) in dry methanol (15 mL) was stirred for 10 minutes followed by the addition of sodium cyanoborohydride (29 mg, 0.47 mmol) in 4 aliquots over 2 hours. Stirring under nitrogen was continued for 16 hours. The solution was cooled to 0^{o}C and saturated aqueous sodium bicarbonate solution was added (2 mL) followed by ethyl acetate (60 mL) and water (50 mL). The layers were separated and the organic layer was washed with water, dried (MgSO₄) and concentrated to a residue. This was chromatographed on a flash silica gel column (5 x 20 cm) eluting with methylene chloride:methanol/20:1 to afford 160 mg (52%) of compound D. MS: (M+H)⁺ 774⁺, R_{f} 0.5 (methylene chloride:methanol/9:1, UV, ninhydrin, PMA).

### E. N²-[[(S)-2-[N-[[(2S-trans)-4-Amino-2-pyrrolidinyl]methyl]-3-methyl-L-valyl]-1,2,3,4-tetrahydro-3-isoquinolinyl]-carbonyl]-L-glutamine trifluoroacetate (1:3)

Compound D (150 mg, 0.19 mmol) was combined with trifluoroacetic acid (3 mL), methylene chloride (10 mL) and triethylsilane (0.4 mL, 2.5 mmol). The solution was stirred for 4 hours followed by concentration under vacuum. Methylene chloride was added and the solution was Concentrated under vacuum. Methanol (7 mL) was added and the solution was filtered through a 0.2 micron acrodisc filter. The resulting solution was concentrated and the residue was dissolved in 3 mL of a 50/50 mixture of 0.1% TFA in methanol and 0.1% TFA in water. This was applied to a YMC C18 column (S-10, ODS 30 x 500 mm) with monitoring at 220 nm, and HPLC purification was performed under the following conditions: Solvent A: 0.1% TFA in 90% water, 10% methanol; Solvent B: 0.1% TFA in 90% methanol, 10% water; 30-60% B in A over 60 minutes. Fractions containing the major peak were pooled and lyophilized to yield 140 mg (79%) of the title compound as a white powder. MS: (M+H)⁺ 517⁺, M.P. 118-120^{o}C.

| Analysis calculated for C₂₆H₄₀N₆O₅S·3.15C₂HF₃O₂, 2.85H₂O: | | | | |
|---|---|---|---|---|
| Calculated: | C 41.84, | H 5.31, | N 9.06, | F 19.06; |
| Found: | C 46.85, | H 4.92, | N 9.03, | F 19.06. |

### Example 12

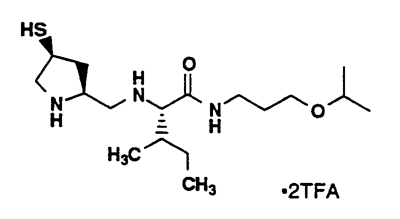

### (2S-cis)-N²-[(4-mercapto-2-pyrrolidinyl)methyl]-N-[3-(1-methylethoxy)propyl]-L-isoleucinamide, trifluoroacetate

### A. (2S-cis)-N-[[1-[(1,1-Dimethylethoxy)-carbonyl]-4-[(triphenylmethyl)thio]-2-pyrrolidinyl]methyl]-L-isoleucine 2-[(diphenylmethyl)amino]-2-oxoethyl ester

The compound of Example 2D (2.0 g, 4.23 mmol) and L-Isoleucine 2-[(diphenylmethyl)amino]-2-oxoethyl ester hydrochloride (2.7 g, 7.0 mmol) were dissolved in methanol (40 mL) and the mixture was stirred for 30 minutes. Glacial acetic acid (0.4 mL) was added and sodium cyanoborohydride (266 mg, 4.23 mmol) was added portionwise over 30 minutes. The reaction mixture was stirred for 16 hours. The mixture was cooled to 0°C, and saturated sodium bicarbonate (10 mL) was slowly added. The mixture was concentrated and the product was extracted into ethyl acetate (70 mL). The ethyl acetate layer was washed with water (50 mL) and brine (50 mL), dried (MgSO₄), concentrated and chromatographed (silica gel, 4.1 X 15 cm, 40 % ethyl acetate, 60 % hexanes). Fractions containing the desired product were combined and concentrated to yield compound A as a clear oil (1.8 g, 52%), MS: (M+H)⁺ 812⁺.

### B. (2S-cis)-N-[[1-[(1,1-Dimethylethoxy)-carbonyl]-4-[(triphenylmethyl)thio]-2-pyrrolidinyl]methyl]-L-isoleucine

Compound A (1.8 g, 2.2 mmol) was dissolved in dimethylformamide (20 mL) and 5N sodium hydroxide (2 mL). The reaction mixture was stirred for 2 hours. The mixture was poured into water (50 mL), washed with diethyl ether (2 X 20 mL), neutralized to pH 6 using 1M potassium bisulfate and extracted with ethyl acetate (50 mL). White particles precipitated out of the ethyl acetate layer and were filtered, air dried, recrystallized from ethyl acetate/hexane and dried under vacuum to yield compound B as a white solid (900 mg, 70%), MS: (M+H)⁺ 589⁺.

### C. (2S-cis)-N²-[[1-[(1,1-Dimethylethoxy)-carbonyl]-4-[(triphenylmethyl)thio]-2-pyrrolidinyl]methyl]-N-[3-(1-methylethoxy)propyl]-L-isoleucinamide

Compound B (100 mg, 0.17 mmol), 1-ethyl-3-(3-dimethylaminopropyl)carbodiimide·HCl (EDC) (33 mg, 0.17 mmol) and 1-hydroxybenzotriazole (HOBT) (23 mg, 0.17 mmol) were stirred in DMF (2 mL) at room temperature for 10 minutes. 3-Isopropoxypropylamine (40 mg, 0.05 mL, 0.34 mmol) was added. The reaction mixture was stirred for 16 hours at room temperature, poured into water (50 mL) and extracted with ethyl acetate (70 mL). The organic layer was washed with water (2 X 50 mL), saturated sodium bicarbonate (50 mL), water (50 mL) and brine (50 mL), dried (MgSO₄) and concentrated to yield compound C as a clear oil (116 mg, 99 %), MS: (M+H)⁺ 688⁺.

### D. (2S-cis)-N²-[(4-mercapto-2-pyrrolidinyl)-methyl]-N-[3-(1-methlyethoxy)propyl]-L-isoleucinamide, trifluoroacetate

Compound C (116 mg, 0.17 mmol ) was dissolved in methylene chloride (7 mL), triethylsilane (0.030 mL, 22 mg, 0.19 mmol) and trifluoroacetic acid (7 mL), and was stirred for 1 hour at room temperature. The mixture was concentrated, dissolved in methylene chloride (50 mL) and concentrated. This latter procedure was repeated 3 times to yield the crude product as a white sticky solid in quantitative yield. The crude solid was purified by preparative HPLC (YMC S-10 ODS column, 30 X 500 mm; solvent A, 0.1% TFA in 90% water, 10 % acetonitrile; solvent B, 0.1% TFA in 10% water, 90% acetonitrile: 20-30% B in 60 minutes, flow rate 20 mL/minute; UV monitored at 220 nm). Fractions containing the desired product were combined, concentrated and lyophilized to provide the title compound as a white solid (50 mg, 51%), Purity > 99% (YMC S3 ODS column, 6 X 150 mm, 220 nm, 1.5 mL/minute, 0-100% B in A over 30 minutes (solvent A, 0.2% H₃PO₄ in 90% water, 10 % methanol; solvent B, 0.2% H₃PO₄ in 10% water, 90% methanol, RT 14.13 min. HI>99 %)), MS: (M+H)⁺ 346⁺, ¹H-NMR (CD₃OD, 400 MHz) δ (ppm) 3.92 (1H, m), 3.71 (1H, m), 3.61-3.34 (7H, m), 3.25-3.12 (3H, m), 2.69 (1H, m), 1.86 (1H, m), 1.70 (3H, m), 1.65 (1H, m), 1.23 (1H, m), 1.14 (6H, d, J=6.0Hz), 1.00-0.94 (6H, m).

### Example 13

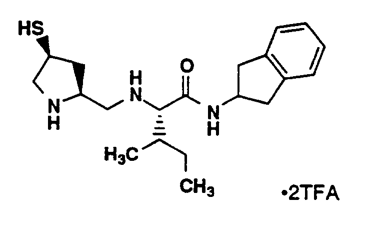

### (2S-cis)-N-(2,3-Dihydro-1H-inden-2-yl)-N²-[(4-mercapto-2-pyrrolidinyl)methyl]-L-isoleucinamide, trifluoroacetate

### A. (2S-cis)-N-(2,3-Dihydro-1H-inden-2-yl)-N²-[[1-[(1,1-dimethylethoxy)carbonyl]-4-[(triphenylmethyl)thio]-2-pyrrolidinyl]methyl]-L-isoleucinamide

Compound A (119 mg, 99%) was prepared from compound B of Example 12 with 2-Aminoindan hydrochloride (44 mg, 0.17 mmol) followed by N,N-diisopropylethylamine (44 mg, 0.060 mL, 0.34 mmol) using the process for preparing compound C of Example 12.
MS: (M+H)⁺ 704⁺.

### B. (2S-cis)-N-(2,3-Dihydro-1H-inden-2-yl)-N²-[(4-mercapto-2-pyrrolidinyl)methyl]-L-isoleucinamide, trifluoroacetate

The title compound (34 mg, 34%) was prepared from compound A using a process analogous to the process for preparing the title compound of Example 12.
MS: (M+H)⁺ 362⁺, ¹H-NMR (CD₃OD, 400 MHz) δ (ppm) 7.20-7.12 (4H, m), 4.63 (1H, m), 3.91 (1H, m), 3.66 (1H, m), 3.57 (1H, m), 3.47 (1H, m), 3.24 (2H, m), 3.19 (2H, m), 2.91-2.80 (3H, m), 2.67 (1H, m), 1.84 (1H, m), 1.78 (1H, m), 1.62 (1H, m), 1.20 (1H, m), 0.99-0.90 (6H, m).

### Example 14

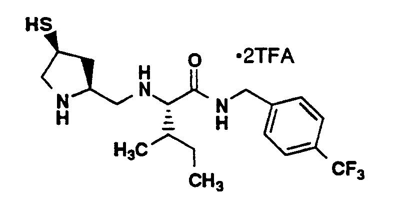

### (2S-cis)-N²-[(4-Mercapto-2-pyrrolidinyl)methyl]-N-[[4-(trifluoromethyl)phenyl]methyl]-L-isoleucinamide, trifluoroacetate

### A. (2S-cis)-N²-[[1-[(1,1-dimethylethoxy)-carbonyl]-4-[(triphenylmethyl)thiol-2-pyrrolidinyl]methyl]-N-[[4-(trifluoromethyl)-phenyl]methyl-L-isoleucinamide

Compound A (126 mg, 99%) was prepared from compound B of Example 12 with 4-(Trifluoromethyl)-benzylamine (60 mg, 0.050 mL, 0.34 mmol) using the process for preparing compound C of Example 12. MS: (M+H)⁺ 746⁺.

### B. (2S-cis)-N²-[(4-Mercapto-2-pyrrolidinyl)methyl]-N-[[4-(trifluoromethyl)phenyl]methyl]-L-isoleucinamide, trifluoroacetate

The title compound (45 mg, 42%) was prepared from compound A using a process analogous to the process for preparing the title compound of Example 12.
MS: (M+H)⁺ 380⁺, ¹H-NMR (CD₃OD, 400 MHz) δ (ppm) 7.63 (2H, d, J=8.1Hz), 7.50 (2H, d, J=8.1Hz), 7.21 (1H, m), 4.56-4.33 (2H, d of d, J=15.0Hz, 63.2Hz), 3.82 (1H, m), 3.66 (1H, m), 3.52 (1H, m), 3.41 (1H, m), 3.13 (2H, m), 3.04 (1H, m), 2.60 (1H, m), 1.79 (1H, m), 1.73 (1H, m), 1.68 (1H, m), 1.21 (1H, m), 0.97-0.90 (6H, m).

### Example 15

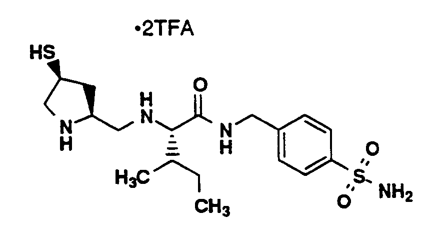

### (2S-cis)-N-[[4-(Aminosulfonyl)phenyl]methyl]-N²-[(4-mercapto-2-pyrrolidinyl)methyl]-L-isoleucinamide, trifluoroacetate

### A. (2S-cis)-N-[[4-(Aminosulfonyl)phenyl]-methyl]-N²-[[1-[(1,1-dimethylethoxy)-carbonyl]-4-[(triphenylmethyl)thio]-2-pyrrolidinyl]methyl]-L-isoleucinamide

Compound A (128 mg, 99%) was prepared from compound B of Example 12 with 4-(Aminomethyl)-benzenesulfonamide (38 mg, 0.17 mmol) followed by N,N-diisopropylethylamine (44 mg, 0.060 mL, 0.34 mmol) using the process for preparing compound C of Example 12.
MS: (M+H)⁺ 757⁺.

### B. (2S-cis)-N-[[4-(Aminosulfonyl)phenyl]-methyl]-N²-[(4-mercapto-2-pyrrolidinyl)methyl]-L-isoleucinamide, trifluoroacetate

The title compound (80 mg, 73%) was prepared from compound A using a process analogous to the process for preparing the title compound of Example 12.
MS (M+H)⁺ 415⁺, ¹H-NMR (CD₃OD, 400 MHz) δ (ppm) 7.86 (2H, m), 7.49 (2H, m), 4.58-4.41 (2H, d of d, J=15.0Hz, 50.0 Hz), 3.90 (2H, m), 3.69 (1H, m), 3.53 (2H, m), 3.15 (2H, m), 2.68 (2H, m), 2.63 (1H, m), 1.85 (1H, m), 1.70 (1H, m), 1.62 (1H, m), 1.20 (1H, m), 0.98-0.91 (6H, m).

### Example 16

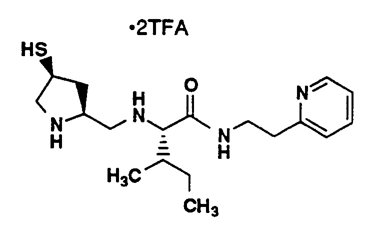

### (2S-cis)-N²-[(4-Mercapto-2-pyrrolidinyl)methyl]-N-[2-(2-pyridinyl)ethyl]-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-[2-(2-pyridinyl)-ethyl]-L-isoleucinamide

Compound A (116 mg, 99%) was prepared from compound B of Example 12 with 2-(2-Aminoethyl)-pyridine (42 mg, 0.041 mL, 0.34 mmol) using the process for preparing compound C of Example 12. MS: (M+H)⁺ 693⁺.

### B. (2S-cis)-N²-[(4-Mercapto-2-pyrrolidinyl)methyl]-N-[2-(2-pyridinyl)ethyl]-L-isoleucinamide, trifluoroacetate

The title compound (30 mg, 30%) was prepared using a process analogous to the process for preparing the title compound of Example 12.
MS: (M+H)⁺ 351⁺, ¹H-NMR (D₂O, 400 MHz) δ (ppm) 8.59 (1H, m), 8.43 (1H, m), 7.87 (2H, m), 4.00 (1H, m), 3.86 (1H, m), 3.74-3.62 (2H, m), 3.59-3.41 (2H, m), 3.30-3.12 (4H, m), 2.69 (1H, m), 1.81 (1H, m), 1.65 (1H, m), 1.21 (1H, m), 0.98 (1H, m), 0.73 (6H, m).

### Example 17

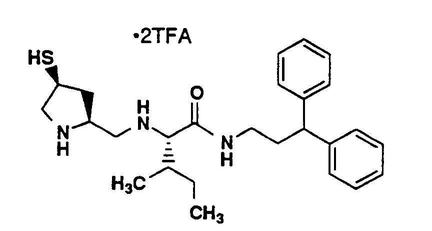

### (2S-cis)-N-(3,3-Diphenylpropyl)-N²-[(4-mercapto-2-pyrrolidinyl)methyl]-L-isoleucinamide, trifluoroacetate

### A. (2S-cis)-N²-[[1-[(1,1-Dimethylethoxy)-carbonyl]-4-[(triphenylmethyl)thio)-2-pyrrolidinyl]methyl]-N-(3,3-diphenylpropyl)-L-isoleucinamide

Compound A (132 mg, 99%) was prepared from compound B of Example 12 with 3,3-Diphenyl-propylamine (36 mg, 0.17 mmol) followed by N,N-diisopropylethylamine (22 mg, 0.030 mL, 0.17 mmol) using the process for preparing compound C of Example 12.
MS: (M+H)⁺ 782⁺.

### B. (2S-cis)-N-(3,3-Diphenylpropyl)-N²-[(4-mercapto-2-pyrrolidinyl)methyl]-L-isoleucinamide, trifluoro-acetate

The title compound (36 mg, 32%) was prepared from Compound A using a process analogous to the process for preparing the title compound of Example 12.
MS: (M+H)⁺ 405⁺, ¹H-NMR (CD₃OD, 400 MHz) δ (ppm) 7.26 (8H, m), 7.15 (2H, m), 4.00 (1H, m), 3.89 (1H, m), 3.65 (1H, m), 3.52 (1H, m), 3.36 (1H, m), 3.27 (1H, m), 3.12 (3H m), 3.01 (1H, m), 2.62 (1H, m), 2.28 (2H, m), 1.98 (1H, m), 1.69 (2H, m), 1.23 (1H, m), 0.97 (6H, m).

### Example 18

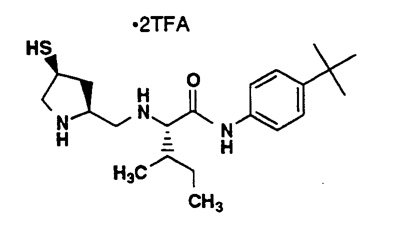

### (2S-cis)-N-[4-(1,1-Dimethylethyl)phenyl]-N²-[(4-mercapto-2-pyrrolidinyl)methyl]-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N-[4-(1,1-dimethylethyl)phenyl]-L-isoleucinamide

Compound A (122 mg, 99%) was prepared from compound B of Example 12 with 4-t-Butylaniline (25 mg, 0.027 mL, 0.17 mmol) followed by N,N-diisopropylethylamine (22 mg, 0.030 mL, 0.17 mmol) using the process for preparing compound C of Example 12.
MS: (M+H)⁺ 720⁺.

### B. (2S-cis)-N-[4-(1,1-Dimethylethyl)phenyl]-N²-[(4-mercapto-2-pyrrolidinyl)methyl]-L-isoleucinamide, trifluoroacetate

The title compound (17 mg, 17%) was prepared from compound A using a process analogous to the process for preparing the title compound of Example 12 .
MS: (M+H)⁺ 378⁺, ¹H-NMR (CD₃OD, 400 MHz) δ (ppm) 7.40 (2H, m), 7.29 (2H, m), 3.74 (1H, m), 3.61 (1H, m), 3.45 (1H, m), 3.38 (1H, m), 3.07 (1H, m), 3.02 (2H, m), 2.55 (1H, m), 1.78 (1H, m), 1.67 (2H, m), 1.21 (9H, s), 1.19 (1H, m), 0.96 (3H, d, J=6.8Hz), 0.88 (3H, t J=7.5Hz).

### Example 19

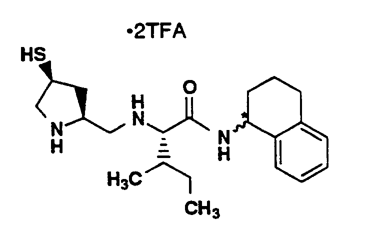

### (2S-cis)-N²-[(4-Mercapto-2-pyrrolidinyl)methyl]-N-(1,2,3,4-tetrahydro-1-naphthalenyl)-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-(1,2,3,4-tetrahydro-1-naphthalenyl)-L-isoleucinamide

Compound A (120 mg, 99%) was prepared from compound B of Example 12 with 1,2,3,4-Tetrahydro-1-naphthylamine (25 mg, 0.024 mL, 0.17 mmol) using the process for preparing compound C of Example 12. MS: (M+H)⁺ 718⁺.

### B. (2S-cis-)-N²-[(4-Mercapto-2-pyrrolidinyl)methyl]-N-(1,2,3,4-tetrahydro-1-naphthalenyl)-L-isoleucinamide, trifluoroacetate

The title compound (60 mg, 58%) was prepared from compound A using a process analogous to the process for preparing the title compound of Example 12.
MS: (M+H)⁺ 376 ⁺, ¹H-NMR (CD₃OD, 400 MHz) δ (ppm) 7.20-7.12 (4H, m), 5.17 (1H, m), 3.91 (1H, m), 3.70 (1H, m), 3.55 (1H, m), 3.48 (1H, m), 3.21-3.16 (3H, m), 2.90-2.64 (3H m), 2.09-1.63 (7H, m), 1.21 (1H, m), 1.05 (3H, d of d, J=6.9Hz, 15.4 Hz), 0.96 (3H, t, J=7.6Hz).

### Example 20

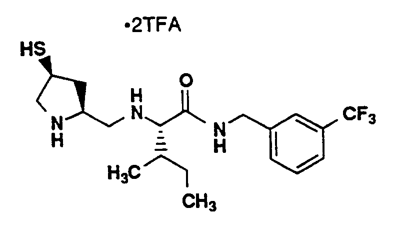

### (2S-cis)-N²-[(4-Mercapto-2-pyrrolidinyl)methyl]-N-[[3-(trifluoromethyl)phenyl]methyl]-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N-[[3-(trifluoromethyl)phenyl]methyl]-L-isoleucinamide

Compound A (126 mg, 99%) was prepared from compound B of Example 12 with 3-Trifluoromethyl-benzylamine (60 mg, 0.050 mL, 0.34 mmol) using the process for preparing compound C of Example 12. MS: (M+H)⁺ 746⁺.

### B. (2S-cis)-N²-[(4-Mercapto-2-pyrrolidinyl)-methyl]-N-[[3-(trifluoromethyl)phenyl]-methyl]-L-isoleucinamide, trifluoroacetate

The title compound (37 mg, 36%) was prepared from compound A using a process analogous to the process for preparing the title compound of Example 12.
MS: (M+H)⁺ 404⁺, ¹H-NMR (CD₃OD, 400 MHz) δ (ppm) 7.62-7.50 (4H, m), 4.59-4.40 (2H, m), 3.73 (1H, m), 3.67 (1H, m), 3.52 (1H, m), 3.24 (1H, m), 3.11 (1H, m), 2.93 (2H, m), 2.56 (1H, m), 1.77-1.59 (3H, m), 1.19 (1H, m), 0.95-0.88 (6H, m).

### Example 21

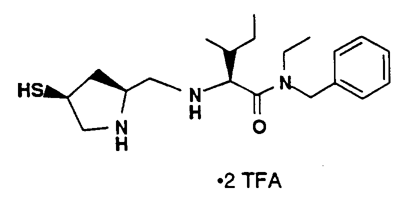

### (2S-cis)-N-Ethyl-N²-[(4-mercapto-2-pyrrolidinyl)methyl]-N-(phenylmethyl)-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-ethyl-N-(phenylmethyl)-L-isoleucinamide

Compound A (85 mg, 71%) was prepared from compound B of Example 12 with ethylbenzylamine (230 mg, 1.7 mmol) using the process for preparing compound C of Example 12.
MS: (M+H)⁺ 706⁺.

### B. (2S-cis)-N-Ethyl-N²-[(4-mercapto-2-pyrrolidinyl]methyl]-N-(phenylmethyl)-L-isoleucinamide, trifluoroacetate

The title compound (18 mg, 27%) was prepared from compound A using a process analogous to the process for preparing the title compound of Example 12.
MS: (M+H)⁺ 364⁺, ¹H-NMR(CD₃OD, 400 MHz) δ (ppm) 7.39 (5H, m), 4.82(2H, m), 3.73 (5H, m), 3.20 (5H, m), 2.69 (1H, m), 1.84 (2H, m) 1.32(4H, m), 1.15(3H, t, J=10.4 Hz),1.02 (3H, m).

### Example 22

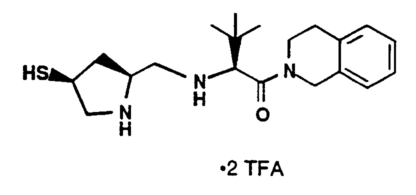

### [2S-[2α(R*),4α]]-1,2,3,4-Tetrahydro-2-[2-[[(4-mercapto-2-pyrrolidinyl)methyl]amino]-3,3-dimethyl-1-oxobutyl]isoquinoline, trifuloroacetate

### A. (S)-2-[2-[[(1,1-Dimethylethoxy)-carbonyl]amino]-3,3-dimethyl-1-oxobutyl]-1,2,3,4-tetrahydro-2-isoquinoline

N-[(1,1-Dimethylethoxy)carbonyl]-3-methyl-L-valine (0.69 g, 3 mmol) was combined with 1,2,3,4-tetrahydroisoquinoline (704 mg, 4 mmol), bromo-tris-pyrrolidino-phosphonium hexafluorophosphate (1.4 g, 3 mmol), dimethylaminopyridine (37 mg, 0.3 mmol) and diisopropylethylamine (0.52 mL, 387 mg, 3 mmol) in methylene chloride (20 mL) under argon. This was stirred for 16 hours, and then methylene chloride (30 mL) and 1 M potassium hydrogen sulfate (40 mL) were added. The layers were separated and the organic layer was washed with 1 M potassium hydrogen sulfate (2 x 30 mL), water (2 x 20 mL), half saturated sodium bicarbonate (3 x 30 mL) and brine (1 x 30 mL). The organic layer was dried (MgSO₄) and concentrated under vacuum. The residue was chromatographed (silica gel, 7.1 X 20 cm, 1:3-2:1/ethyl acetate:hexane) and fractions containing the desired compound were collected and concentrated to obtain compound A (1.03 g, 99%) as a clear glass. MS: (M+H)⁺ 347⁺, ¹H-NMR(CDCl₃, 270 MHz) δ (ppm) 7.12 (4H, m), 5.51 (1H, m) 4.68 (3H, m) , 3.81 (3H, m), 2.86 (2H, m), 1.37 (9H,s),0.94 (9H, 2s).

### B. (S)-2-(2-Amino-3,3-dimethyl-1-oxobutyl)-1,2,3,4-tetrahydro-2-isoquinoline hydrochloride

Compound A (513 mg, 1.3 mmol) was added to methylene chloride (5 mL) and 4N HCl/dioxane (10 mL). The mixture was stirred for 2 hours and then concentrated under vacuum. Ethyl ether was added and the precipitate was filtered, washed with ethyl ether and dried under vacuum to give 1.12g (92%, 2.42 mmol) of compound B as a white crystalline solid. MS: (M+H)⁺ 247⁺.

### C. [2S-[2α(R*),4α]]-2-[2-[[[1-[(1,1-Dimethylethoxy)carbonyl]-4-[(triphenylmethyl)thio]-2-pyrrolidinyl]methyl]amino]-3,3-dimethyl-1-oxobutyl]isoquinoline

A solution of compound B (420 mg, 1.5 mmol), compound D from Example 2 (310 mg, 0.66 mmol), glacial acetic acid (0.3 mL) and 3Ȧ molecular sieves (0.4 g) in dry methanol (15 mL) was stirred for 10 minutes and then NaBH₃CN (95 mg, 1.5 mmol) was added in 4 aliquots over 2 hours. Stirring under nitrogen was continued for 16 hours. The solution was cooled to 0^{o}C and saturated aqueous sodium bicarbonate solution was added (2 mL) followed by ethyl acetate (60 mL) and water (50 mL). The layers were separated and the organic layer was washed with water, dried (MgSO₄) and concentrated to a residue. This was chromatographed on a flash silica gel column (5 x 20 cm) eluting with methylene chloride:methanol/9:1 to afford 428 mg (92%) of compound C as a slightly yellow glass. MS: (M+H)⁺ 704⁺.

### D. [2S-[2α(R*),4α]]-1,2,3,4-Tetrahydro-2-[2-[[(4-mercapto-2-pyrrolidinyl)methyl]amino]-3,3-dimethyl-1-oxobutyl]isoquinoline, trifuloroacetate

To a solution of compound C (180 mg, 0.26 mmol) in methylene chloride (5 mL), was added triethylsilane (0.041 mL, 30 mg, 0.26 mmol) and trifluoroacetic acid (10 mL). The solution was stirred for 3 hours and concentrated under vacuum. The resulting yellow residue was triturated with hexanes and the solvent was removed. The residue was dissolved in 3 mL of a 50/50 mixture of 0.1%TFA in acetonitrile and 0.1%TFA in water. This was applied to a YMC C18 column (S-10, ODS 30 x 500 mm) with monitoring at 220 nm, and HPLC purification was performed under the following conditions; Solvent A; 0.1%TFA in 90% water, 10% acetonitrile, Solvent B; 0.1%TFA in 90% acetonitrile, 10% water; 10-40%B in A over 60 minutes. Fractions containing the major peak were pooled and lyophilized to yield 114 mg (75%) of the title compound as a white powder. MS: (M+H)⁺ 362⁺, ¹H-NMR(CD₃OD, 270 MHz) δ (ppm) 7.35 (4H, s), 5.01(3H, m) 3.95 (5H, m), 3.27(1H, m), 3.08(4H, m), 2.72(1H, m), 1.80 (1H, m), 1.21(9H, 2s).

### Example 23

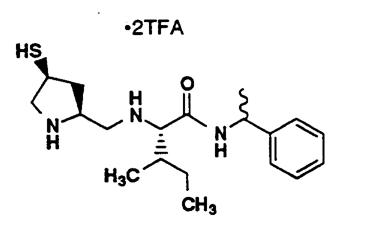

### (2S-cis-)-N²-[(4-Mercapto-2-pyrrolidinyl)methyl]-N-(1-phenylethyl)-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N-(1-phenylethyl)-L-isoleucinamide

Compound A (117 mg, 99%) was prepared from compound B of Example 12 with (±)-1-phenylethylamine (21 mg, 0.17 mmol) followed by N,N-diisopropylethylamine (44 mg, 0.060 mL, 0.34 mmol) using the process for preparing compound C of Example 12.
MS: (M+H)⁺ 692⁺.

### B. (2S-cis-)-N²-[(4-Mercapto-2-pyrrolidinyl)methyl]-N-(1-phenylethyl)-L-isoleucinamide, trifluoroacetate

The title compound (47 mg, 48%) was prepared from compound A using a process analogous to the process for preparing the title compound of Example 12.
MS: (M+H)⁺ 350⁺, ¹H-NMR (CD₃OD, 400-MHz) δ (ppm) 7.35-7.20 (5H, m), 5.05 (1H, m), 3.95 (0.5H, m), 3.78 (0.5H, m), 3.71 (0.5H, m), 3.63 (0.5H, m), 3.57 (2H, m), 3.45 (0.5H m), 3.25-3.08 (2H, m), 2.94 (0.5H, m), 2.69 (0.5H, q), 2.53 (0.5H, q), 1.94-1.68 (2.5H, m), 1.60 (0.5H, m), 1.49 (1.5H, d, J=5.1Hz), 1.47 (1.5H, d, J=5.1Hz), 1.28 (0.5H m), 1.10 (0.5H, m), 1.04-0.96 (3H, m), 0.87 (3H, m).

### Example 24

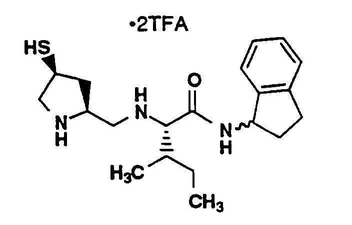

### (2s-cis)-N-(2,3-Dihydro-1H-inden-1-yl)-N²-[(4-mercapto-2-pyrrolidinyl)methyl]-L-isoleucinamide, trifluoroacetate

### A. (2S-cis)-N-(2,3-Dihydro-1H-inden-1-yl)-N²-[[1-[(1,1-dimethylethoxy)carbonyl]-4-[(triphenylmethyl)thio]-2-pyrrolidinyl]-methyl]-L-isoleucinamide

Compound A (119 mg, 99%) was prepared from compound B of Example 12 with 1-Aminoindan (23 mg, 0.022 mL, 0.17 mmol) followed by N,N-diisopropyl-ethylamine (22 mg, 0.030 mL, 0.17 mmol) using the process for preparing compound C of Example 12.
MS: (M+H)⁺ 704⁺.

### B. (2S-cis)-N-(2,3-Dihydro-1H-inden-1-yl)-N²-[(4-mercapto-2-pyrrolidinyl)methyl]-L-isoleucinamide, trifluoroacetate

The title compound (28 mg, 28%) was prepared from compound A using a process analogous to the process for preparing the title compound of Example 12.
18.77 min. (49.5 %), HI>99 %), MS: (M+H)⁺ 362⁺, ¹H-NMR (CD₃OD, 400 MHz) δ (ppm) 7.40-7.32 (2H, m), 7.01-6.96 (2H, m), 4.60 (2H, d, J=14.1Hz), 4.42 (2H, d, J=14.1Hz), 3.81 (1H, m), 3.66 (1H, m), 3.52 (1H, m), 3.39 (1H, m), 3.16 (3H, m), 3.06 (1H, m), 2.65 (1H, m), 1.82-1.66 (2H, m), 1.60 (1H, m), 1.15 (1H, m), 0.92-0.87 (6H, m).

### Example 25

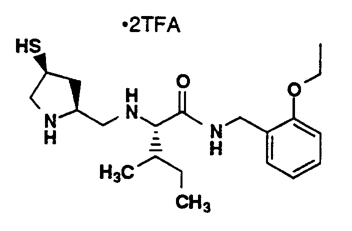

### (2S-cis)-N-[(2-Ethoxyphenyl)methyl]-N²-[(4-mercapto-2-pyrrolidinyl)methyl]-L-isoleucinamide, trifluoroacetate

### A. (2S-cis)-N²-[[1-[(1,1-Dimethyl-ethoxy)carbonyl]-4-[(triphenylmethyl)thio]-2-pyrrolidinyl]methyl]-N-[(2-ethoxyphenyl)methyl]-L-isoleucinamide

Compound A (122 mg, 99%) was prepared from compound B of Example 12 with 2-Ethoxybenzylamine (26 mg, 0.025 mL, 0.17 mmol) followed by N,N-diisopropylethylamine (22 mg, 0.030 mL, 0.17 mmol) using the process for preparing compound C of Example 12.
MS: (M+H)⁺ 722⁺.

### B. (2S-cis)-N-[(2-Ethoxyphenyl)methyl]-N²-[(4-mercapto-2-pyrrolidinyl)methyl]-L-isoleucinamide, trifluoroacetate

The title compound (37 mg, 36%) was prepared from compound A using a process analogous to the process for preparing the title compound of Example 12.
MS: (M+H)⁺ 380⁺, ¹H-NMR (CD₃OD, 400 MHz) δ (ppm) 7.24 (2H, m), 6.94 (1H, d, J=8.1Hz), 6.88 (1H, m), 4.50 (1H, d, J=14.5Hz), 4.35 (1H, d, J=14.5Hz), 4.08 (2H, q, J=6.8Hz, 14.1Hz), 3.78 (1H, m), 3.65 (2H, m), 3.51 (1H, m), 3.11 (1H, m), 3.00 (2H, m), 2.60 (1H, m), 1.80-1.70 (3H, m), 1.42 (3H, t, J=7.0Hz), 1.18 (1H, m), 0.94-0.89 (6H, m).

### Example 26

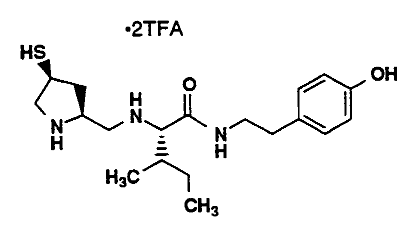

### (2S-cis)-N-[2-(4-Hydroxyphenyl)ethyl]-N²-[(4-mercapto-2-pyrrolidinyl)methyl]-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N-[2-(4-hydroxyphenyl)ethyl]-L-isoleucinamide

Compound A (120 mg, 99%) was prepared from compound B of Example 12 with tyramine (23 mg, 0.17 mmol) followed by N,N-diisopropylethylamine (22 mg, 0.030 mL, 0.17 mmol) using the process for preparing compound C of Example 12. MS: (M+H)⁺ 708⁺.

### B. (2S-cis)-N-[2-(4-Hydroxyphenyl)ethyl]-N²-[(4-mercapto-2-pyrrolidinyl)methyl]-L-isoleucinamide, trifluoroacetate

The title compound (43 mg, 43%) was prepared from compound A using a process analogous to the process for preparing the title compound of Example 12.
MS: (M+H)⁺ 366⁺, ¹H-NMR (CD₃OD, 400 MHz) δ (ppm) 7.05 (1H, s), 7.03 (1H, d, J=2.1Hz), 6.71 (1H, d, J=1.7Hz), 6.69 (1H, d, J=2.9Hz), 3.82 (1H, m), 3.67 (1H, m), 3.56-3.46 (3H, m), 3.44 (1H, m), 3.15 (2H, m), 2.93 (1H, m), 2.74 (2H, m), 2.64 (1H, m), 1.72 (1H, m), 1.68 (1H, m), 1.53 (1H, m), 1.11 (1H, m), 0.90 (6H, m).

### Example 27

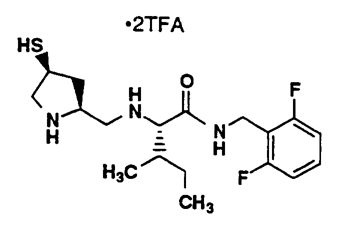

### (2S-cis)-N-[(2,6-Difluorophenyl)methyl]-N²-[(4-mercapto-2-pyrrolidinyl)methyl]-L-isoleucinamide, trifluoroacetate

### A. (2S-cis)-N-[(2,6-Difluorophenyl)methyl]-N²-[[1-[(1,1-dimethylethoxy)carbonyl]-4-[(triphenylmethyl)thio]-2-pyrrolidinyl]methyl]-L-isoleucinamide

Compound A (120 mg, 99%) was prepared from compound B of Example 12 with 2,6-Difluorobenzylamine (24 mg, 0.17 mmol) followed by N,N-diisopropylethylamine (22 mg, 0.030 mL, 0.17 mmol) using the process for preparing compound C of Example 12.
MS: (M+H)⁺ 714⁺.

### B. (2S-cis)-N-[(2,6-Difluorophenyl)methyl]-N²-[(4-mercapto-2-pyrrolidinyl)methyl]-L-isoleucinamide, trifluoroacetate

The title compound (38 mg, 37%) was prepared from compound A using a process analogous to the process for preparing the title compound of Example 12.
MS: (M+H)⁺ 372⁺, ¹H-NMR (CD₃OD, 400 MHz) δ (ppm) 7.021-7.06 (3H, m), 5.36 (1H, m), 3.77 (1H, m), 3.59 (1H, m), 3.45 (1H, m), 3.27 (1H, m), 3.06 (2H, m), 2.93 (1H, m), 2.80 (1H, m), 2.62-2.35 (1H, m), 1.88-1.54 (3H, m), 1.12 (1H, m), 0.96-0.91 (3H, d of d, J=6.8Hz, 12.8hz), 0.86 (3H, t, J=7.4Hz).

### Example 28

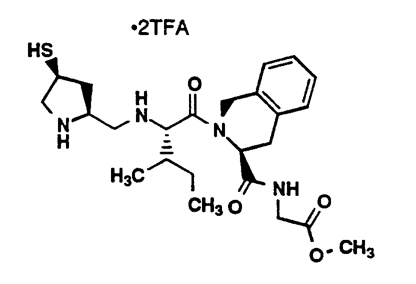

### N-[[(S)-1,2,3,4-Tetrahydro-2-[N-[[(2S-cis)-4-mercapto-2-pyrrolidinyl]methyl]-L-isoleucyl]-3-isoquinolinyl]carbonyl]glycine, methyl ester, trifluoroacetate

### A. (S)-N-[[2-[(1,1-Dimethylethoxy)carbonyl]-1,2,3,4-tetrahydro-3-isoquinolinyl]-carbonyl]glycine methyl ester

(S)-3,4-Dihydro-2,3(1H)-isoquinoline-dicarboxylic acid 2-(1,1-dimethylethyl) ester (1.00 g, 3.61 mmol), 1-ethyl-3-(3-dimethylamino-propyl)carbodiimide·HCl (761 mg, 3.97 mmol) and 1-hydroxybenzotriazole (536 mg, 3.97 mmol) were stirred in DMF (10 mL) at room temperature for 20 minutes. Glycine methyl ester hydrochloride (500 mg, 3.97 mmol) was added followed by N,N-diisopropylethylamine (982 mg, 1.33 mL, 7.58 mmol). The reaction mixture was stirred for 16 hours at room temperature, poured into water (100 mL) and extracted with ethyl acetate (100 mL). The organic layer was washed with water (3 X 100 mL), saturated NaHCO₃ (100 mL), water (100 mL) and brine (100 mL), dried (MgSO₄) and concentrated to yield compound A as a clear oil (1.25 g, 99 %), MS: (M+H)⁺ 349⁺.

### B. N-[[(S)-2-[N-[[(2S-cis)-1-[(1,1-Dimethylethoxy)carbonyl]-4-[(triphenylmethyl)thio]-2-pyrrolidinyl]methyl]-L-isoleucyl]-1,2,3,4-tetrahydro-3-isoquinolinyl]carbonyl]glycine methyl ester

Compound A (100 mg, 0.28 mmol) was stirred in dimethyl sulfide (0.2 mL) and 4N HCl in dioxane (10 mL) for 40 minutes. The mixture was concentrated, dissolved in methylene chloride (50 mL) and concentrated. This latter procedure was repeated five times to yield the amine as a clear glass. This amine and N,N-diisopropylethylamine (22 mg, 0.030 mL, 0.17 mmol) were dissolved in DMF (3 mL) and added to a stirred solution of compound B of Example 12, 1-ethyl-3-(3-dimethyl-aminopropyl)carbodiimide·HCl (33 mg, 0.17 mmol) and 1-hydroxybenzotriazole (23 mg, 0.17 mmol) in DMF (1 mL) at room temperature. The reaction mixture was stirred for 16 hours at room temperature, poured into water (50 mL) and extracted with ethyl acetate (70 mL). The organic layer was washed with water (2 X 50 mL), saturated NaHCO₃ (50 mL), water (50 mL) and brine (50 mL), dried (MgSO₄) and concentrated to yield compound B as a clear oil (100 mg, 72 %), MS: (M+H)⁺ 819⁺.

### C. N-[[(S)-1,2,3,4-Tetrahydro-2-[N-[[(2S-cis)-4-mercapto-2-pyrrolidinyl]methyl]-L-isoleucyl]-3-isoquinolinyl]carbonyl]glycine, methyl ester,trifluoroacetate

Compound B (100 mg, 0.12 mmol) was dissolved in methylene chloride (7 mL), triethylsilane (0.030 mL, 22 mg, 0.19 mmol) and trifluoroacetic acid (7 mL), and was stirred for 1 hour at room temperature. The mixture was concentrated, dissolved in methylene chloride (50 mL) and concentrated. This latter procedure was repeated 3 times to yield the crude product as a white sticky solid in quantitative yield. The crude solid was purified by preparative HPLC (YMC S-10 ODS column, 30 X 500 mm; solvent A, 0.1% TFA in 90% water, 10 % acetonitrile; solvent B, 0.1% TFA in 10% water, 90% acetonitrile: 20-30% B in 40 minutes, flow rate 20 mL/minute; UV monitored at 220 nm). Fractions containing the desired product were combined, concentrated and lyophilized to provide the title compound as a white solid (28 mg, 33 %), Purity > 99 % (YMC S3 ODS column, 6 X 150 mm, 220 nm, 1.5 mL/minute, 0-100% B in A over 30 minutes (solvent A, 0.2% H₃PO₄ in 90% water, 10 % methanol; solvent B, 0.2% H₃PO₄ in 10% water, 90% methanol, RT 15.83 minutes, HI>99 %), MS: (M+H)⁺ 477⁺, ¹H-NMR (CD₃OD, 400 MHz) δ (ppm) 7.13 (4H, m), 4.74-4.57 (4H, m), 3.92-3.62 (4H, m), 3.58 (3H, m), 3.41 (1H, m), 3.11-2.94 (4H, m), 2.89 (1H, m), 2.58-2.41 (1H, m), 1.90-1.69 (1H, m), 1.69-1.50 (2H, m), 1.13 (1H, m), 1.04 (3H, m), 0.85 (3H, m).

### Example 29

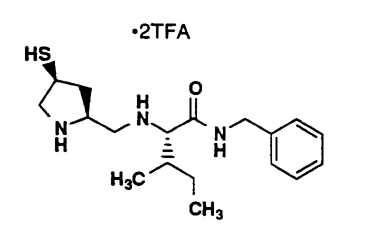

### (2S-cis)-N²-[(4-Mercapto-2-pyrrolidinyl)methyl]-N-(phenylmethyl)-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N-(phenylmethyl)-L-isoleucinamide

Compound A (114 mg, 99%) was prepared from compound B of Example 12 with benzylamine (18 mg, 0.019 mL, 0.17 mmol) followed by N,N-diisopropylethylamine (22 mg, 0.030 mL, 0.17 mmol) using the process for preparing compound C of Example 12.
MS: (M+H)⁺ 678⁺.

### B. (2S-cis)-N²-[(4-Mercapto-2-pyrrolidinyl)methyl]-N-(phenylmethyl)-L-isoleucinamide, trifluoroacetate

The title compound (45 mg, 47%) was prepared from compound A using a process analogous to the process for preparing the title compound of Example 12.
MS: (M+H)⁺ 336⁺, ¹H-NMR (CD₃OD, 400 MHz) δ (ppm) 7.28-7.21 (5H, m), 4.39-4.23 (2H, d of d, J=14.9Hz, 50.0 Hz), 3.85 (1H, m), 3.63-3.56 (2H, m), 3.49 (1H, m), 3.34 (1H, m), 3.14 (2H, m), 2.58 (1H, m), 1.83 (1H, m), 1.62 (1H, m), 1.39 (1H, m), 1.11 (1H, m), 0.81-0.85 (6H, m).

### Example 30

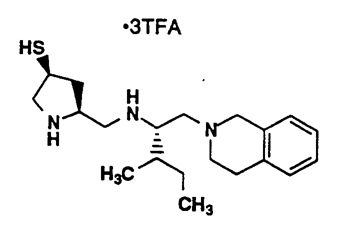

### [2S-[2α(2R*,3R*),4α]]-1,2,3,4-Tetrahydro-2-[2-[[(4-mercapto-2-pyrrolidinyl)methyl]amino]-3-methylpentyl]isoquinoline, trifluoroacetate

### A. [S-(R*,R*)]-[[1-[(Methoxymethyl-amino)carbonyl]-2-methylbutyl]amino]carbamic acid 1,1-dimethylethyl ester

N-[(1,1-Dimethylethoxy)carbonyl]-L-isoleucine (2.0 g, 8.33 mmol) and N,O-dimethylhydroxylamine·HCl (893 mg, 9.16 mmol), bromo-tris pyrrolidino-phosphonium hexafluorophosphate (2.10 g, 4.41 mmol) and 4-dimethylaminopyridine (100 mg, 0.815 mmol) were dissolved in methylene chloride (20 mL). N,N-Diisopropylethylamine (2.3 g, 3.1 mL, 17.5 mmol) were added. The reaction mixture was stirred for 2 hours at room temperature, concentrated and chromatographed (silica gel, 5.0 X 15 cm, 40% ethyl acetate, 60% hexane). Fractions containing the desired compound were collected and concentrated to yield compound A as a clear oil (2.2 g, 99 %), MS: (M+H)⁺ 275⁺.

### B. [S-(R*,R*)]-(1-Formyl-2-methylbutyl)carbamic acid 1,1-dimethylethyl ester

Compound A (1.00 g, 3.64 mmol) was dissolved in THF (10 mL) and the solution was cooled to 0 °C. 1M lithium aluminum hydride in THF (3.64 mL, 3.64 mmol) was added dropwise over 30 minutes. The reaction mixture was stirred for an additional 30 minutes at 0°C under nitrogen. Diethyl ether (200 mL) was added and the reaction mixture was quenched with dropwise addition of 1M potassium bisulfate (100 mL) at 0°C. The mixture was stirred for an additional 1 hour at 0°C and the layers were separated. The organic layer was washed with saturated 1M potassium bisulfate (2 X 100 mL), water (100 mL), sodium bicarbonate (2 X 100 mL) and brine (100 mL), dried (MgSO₄), and concentrated to yield compound B as a clear oil (690 mg, 88%) which was used immediately in the next step without further purification.

### C. [S-(R*,R*)]-[2-Methyl-1-[(1,2,3,4-tetrahydro-2-isoquinolinyl)methyl]butyl]-carbamic acid 1,1-dimethylethyl ester

Compound B (690 mg, 3.20 mmol) and 1,2,3,4-tetrahydroisoquinoline (852 mg, 0.80 mL, 6.4 mmol) were dissolved in dry methanol (10 mL). Molecular sieve (800 mg, 3 angstrom) was added and the mixture was stirred for 30 minutes. Glacial acetic acid (2 mL) was added and sodium cyanoborohydride (201 mg, 3.20 mmol) was added portionwise over 1 hour. The reaction mixture was stirred for 16 hours. The mixture was cooled to 0°C and saturated NaHCO₃ (30 mL) was slowly added. The mixture was concentrated and the product was extracted into ethyl acetate (100 mL). The ethyl acetate layer was washed with water (100 mL) and brine (50 mL), dried (MgSO₄), concentrated and chromatographed (silica gel, 4.1 X 15 cm, 40% ethyl acetate, 60% hexanes). Fractions containing the desired product were combined and concentrated to yield compound C as a clear oil (900 mg, 84%), MS: (M+H)⁺ 333⁺.

### D. [2S-[2α(1R*,2R*),4α]]-2-[[[2-Methyl-1-[(1,2,3,4-tetrahydro-2-isoquinolinyl)-methyl]butyl]amino]methyl]-4-[(triphenylmethyl)thio]-1-pyrrolidinecarboxylic acid 1,1-dimethylethyl ester trifluoroacetate

Compound C (340 mg, 1.02 mmol) was stirred in dimethyl sulfide (0.4 mL) and 4N HCl in dioxane (10 mL) for 40 minutes. The mixture was concentrated, dissolved in methylene chloride (30 mL) and concentrated. This latter procedure was repeated five times to yield the amine as a clear glass (270 mg, 99%), MS: (M+H)⁺ 233⁺. This amine hydrochloride (270 mg, 1.02 mmol) and (2S-cis)-2-Formyl-4-[(triphenylmethyl)thio]-1-pyrrolidinecarboxylic acid 1,1-dimethylethyl ester (300 mg, 0.56 mmol) were dissolved in methanol (10 mL). Molecular sieves (3 angstroms, 200 mg) were added and the mixture was stirred for 30 minutes. Glacial acetic acid (0.2 mL) was added and sodium cyanoborohydride (35 mg, 0.56 mmol) was added portionwise over 30 minutes. The reaction mixture was stirred for 16 hours. The mixture was cooled to 0°C and saturated NaHCO₃ (10 mL) was slowly added. The mixture was concentrated and the product was extracted into ethyl acetate (70 mL). The ethyl acetate layer was washed with water (50 mL) and brine (50 mL), dried (MgSO₄), concentrated and chromatographed (silica gel, 4.1 X 15 cm, 40% ethyl acetate, 60% hexanes). Fractions containing the desired product were combined and concentrated to yield compound D as a clear oil (200 mg, 51%), MS: (M+H)⁺ 690⁺.

### E. [2S-[2α(2R*,3R*),4α]]-1,2,3,4-Tetrahydro-2-[2-[[(4-mercapto-2-pyrrolidinyl)methyl]-amino]-3-methylpentyl]isoquinoline, trifluoroacetate

Compound D (200 mg, 0.29 mmol) was dissolved in methylene chloride (7 mL), triethylsilane (0.030 mL, 22 mg, 0.19 mmol) and trifluoroacetic acid (7 mL), and was stirred for 1 hour at room temperature. The mixture was concentrated, dissolved in methylene chloride (50 mL) and concentrated. This latter procedure was repeated 3 times to yield the crude product as a white sticky solid in quantitative yield. The crude solid was purified by preparative HPLC (YMC S-10 ODS column, 30 X 500 mm; solvent A, 0.1% TFA in 90% water, 10 % acetonitrile; solvent B, 0.1% TFA in 10% water, 90% acetonitrile: 20-30% B in 40 minutes, flow rate 20 mL/minute; UV monitored at 220 nm). Fractions containing the desired product were combined, concentrated and lyophilized to provide the title compound (49 mg, 7%), Purity > 99 % (YMC S3 ODS column, 6 X 150 mm, 220 nm, 1.5 mL/min, 0-100% B in A over 30 minutes (solvent A, 0.2% H₃PO₄ in 90% water, 10 % methanol; solvent B, 0.2% H₃PO₄ in 10% water, 90% methanol, RT 12.13 min. , HI>99 %), [α]_{D}²⁵ = +30.0° (c=0.15, methanol), MS: (M+H)⁺ 348⁺, ¹H-NMR (CD₃OD, 400 MHz) δ (ppm) 7.31-7.19 (4H, m), 4.51 (2H, m), 3.78 (1H, m), 3.68 (2H, m), 3.53 (1H, m), 3.29-3.10 (8H, m), 2.84 (1H, m), 2.58 (1H, m), 1.86 (1H, m), 1.72 (1H, m), 1.38 (1H, m), 1.25 (1H, m), 0.98 (3H, t, J=7.5Hz), 0.90 (3H, d, J=7.3Hz).

### Example 31

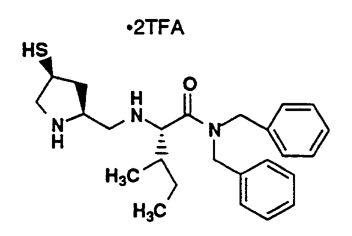

### (2S-cis)-N²-[(4-Mercapto-2-pyrrolidinyl)methyl]-N,N-bis(phenylmehyl)-L-isoleucinamide, trifluoroacetate

### A. (2S-cis)-N²-[[1-[(1,1-Dimethylethoxy)-carbonyl]-4-[(triphenylmethyl]thio]-2-pyrrolidinyl]methyl]-N,N-bis(phenylmethyl)-L-isoleucinamide

Compound A (130 mg, 99%) was prepared from compound B of Example 12 with dibenzylamine (34 mg, 0.034 mL, 0.17 mmol) using the process for preparing compound C of Example 12.
MS: (M+H)⁺ 768⁺.

### B. (2S-cis)-N²-[(4-Mercapto-2-pyrrolidinyl)methyl]-N,N-bis(phenylmehyl)-L-isoleucinamide, trifluoroacetate

The title compound (39 mg, 35%) was prepared from compound A using a process analogous to the process for preparing the title compound of

### Example 12. MS: (M+H)⁺ 426⁺, ¹H-NMR (CD₃OD, 400 MHz) δ (ppm) 7.57-7.30 (10H, m), 5.32-5.20 (1H, m), 4.95-4.80 (1H, m), 4.65-4.45 (1H, m), 4.38-4.27 (1H, m), 4.00-3.56 (4H, m), 3.28-3.04 (2H, m), 2.88-2.61 (1H, m), 1.95-1.67 (3H, m), 1.28 (1H, m), 1.16 (3H, m), 0.99 (3H, m).

### Example 32

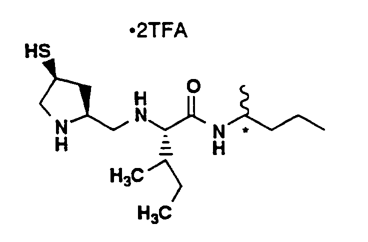

### (2S-cis)-N²-[(4-Mercapto-2-pyrrolidinyl)methyl]-N-(1-methylbutyl)-L-isoleucineamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-(1-methylbutyl)-L-isoleucinamide

Compound A (111 mg, 99%) was prepared from compound B of Example 12 with 1-methylbutylamine (15 mg, 0.020 mL, 0.17 mmol) followed by N,N-diisopropyl-ethylamine (22 mg, 0.030 mL, 0.17 mmol) using the process for preparing compound C of Example 12.
MS: (M+H)⁺ 658⁺.

### B. (2S-cis)-N²-[(4-Mercapto-2-pyrrolidinyl)-methyl]-N-(1-methylbutyl)-L-isoleucineamide, trifluoroacetate

The title compound (25 mg, 27%) was prepared from compound A using a process analogous to the process for preparing the title compound of Example 12.
MS: (M+H)⁺ 315⁺, ¹H-NMR (CD₃OD, 400 MHz) δ (ppm) 4.08(1H, m), 3.86 (1H, m), 3.73 (1H, m), 3.62 (1H, m), 3.42-3.22 (4H, m), 2.87 (1H, m), 2.08-1.89 (2H, m), 1.70 (1H, m), 1.52 (4H, m), 1.40 (1H, m), 1.25-1.01 (12H, m).

### Example 33

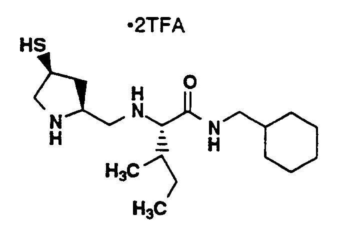

### (2S-cis)-N-(Cyclohexylmethyl)-N²-[(4-mercapto-2-pyrrolidinyl)methyl]-L-isoleucinamide, trifluoroacetate

### A. (2S-cis)-N-(Cyclohexylmethyl)-N²-[[1-[(1,1-dimethylethoxy)carbonyl]-4-[(triphenylmethyl)thio]-2-pyrrolidinyl]methyl]-L-isoleucinamide

Compound A (116 mg, 99%) was prepared from compound B of Example 12 with cyclohexane-methylamine (19 mg, 0.022 mL, 0.17 mmol) followed by N,N-diisopropylethylamine (22 mg, 0.030 mL, 0.17 mmol) using the process for preparing compound C of Example 12.
MS: (M+H)⁺ 672⁺.

### B. (2S-cis)-N-(Cyclohexylmethyl)-N²-[(4-mercapto-2-pyrrolidinyl)methyl]-L-isoleucinamide, trifluoroacetate

The title compound (28 mg, 29%) was prepared from compound A using a process analogous to the process for preparing the title compound of Example 12.
MS: (M+H)⁺ 342⁺, ¹H-NMR (CD₃OD, 400 MHz) δ (ppm) 3.84 (1H, m), 3.68 (1H, m), 3.53 (1H, m), 3.39 (1H, m), 3.17 (3H, m), 3.01 (2H, m), 2.65 (1H, m), 1.75 (9H, m), 1.50 (1H, m), 1.24 (3H, m), 0.98 (8H, m).

### Example 34

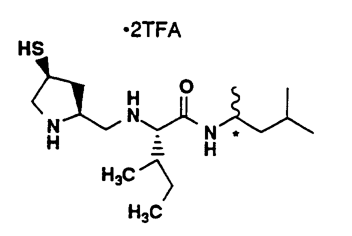

### (2S-cis)-N-(1,3-Dimethylbutyl)-N²-[(4-mercapto-2-pyrrolidinyl)methyl]-L-isoleucinamide, trifluoroacetate

### A. (2S-cis)-N-(1,3-Dimethylbutyl)-N²-[[1-[(1,1-dimethylethoxy)carbonyl]-4-[(triphenylmethyl)thio]-2-pyrrolidinyl]methyl]-L-isoleucinamide

Compound A (114 mg, 99%) was prepared from compound B of Example 12 with 1,3-Dimethylbutyl-amine (17 mg, 0.024 mL, 0.17 mmol) followed by N,N-diisopropylethylamine (22 mg, 0.030 mL, 0.17 mmol) using the process for preparing compound C of Example 12.
MS: (M+H)⁺ 672⁺.

### B. (2S-cis)-N-(1,3-Dimethylbutyl)-N²-[(4-mercapto-2-pyrrolidinyl)methyl]-L-isoleucinamide, trifluoroacetate

The title compound (30 mg, 32%) was prepared from compound A using a process analogous to the process for preparing the title compound of Example 12.
MS: (M+H)⁺ 344⁺, ¹H-NMR (CD₃OD, 400 MHz) δ (ppm) 3.78-3.53 (3H, m), 3.50-3.28 (2H, m), 3.15-3.00 (2H, m), 2.89 (3H, m), 2.52 (1H, m), 1.71-1.51 (8H, m), 1.40 (1H, m), 1.16 (3H, m), 0.95-0.78 (8H, m).

### Example 35

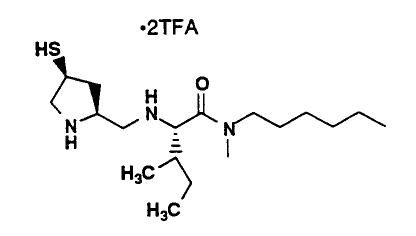

### (2S-cis)-N-Hexyl-N²-[(4-mercapto-2-pyrrolidinyl)methyl]-N-methyl-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-hexyl-N-methyl-L-isoleucinamide

Compound A (116 mg, 99%) was prepared from compound B of Example 12 with N-Methylhexylamine (20 mg, 0.027 mL, 0.17 mmol) followed by N,N-diisopropylethylamine (22 mg, 0.030 mL, 0.17 mmol) using the process for preparing compound C of Example 12.
MS: (M+H)⁺ 686⁺.

### B. (2S-cis)-N-Hexyl-N²-[(4-mercapto-2-pyrrolidinyl)methyl]-N-methyl-L-isoleucinamide, trifluoroacetate

The title compound (40 mg, 41%) was prepared from compound A using a process analogous to the process for preparing the title compound of Example 12.
MS: (M+H)⁺ 344⁺, ¹H-NMR (CD₃OD, 400 MHz) δ (ppm) 3.79 (2H, m), 3.70-3.61 (2H, m), 3.51 (1H, m), 3.41 (1H, m), 3.21-3.08 (4H, m), 2.95 (2H, m), 2.60 (1H, m), 1.90 (1H, m), 1.81 (1H, m), 1.67 (2H, m), 1.51 (2H, m), 1.33 (6H, m), 0.94 (9H, m).

### Example 36

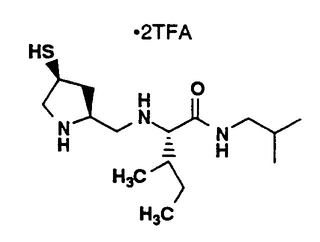

### (2S-cis)-N²-[(4-Mercapto-2-pyrrolidinyl)methyl]-N-(2-methylpropyl)-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-(2-methylpropyl)-L-isoleucinamide

Compound A (109 mg, 99%) was prepared from compound B of Example 12 with isobutylamine (12 mg, 0.017 mL, 0.17 mmol) followed by N,N-diisopropylethylamine (22 mg, 0.030 mL, 0.17 mmol) using the process for preparing compound C of Example 12. MS: (M+H)⁺ 644⁺.

### B. (2S-cis)-N²-[(4-Mercapto-2-pyrrolidinyl)methyl]-N-(2-methylpropyl)-L-isoleucinamide, trifluoroacetate

The title compound (49 mg, 54%) was prepared from compound A using a process analogous to the process for preparing the title compound of Example 12.
MS: (M+H)⁺ 302⁺, ¹H-NMR (CD₃OD, 400 MHz) δ (ppm) 4.40 (1H, m), 4.21 (1H, m), 4.12-3.95 (2H, m), 3.79-3.58 (3H, m), 3.50 (1H, m), 3.20 (1H, m), 2.45-2.13 (4H, m), 1.78 (1H, m), 1.59-1.38 (13H, m).

### Example 37

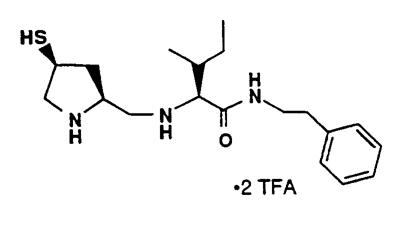

### (2S-cis)-N²-[(4-Mercapto-2-pyrrolidinyl)methyl]-N-(2-phenylethyl)-L-isoleucinamide, trifluoroacetate

### A. (2S-cis)-N²-[[1-[(1,1-Dimethylethoxy)-carbonyl]-4-[(triphenylmethyl)thio]-2-pyrrolidinyl]methyl]-N-(2-phenylethyl)-L-isoleucinamide

A (117 mg, 99%) was prepared from compound B of Example 12 with 2-phenylethylamine (21.5µl, 0.17 mmol) along with N,N-diisopropylethylamine (30µl, 0.17 mmol) using the process for preparing compound C of Example 12.
MS: (M+H)⁺ 692⁺.

### B. (2S-cis)-N²-[(4-Mercapto-2-pyrrolidinyl)methyl]-N-(2-phenylethyl)-L-isoleucinamide, trifluoroacetate

The title compound (31 mg, 32%) was prepared from compound A using a process analogous to the process for preparing the title compound of Example 12.
MS: (M+H)⁺ 350⁺, ¹H-NMR(CD₃OD, 400 MHz) δ (ppm) 7.26 (5H, m), 3.72(1H, m), 3.55(4H, m), 3.10 (1H, m), 3.01 (1H, m), 2.86 (3H, m), 2.60(1H, m), 1.72(1H, m),1.55 (1H, m), 1.10(1H, m), 0.89(6H, m).

### Example 38

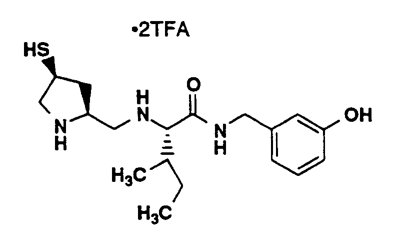

### (2S-cis)-N-[(3-Hydroxyphenyl)methyl]-N²-[(4-mercapto-2-pyrrolidinyl)methyl]-L-isoleucinamide, trifluoroacetate

### A. 3-(Aminomethyl)phenol

3-Hydroxybenzonitrile (1.2 g, 0.010 mol) was dissolved in anhydrous diethyl ether (30 mL) and LiAlH₄ (1M solution in THF, 10 mL, 0.010 mol) was added dropwise over 30 minutes at room temperature. The mixture was stirred at room temperature for 2 hours and cooled to 0°C, and 15% NaOH (50 mL) was added. The mixture was neutralized with TFA. Half of the crude mixture was purified by preparative HPLC (YMC S-10 ODS colum, 50 X 500 mm; solvent A, 0.1% TFA in 90% water, 10% acetonitrile; solvent B, 0.1% TFA in 10% water, 90% acetonitrile: 0% B in 40 minutes, flow rate 50 mL/minute; UV monitored at 220 nm). Fractions containing the desired product were combined, concentrated and lyophilized to provide compound A (450 mg, 73%), MS: (M+H)⁺ 124⁺.

### B. (2S-cis)-N²-[[1-[(1,1-Dimethylethoxy)carbonyl]-4-[(triphenylmethyl)thio]-2-pyrrolidinyl]methyl]-N-[(3-hydroxyphenyl)methyl]-L-isoleucinamide

Compound B (117 mg, 99%) was prepared from compound B of Example 12 with compound A (42 mg, 0.34 mmol) from above followed by N,N-diisopropylethylamine (22 mg, 0.030 mL, 0.17 mmol) using the process for preparing compound C of Example 12.
MS: (M+H)⁺ 694⁺.

### C. (2S-cis)-N-[(3-Hydroxyphenyl)methyl]-N²-[(4-mercapto-2-pyrrolidinyl)methyl]-L-isoleucinamide, trifluoroacetate

The title compound (34 mg, 34%) was prepared from compound B using a process analogous to the process for preparing the title compound of Example 12.
MS: (M+H)⁺ 352⁺, ¹H-NMR (CD₃OD, 400 MHz) δ(ppm) 7.13 (1H, t, J=7.7Hz), 6.75 (2H, m), 6.69 (1H, m), 4.42-4.27 (2H, d of d, J=14.5Hz, 43.2 Hz), 3.87 (1H, m), 3.66 (1H, m), 3.51 (3H, m), 3.15 (2H, m), 2.62 (1H, m), 1.86 (1H, m), 1.71 (1H, m), 1.63 (1H, m), 1.20 (1H, m), 0.98-0.90 (6H, m).

### Example 39

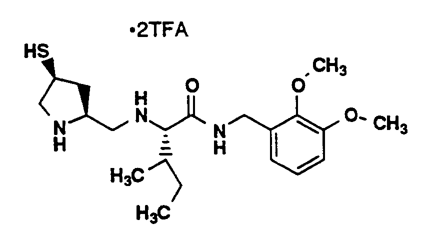

### (2S-cis)-N-[(2,3-Dimethoxyphenyl)methyl]-N²-[(4-mercapto-2-pyrrolidinyl)methyl]-L-isoleucinamde, trifluoroacetate

### A. (2S-cis)-N-[(2,3-Dimethoxyphenyl)methyl]-N²-[[1-[(1,1-dimethylethoxy)carbonyl]-4-[(triphenylmethyl)thio]-2-pyrrolidinyl]methyl]-L-isoleucinamide

Compound A (125 mg, 99%) was prepared from compound B of Example 12 with 2,3-dimethoxy-benzylamine (28 mg, 0.025 mL, 0.17 mmol) followed by N,N-diisopropylethylamine (22 mg, 0.030 mL, 0.17 mmol) using the process for preparing compound C of Example 12.
MS: (M+H)⁺ 738⁺.

### B. (2S-cis)-N-[(2,3-Dimethoxyphenyl)methyl]-N²-[(4-mercapto-2-pyrrolidinyl)methyl]-L-isoleucinamde, trifluoroacetate

The title compound (39 mg, 37%) was prepared from compound A using a process analogous to the process for preparing the title compound of Example 12.
MS: (M+H)⁺ 396⁺, ¹H-NMR (CD₃OD, 400 MHz) δ (ppm) 7.03 (1H, t, J=8.0Hz), 6.97 (1H, d J=9.8Hz), 6.89 (1H, d, J=9.4Hz), 4.45 (2H, d of d), 3.89 (1H, m), 3.85 (3H, s), 3.84 (3H, s), 3.68 (1H, m), 3.53 (2H, m), 3.18 (3H, m), 2.63 (1H, m), 1.86 (1H, m), 1.72 (1H, m), 1.61 (1H, m), 1.18 (1H, m), 0.98-0.90 (6H, m).

### Example 40

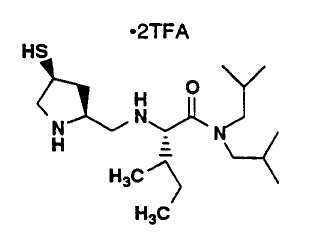

### (2S-cis)-N²-[(4-Mercapto-2-pyrrolidinyl)methyl]-N,N-bis(2-methylpropyl)-L-isoleucinamide, trifluoroacetate

### A. (2S-cis)-N²-[[1-[(1,1-Dimethylethoxy)carbonyl]-4-[(triphenylmethyl)thio]-2-pyrrolidinyl]methyl]-N,N-bis(2-methylpropyl)-L-isoleucinamide

Compound A (125 mg, 99%) was prepared from compound B of Example 12 with diisobutylamine (34 mg, 0.034 mL, 0.17 mmol) followed by N,N-diisopropylethylamine (22 mg, 0.030 mL, 0.17 mmol) using the process for preparing compound C of Example 12.
MS: (M+H)⁺ 738⁺.

### B. (2S-cis)-N²-[(4-Mercapto-2-pyrrolidinyl)methyl]-N,N-bis(2-methylpropyl)-L-isoleucinamide, trifluoroacetate

The title compound (42 mg, 42%) was prepared from compound A using a process analogous to the process for preparing the title compound of Example 12.
MS: (M+H)⁺ 358⁺, ¹H-NMR (CD₃OD, 400 MHz) δ (ppm) 3.90 (1H, m), 3.81 (2H, m), 3.67 (1H, m), 3.53 (1H, m), 3.26 (1H, m), 3.16-3.07 (2H, m), 3.00 (2H, m), 2.74 (1H, m), 2.64 (1H, m), 2.02 (2H, m), 1.77 (1H, m), 1.72 (1H, m), 1.60(1H, m), 1.50 (1H, m), 1.12 (3H, d, J=6.8 Hz), 0.98 (3H, d, J=6.4 Hz), 0.93 (9H, m), 0.86 (3H, d, J=6.4 Hz).

### Example 41

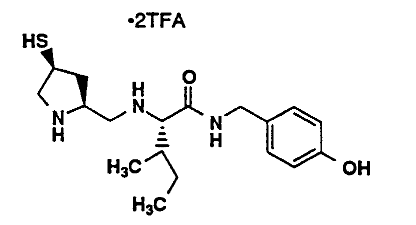

### (2S-cis)-N-[(4-Hydroxyphenyl)methyl]-N²-[(4-mercapto-2-pyrrolidinyl)methyl]-L-isoleucinamide, trifluoroacetate

### A. 4-(Aminomethyl)phenol

4-Hydroxybenzonitrile (1.2 g, 0.010 mol) was used in the process for preparing compound A of Example 38 to provide compound A (1.2 g, 97%), MS: (M+H)⁺ 124⁺.

### B. (2S-cis)-N²-[[1-[(1,1-Dimethylethoxy)carbonyl]-4-[(triphenylmethyl)thio]-2-pyrrolidinyl]methyl]-N-[(4-hydroxyphenyl)methyl]-L-isoleucinamide

Compound B (117 mg, 99%) was prepared from compound B of Example 12 with compound A (42 mg, 0.34 mmol) from above followed by N,N-diisopropylethylamine (22 mg, 0.030 mL, 0.17 mmol) using the process for preparing compound C of Example 12.
MS: (M+H)⁺ 694⁺.

### C. (2S-cis)-N-[(4-Hydroxyphenyl)methyl]-N²-[(4-mercapto-2-pyrrolidinyl)methyl]-L-isoleucinamide, trifluoroacetate

The title compound (29 mg, 29%) was prepared from compound B from above using a process analogous to the process for preparing the title compound of Example 12.
MS: (M+H)⁺ 352⁺, ¹H-NMR (CD₃OD, 400 MHz) δ (ppm) 7.14 (2H, d, J=8.5Hz), 6.74 (2H, d, J=6.4Hz), 4.41-4.22 (2H, d of d, J=14.5Hz, 59.0 Hz), 3.91 (1H, m), 3.67 (1H, m), 3.57 (1H, m), 3.50 (1H, m), 3.36 (1H, m), 3.16 (2H, m), 2.66 (1H, m), 1.87 (1H, m), 1.72 (1H, m), 1.69 (1H, m), 1.20 (1H, m), 0.98-0.90 (6H, m).

### Example 42

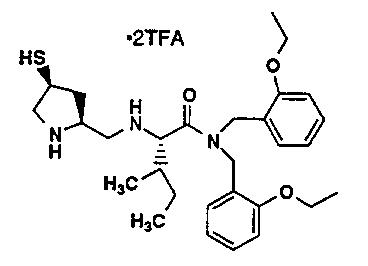

### (2S-cis)-N,N-Bis[(2-ethoxyphenyl)methyl]-N²-[(4-mercapto-2-pyrrolidinyl)methyl]-L-isoleucinamide, trifluoroacetate

### A. 2-Ethoxy-N-[(2-ethoxyphenyl)methyl]-benzenemethanamine

2-Ethoxybenzenemethanamine (300 mg, 0.30 mL, 1.98 mmol) and 2-ethoxybenzaldehyde (297 mg, 0.28 mL, 1.98 mmol) were dissolved in dry methanol (10 mL). Glacial acetic acid (0.2 mL) was added and sodium cyanoborohydride (124 mg, 1.98 mmol) was added portionwise over 1 hour. The reaction mixture was stirred for 16 hours. The mixture was cooled to 0°C and saturated NaHCO₃ (20 mL) was slowly added. The mixture was concentrated and the product was extracted into ethyl acetate (100 mL). The ethyl acetate layer was washed with water (100 mL) and brine (50 mL), dried (MgSO₄), concentrated and chromatographed (silica gel, 4.1 X 15 cm, 40% ethyl acetate, 60% hexanes). Fractions containing the desired product were combined and concentrated to yield compound A as a clear oil (450 mg, 80%), MS: (M+H)⁺ 286⁺.

### B. (2S-cis)-N²-[[1-[(1,1-Dimethylethoxy)-carbonyl]-4-[(triphenylmethyl)thio]-2-pyrrolidinyl]methyl]-N,N-bis[(2-ethoxyphenyl)methyl]-L-isoleucinamide

Compound B (145 mg, 99%) was prepared from compound B of Example 12 with compound A (97 mg, 0.34 mmol) from above followed by N,N-diisopropylethylamine (22 mg, 0.030 mL, 0.17 mmol) using the process for preparing compound C of Example 12.
MS: (M+H)⁺ 856⁺.

### C. (2S-cis)-N,N-Bis[(2-ethoxyphenyl)methyl]-N²-[(4-mercapto-2-pyrrolidinyl)methyl]-L-isoleucinamide, trifluoroacetate

The title compound (61 mg, 48%) was prepared from compound B above using a process analogous to the process for preparing the title compound of Example 12.
MS: (M+H)⁺ 514⁺, ¹H-NMR (CD₃OD, 400 MHz) δ (ppm) 7.14 (4H, m), 6.74 (4H, m), 4.37 (2H, m), 4.23 (2H, m), 3.91 (2H, m), 3.68 (2H, m), 3.57 (1H, m), 3.51 (1H, m), 3.36 (1H, m), 3.17 (3H, m), 2.66 (1H, m), 1.87 (1H, m), 1.70 (1H, m), 1.72 (1H, m), 1.61(1H, m), 1.19 (1H, m), 0.98-0.90 (12H, m).

### Example 43

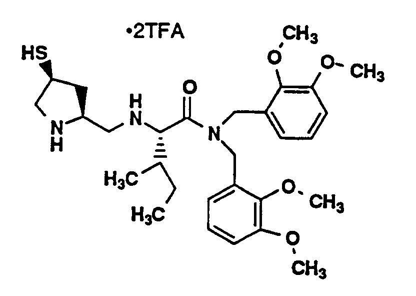

### (2S-cis)-N,N-Bis[(2,3-dimethoxyphenyl)methyl]-N²-[(4-mercapto-2-pyrrolidinyl))methyl]-L-isoleucinamide, trifluoroacetate

### A. 2,3-Dimethoxy-N-[(2,3-dimethoxyphenyl)methyl]benzenemethanamine

2,3-Dimethoxybenzenemethanamine (500 mg, 0.44 mL, 3.0 mmol) and 2,3-dimethoxybenzaldehyde (500 mg, 3.0 mmol) were used in the process for preparing compound A of Example 42 to provide compound A as a clear oil (650 mg, 69%), MS: (M+H)⁺ 318⁺.

### B. (2S-cis)-N,N-Bis[(2,3-dimethoxyphenyl)methyl]-N²-[[1-[(1,1-dimethylethoxy)carbonyl]-4-[(triphenylmethyl)thio]-2-pyrrolidinyl]methyl]-L-isoleucinamide

Compound B (150 mg, 99%) was prepared from compound B of Example 12 with compound A (108 mg, 0.34 mmol) from above followed by N,N-diisopropylethylamine (22 mg, 0.030 mL, 0.17 mmol) using the process for preparing compound C of Example 12.
MS: (M+H)⁺ 888⁺.

### C. (2S-cis)-N,N-Bis[(2,3-dimethoxyphenyl)-methyl]-N²-[(4-mercapto-2-pyrrolidinyl)-methyl]-L-isoleucinamide, trifluoro-acetate

The title compound (80 mg, 61%) was prepared from compound B using a process analogous to the process for preparing the title compound of Example 12.
MS: (M+H)⁺ 546⁺, ¹H-NMR (CD₃OD, 400 MHz) δ (ppm) 7.04-6.87 (4H, m), 6.70 (2H, m), 4.91 (1H, d, J=14.5Hz), 4.62 (1H, d, J=16.7Hz), 4.31 (2H, d of d, J=6.4Hz, 16.7Hz), 4.02 (1H, m), 3.78 (3H, s), 3.75 (3H, s), 3.738 (3H, s), 3.67 (3H, s), 3.57 (2H, m) , 3.42 (1H, m), 3.05 (1H, m), 2.98 (2H, m), 2.55 (1H, m), 1.77 (1H, m), 1.58-1.46 (2H, m), 1.08 (1H, m), 0.98 (3H, d, J=7.3Hz), 0.82 (3H, t, J=7.2Hz).

### Example 44

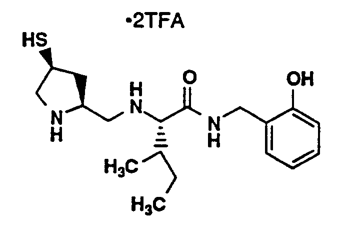

### (2S-cis)-N-[(2-Hydroxyphenyl)methyl]-N²-[(4-mercapto-2-pyrrolidinyl)methyl]-L-isoleucinamide, trifluoroacetate

### A. 2-(Aminomethyl)phenol

2-Hydroxybenzonitrile (1.2 g, 0.010 mol) was used in the process for preparing compound A of Example 38 to provide compound A (1.2 g, 97%), MS: (M+H)⁺ 124⁺.

### B. (2S-cis)-N²-[[1-[(1,1-Dimethylethoxy)-carbonyl]-4-[(triphenylmethyl)thio]-2-pyrrolidinyl]methyl]-N-[(2-hydroxyphenyl)methyl]-L-isoleucinamide

Compound B (117 mg, 99%) was prepared from compound B of Example 12 with compound A (42 mg, 0.34 mmol) from above followed by N,N-diisopropylethylamine (22 mg, 0.030 mL, 0.17 mmol) using the process for preparing compound C of Example 12.
MS: (M+H)⁺ 694⁺.

### C. (2S-cis)-N-[(2-Hydroxyphenyl)methyl]-N²-[(4-mercapto-2-pyrrolidinyl)methyl]-L-isoleucinamide, trifluoroacetate

The title compound (12 mg, 12%) was prepared from compound B using a process analogous to the process for preparing the title compound of Example 12.
MS: (M+H)⁺ 352⁺, ¹H-NMR (CD₃OD, 400 MHz) δ (ppm) 7.17 (1H, m), 7.13 (1H, m), 6.80 (2H, m), 4.39 (2H, d of d, J=14.1Hz, 20.5 Hz), 3.71 (1H, m), 3.66 (1H, m), 3.50 (1H, m), 3.24 (1H, m), 3.10 (1H, m), 2.96 (2H, m), 2.56 (1H, m), 1.80-1.55 (3H, m), 1.19 (1H, m), 0.93-0.87 (6H, m).

### Example 45

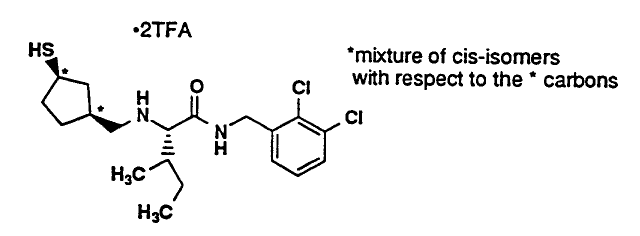

### cis-N-[(2,3-Dichlorophenyl)methyl]-N²-[(3-mercaptocyclopentyl)methyl]-L-isoleucinamide, trifluoroacetate

### A. cis-N-Methoxy-3-[(triphenylmethyl)thio]cyclopentanecarboxamide

Cis-3-[(Triphenylmethyl)thio]-cyclopentanecarboxylic acid (1.0 g, 2.58 mmol), N,O-dimethylhydroxamate·HCl (278 mg, 2.84 mmol), bromo-tris-pyrrolidino-phosphonium hexafluorophosphate (1.33 g, 2.84 mmol) and 4-dimethylaminopyridine (100 mg, 0.815 mmol) were dissolved in methylene chloride (20 mL). N,N-Diisopropylethylamine (701 mg, 1.0 mL, 5.42 mmol) were added dropwise and the reaction mixture was stirred for 2 hours at room temperature, concentrated and chromatographed (silica gel, 5.0 X 15 cm, 40% ethyl acetate, 60% hexane). Fractions containing the desired compound were collected and concentrated to yield compound A as a clear oil (1.0 g, 90 %), MS: (M+H)⁺ 432⁺.

### B. cis-3-[(Triphenylmethyl)thio]cyclopentanecarboxaldehyde

Compound A (950 mg, 2.20 mmol) was dissolved in THF (5 mL) and the solution was cooled to 0°C. 1M LiAlH₄ in THF (2.2 mL, 2.2 mmol) was added dropwise over 30 minutes. The reaction mixture was stirred for additional 30 minutes at 0°C under nitrogen. Diethyl ether (100 mL) was added and the reaction mixture was quenched with dropwise addition of 1M potassium bisulfate (100 mL) at 0°C. The mixture was stirred for an additional 1 hour at 0°C and the layers were separated. The organic layer was washed with saturated 1M KHSO₄ (100 mL), NaHCO₃ (100 mL), 1M KHSO₄ (100 mL) and brine (100 mL), dried (MgSO₄), and concentrated to yield compound B as a clear oil (818 mg, 99%) which was used immediately in the next step without further purification.

### C. cis-N-[[3-[(Triphenylmethyl)thio]-cyclopentyl]methyl]-L-isoleucine 2-[(diphenylmethyl)amino]-2-oxoethyl ester

Compound B (818 mg, 2.2 mmol) and L-Isoleucine 2-[(diphenylmethyl)amino]-2-oxoethyl ester hydrochloride (1.3 g, 3.3 mmol) were dissolved in dry methanol (40 mL). Molecular sieves (3 angstrom, 2.0 g) were added and the mixture was stirred for 30 minutes. Glacial acetic acid (0.9 mL) was added and sodium cyanoborohydride (138 mg, 2.2 mmol) was added portionwise over 1 hour. The reaction mixture was stirred for 16 hours. The mixture was cooled to 0°C and saturated NaHCO₃ (10 mL) was slowly added. The mixture was concentrated and the product was extracted into ethyl acetate (100 mL). The ethyl acetate layer was washed with water (100 mL) and brine (50 mL), dried (MgSO₄), concentrated and chromatographed (silica gel, 4.1 X 15 cm, 40% ethyl acetate, 60% hexanes). Fractions containing the desired product were combined and concentrated to yield compound C as a clear oil (1.2 g, 77%), MS: (M+H)⁺ 711⁺.

### D. cis-N-[[3-(Triphenylmethyl)thio]-cyclopentyl]methyl]-L-isoleucine

Compound C (1.0 g, 1.4 mmol) was dissolved in DMF (5 mL) and 5N NaOH (0.2 mL). The reaction mixture was stirred for 2 hours. The mixture was poured into water (50 mL), neutralized to pH 6 using 1M KHSO₄ and extracted with ethyl acetate (50 mL). White particles precipitated out of the ethyl acetate layer and were filtered, air dried, recrystallized from ethyl acetate/hexane and dried under vacuum to yield compound D as a white solid (400 mg, 60%), MS: (M+H)⁺ 488⁺.

### E. cis-N-[(2,3-Dichlorophenyl)methyl]-N²-[(3-[(triphenylmethyl)thio]cyclopentyl]methyl]-L-isoleucinamide

Compound E (132 mg, 99%) was prepared from compound D (100 mg, 0.21 mmol), and 2,3-Dichlorobenzylamine (40 mg, 0.23 mmol) followed by N,N-diisopropyl-ethylamine (29 mg, 0.040 mL, 0.23 mmol) using a process analogous to the process for preparing compound C of Example 12.
MS: (M+Na)⁺ 667⁺.

### F. cis-N-[(2,3-Dichlorophenyl)methyl]-N²-[(3-mercaptoccylopentyl)methyl]-L-isoleucinamide, trifluoroacetate

The title compound (62 mg, 48%) was prepared from compound E using a process analogous to the process for preparing the title compound of Example 12.
MS: (M+H)⁺ 403⁺, ¹H-NMR (CD₃OD, 400 MHz) δ (ppm) 7.41 (1H, d, J=7.7Hz), 7.32 (1H, d, J=6.0Hz), 7.21 (1H, t, J=8.1Hz), 4.48 (2H, s), 3.60 (1H, d, J=5.1Hz), 3.11 (1H, m), 2.95 (1H m), 2.80 (1H, m), 2.19 (2H, m), 1.99 (1H, m), 1.84 (2H, m), 1.47 (4H, m), 1.13 (1H, m), 0.87 (6H, m).

### Example 46

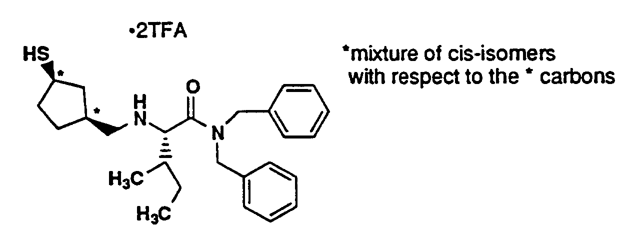

### cis-N²-[(3-Mercaptocyclopentyl)methyl]-N,N-bis(phenylmethyl)-L-isoleucinamide, trifluoroacetate

### A. cis-N,N-Bis(phenylmethyl)-N²-[[3-[(triphenylmethyl)thio]cyclopentyl]methyl]-L-isoleucinamide

Compound A (137 mg, 99%) was prepared from compound D from Example 45 (100 mg, 0.21 mmol) and dibenzylamine (103 mg, 0.10 mL, 0.52 mmol) followed by N,N-diisopropylethylamine (29 mg, 0.040 mL, 0.23 mmol) using a process analogous to the process for preparing compound C of Example 12.
MS: (M+H)⁺ 667⁺.

### B. cis-N²-[(3-Mercaptocyclopentyl)methyl]-N,N-bis(phenylmethyl)-L-isoleucinamide, trifluoroacetate

The title compound was prepared from compound A using a process analogous to the process for preparing the title compound of Example 12.
MS: (M+H)⁺ 425⁺, ¹H-NMR (CD₃OD, 400 MHz) δ (ppm) 7.44-7.24 (10H, m), 5.11 (0.5H, d, J=3.8Hz), 5.07 (0.5H, d, J=4.3Hz), 4.75 (1H, d, J=10.7Hz), 4.55 (1H, d, J=7.1Hz), 4.38 (0.5H, d, J=3.4Hz), 4.34 (0.5H, d, J=3.4Hz), 4.24 (1H, d, J=4.3Hz), 3.20 (1H, m), 2.78 (2H, m), 2.24 (1H, m), 2.07 (2H, m), 1.96 (1H, m), 1.81 (1H, m), 1.66 (1H, m), 1.56 (1H, m), 1.43 (1H, m), 1.20 (2H, m), 1.05 (3H, d, J=6.8Hz), 0.93 (3H, t, J=7.4Hz)

### Example 47

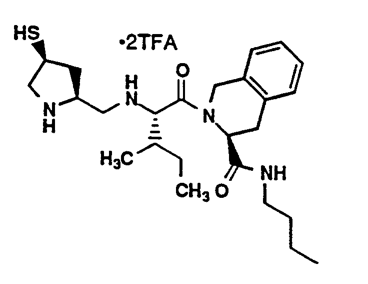

### [2S-[2α(R*),4α]]-N-Butyl-1,2,3,4-tetrahydro-2-[N-[[4-mercapto-2-pyrrolidinyl]-methyl]-L-isoleucyl]-3-isoquinolinecarboxamide, trifluoroacetate

### A. (S)-3-[(Butylamino)carbonyl]-3,4-dihydro-2(1H)-isoquinolinecarboxylic acid 1,1-dimethylethyl ester

(S)-3,4-Dihydro-2,3(1H)-isoquinoline-dicarboxylic acid 2-(1,1-dimethylethyl) ester (1.00 g, 3.61 mmol), 1-ethyl-3-(3-dimethylamino-propyl)carbodiimide·HCl (767 mg, 4.00 mmol) and 1-hydroxybenzotriazole (540 mg, 4.00 mmol) were stirred in DMF (10 mL) at room temperature for 20 minutes. Butylamine (500 mg, 3.97 mmol) was added followed by N,N-diisopropylethylamine (518 mg, 0.70 mL, 4.00 mmol). The reaction mixture was stirred for 16 hours at room temperature, poured into water (100 mL) and extracted with ethyl acetate (100 mL). The organic layer was washed with water (3 X 100 mL), saturated NaHCO₃ (100 mL), water (100 mL) and brine (100 mL), dried (MgSO₄) and concentrated to yield compound A as a clear oil (1.2 g, 99 %), MS: (M+H)⁺ 333⁺.

### B. [2S-[2α(R*),4α]]-N-Butyl-2-[N-[[1-[(1,1-dimethylethoxy)carbonyl]-4-[(triphenylmethyl)thio]-2-pyrrolidinyl)methyl]-L-isoleucyl]-1,2,3,4-tetrahydro-3-isoquinolinecarboxamide trifluoroacetate (1:2)

Compound A (600 mg, 1.80 mmol) was stirred in dimethyl sulfide (0.4 mL) and 4N HCl in dioxane (10 mL) for 40 minutes. The mixture was concentrated, dissolved in methylene chloride (50 mL) and concentrated. This latter procedure was repeated five times to yield the amine as a clear glass. This amine hydrochloride and N,N-diisopropylethylamine (44 mg, 0.060 mL, 0.34 mmol) were dissolved in DMF (3 mL) and added to a stirred solution of compound B of Example 12. This mixture was used to prepare compound B as a clear oil (136 mg, 99%) following the process for preparing compound C of Example 12.
MS: (M+H)⁺ 803⁺.

### C. [2S-[2α(R*),4α]]-N-Butyl-1,2,3,4-tetrahydro-2-[N-[[4-mercapto-2-pyrrolidinyl]-methyl]-L-isoleucyl]-3-isoquinolinecarboxamide, trifluoroacetate

The title compound (59 mg, 50%) was prepred from compound B using a process analogous to the process for preparing the title compound of Example 12.
MS: (M+H)⁺ 461⁺, ¹H-NMR (CD₃OD, 400 MHz) δ (ppm) 7.15 (4H, m , 4.71-4.52 (4H, m), 4.16 (1H, m), 3.88 (1H, m), 3.60 (1H, m), 3.55 (1H, m), 3.41 (1H, m), 3.13-3.02 (5H, m), 2.90 (1H, m), 2.64-2.47 (1H, m), 1.97-1.72 (1H, m), 1.70-1.50 (2H, m), 1.34 (1H, m), 1.26-1.12 (2H, m), 1.06-0.64 (10H, m).

### Example 48

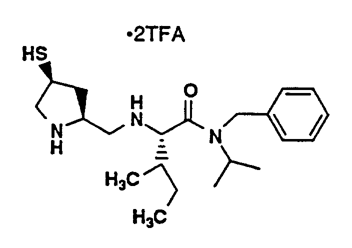

### (2S-cis)-N²-[(4-Mercapto-2-pyrrolidinyl)methyl]-N-(1-methylethyl)-N-(phenylmethyl)-L-isoleucinamide, trifluoroacetate

### A. N-(1-Methylethyl)benzenemethanamine

2-Propanamine (591 mg, 0.85 mL, 10 mmol) and benzaldehyde (1.06 g, 10 mmol) were dissolved in dry methanol (20 mL). Molecular sieves (2 g, 3 angstroms) were added and the mixture were stirred for 30 minutes. Glacial acetic acid (0.4 mL) was added and sodium cyanoborohydride (628 mg, 10 mmol) was added portionwise over 1 hour. The reaction mixture was stirred for 16 hours. The mixture was cooled to 0°C and saturated NaHCO₃ (40 mL) was slowly added. The mixture was concentrated and the product was extracted into ethyl acetate (100 mL). The ethyl acetate layer was washed with water (100 mL) and brine (50 mL), dried (MgSO₄), concentrated and chromatographed (silica gel, 4.1 X 15 cm, 5% methanol, 95% methylene chloride). Fractions containing the desired product were combined and concentrated to yield compound A as a clear oil (500 mg, 33%), MS: (M+H)⁺ 150⁺.

### B. (2S-cis)-N²-[[1-[(1,1-Dimethylethoxy)carbonyl]-4-[(triphenylmethyl)thio]-2-pyrrolidinyl]methyl]-N-(1-methylethyl)-N-(phenylmethyl)-L-isoleucinamide

Compound B (12 mg, 10%) was prepared from compound B of Example 12 with compound A (100 mg, 0.68 mmol) from above followed by N,N-diisopropylethylamine (22 mg, 0.030 mL, 0.17 mmol) using the process for preparing compound C of Example 12.
MS: (M+H)⁺ 720⁺.

### C. (2S-cis)-N²-[(4-Mercapto-2-pyrrolidinyl)methyl]-N-(1-methylethyl]-N-(phenylmethyl)-L-isoleucinamide, trifluoroacetate

The title compound (7 mg, 7%) was prepared from compound B (12 mg, 0.017 mmol) using a process analogous to the process for preparing the title compound of Example 12.
MS: (M+H)⁺ 378⁺, ¹H-NMR (CD₃OD, 400 MHz) δ (ppm) 7.30 (5H, m), 4.52 (1H, m), 4.35 (1H, m), 4.19 (1H, m), 3.90 (1H, m), 3.57 (2H, m), 3.45 (1H, m), 3.06 (1H, m), 2.92 (2H, m), 2.51 (1H, m), 1.75-1.54 (3H, m), 1.20-1.04 (6H m), 1.02 (2H, d, J=6.8Hz), 0.92-0.85 (4H, m), 0.80 (1H, t).

## Claims

1. A compound of the formula 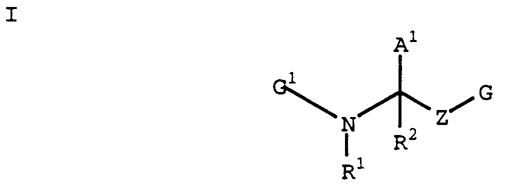 or an enantiomer, diastereomer, pharmaceutically acceptable salt or solvate thereof, wherein:
G is 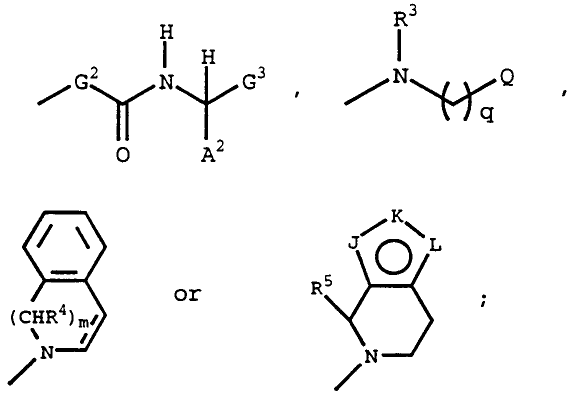 when G is 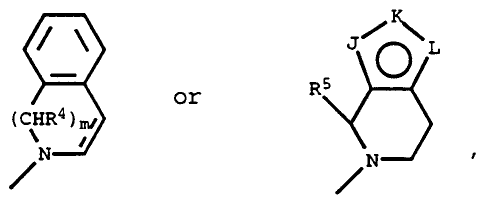 it is optionally substituted, at any available position or positions, with halo, alkyl or substituted alkyl having 1 to 20 carbon atoms, alkoxy, or a combination of these groups;
G¹ is 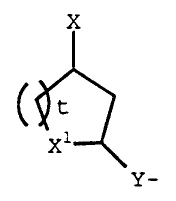 optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, substituted carbamyl, alkoxycarbonyl, phenyl, substituted phenyl, or a combination of these groups;
G² is 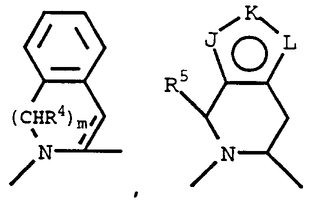 or -NR⁶-CH(Q¹)-;
J, K and L are each, independently, N, NR⁷, O, S or CR⁶ with the provisos that only one of the groups J, K and L can be O or S, and at least one of the groups J or L must be N, NR⁷, O or S to form a fused five-membered heteroring; the bond between J and K or K and L may also form one side of a phenyl ring fused to the fused five-membered heteroring;
Q is alkyl, cycloalkyl, substituted alkyl, aryl, pyrrolyl, furyl, thiophenyl, imidazolyl, oxazolyl, thiazolyl, pyrazolyl, pyrrolidyl or pyridyl;
Q¹, A¹ and A² are each, independently, H, alkyl, substituted alkyl, phenyl or substituted phenyl;
G³ is R⁸, -C(O)OR⁸, -C(O)NR⁸R⁹, -C(O)N(R¹⁰)OR⁸, -C(O)NHSO₂R¹¹ or -CH₂OR¹¹;
X is -SH, -OH or NHR¹²;
X¹ is -NR¹³, -CH₂- or -CH(NHR¹⁴)-;
Y and Z are each, independently, -CH₂- or -C(O)-;
R¹ - R¹⁴ are each, independently, H or alkyl having 1 to 20 carbon atoms;
R³ may also be substituted alkyl or cycloalkyl; R⁴, R⁵ and R¹¹ may also be aryl or aralkyl; R⁷, R⁸, R⁹ and R¹⁰ may also be aralkyl; and R¹², R¹³ and R¹⁴ may also be substituted alkyl or aralkyl;
m is 0 or an integer from 1 to 2;
q is 0 or an integer from 1 to 3;
t is an integer from 1 to 2; and
the dotted line represents an optional double bond.

2. A compound of Claim 1, wherein G¹ is 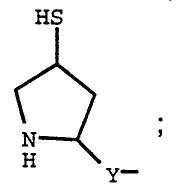 G² is 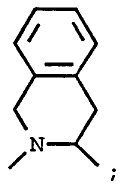 and A¹ and A² are each, independently, H or D-, L- or D,L- -CH₃, -CH(CH₃)₂, -CH₂CH(CH₃)₂, -CH(CH₃)CH₂CH₃, -C(CH₃)₃, -CH₂OH, -CH₂CH₂OH, -CH₂CH₂CH₂OH, -CH(OH)CH₃, 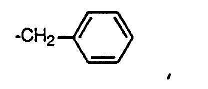 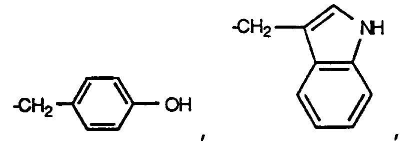 -CH₂CH₂CH₂CH₂NH₂, 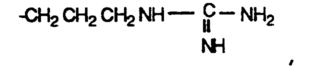 -CH₂C(O)OH, -CH₂CH₂C(O)OH, -CH₂C(O)NH₂, -CH₂CH₂C(O)NH₂, -CH₂CH₂OCH₃, -CH₂CH₂CONR¹⁵R¹⁶, -CH₂SH, -CH₂CH₂SH, -CH₂CH₂SOCH₃, -CH₂CH₂SO₂CH₃ or -CH₂CH₂SCH₃, where R¹⁵ and R¹⁶ are each, independently, hydrogen or alkyl, or R¹⁵ and R¹⁶, taken together, form a 5- to 7-membered, optionally substituted, saturated ring with the N-atom to which they are attached.

3. A compound of Claim 1, wherein G² is -NR⁶CH(Q¹)- and Q¹ is

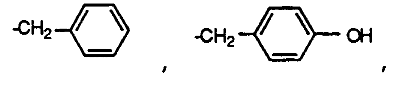

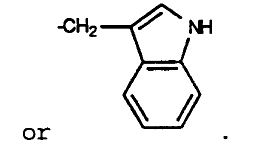

4. A compound of Claim 1, wherein A¹ and A² are each, independently, H or D-, L- or D,L- -CH₃, -CH(CH₃)₂, -CH₂CH(CH₃)₂, -CH(CH₃)CH₂CH₃, -C(CH₃)₃, -CH₂OH, -CH₂CH₂OH,-CH₂CH₂CH₂OH, -CH(OH)CH₃, 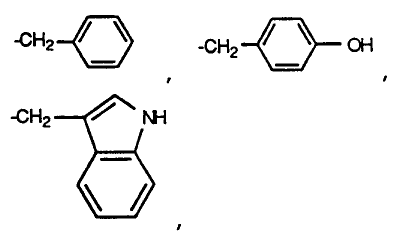 -CH₂CH₂CH₂CH₂NH₂, 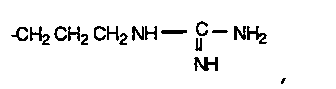 -CH₂C(O)OH, -CH₂CH₂C(O)OH, -CH₂C(O)NH₂, -CH₂CH₂C(O)NH₂, -CH₂CH₂OCH₃, -CH₂CH₂CONR¹⁵R¹⁶, -CH₂SH, -CH₂CH₂SH, -CH₂CH₂SOCH₃, -CH₂CH₂SO₂CH₃ or -CH₂CH₂SCH₃, where R¹⁵ and R¹⁶ are each, independently, hydrogen or alkyl, or R¹⁵ and R¹⁶, taken together, form a 5- to 7-membered, optionally substituted, saturated ring with the N-atom to which they are attached.

5. A compound of Claim 1, wherein A¹ is L--CH₃, -CH(CH₃)₂, -CH₂CH(CH₃)₂, -C(CH₃)₃, 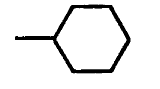 -CH(CH₃)CH₂CH₃, -CH₂OH or -CH(OH)CH₃.

6. A compound of Claim 1, wherein A² is L--CH₂CH₂SCH₃, -CH₂CH₂OH, -CH₂CH₂CH₂OH, -CH₂CH₂OCH₃, -CH₂CH₂CONR¹²R¹³, -CH₂CH₂SH, -CH₂CH₂SOCH₃ or -CH₂CH₂SO₂CH₃.

7. A compound of Claim 1, wherein G² is 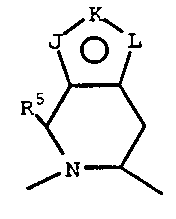 and the fused five-membered optionally substituted heteroring is

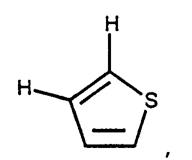

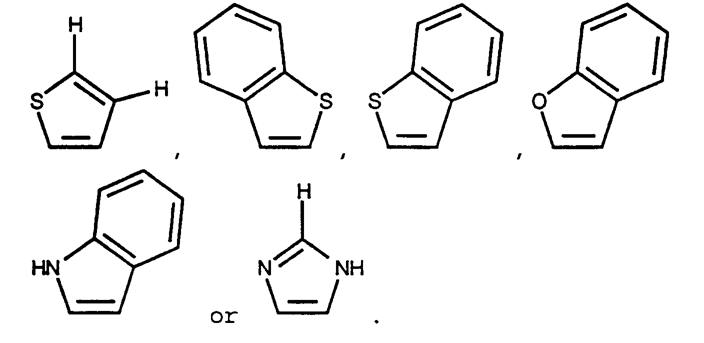

8. A compound of Claim 1, wherein G¹ is

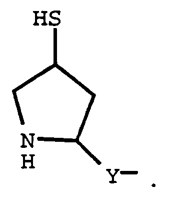

9. A compound of Claim 1, wherein G² is

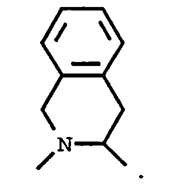

10. A compound of Claim 1, wherein G¹ is 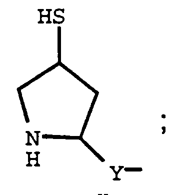 G² is 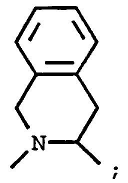 G³ is -C(O)OH, -C(O)OR⁸ or -C(O)NHSO₂R¹¹; A¹ is L--CH₃, -CH(CH₃)₂, -CH₂CH(CH₃)₂, -C(CH₃)₃, 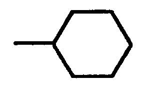 -CH(CH₃)CH₂CH₃, -CH₂OH or -CH(OH)CH₃; A² is L--CH₂CH₂SCH₃, -CH₂CH₂OH, -CH₂CH₂CH₂OH, -CH₂CH₂C(O)NH₂, -CH₂CH₂OCH₃, -CH₂CH₂CONR¹⁵R¹⁶, -CH₂CH₂SH, -CH₂CH₂SOCH₃ or -CH₂CH₂SO₂CH₃; and R¹, R², R³ and R⁴ are H.

11. A compound of Claim 1, selected from the group consisting of:
N-[[(S)-1,2,3,4-Tetrahydro-2-[N-[[(2S-trans)-4-mercapto-2-pyrrolidinyl]methyl]-L-valyl]-3-isoquinolinyl]carbonyl]-L-methionine;
N-[[(S)-1,2,3,4-Tetrahydro-2-[N-[[(2S-cis)-4-mercapto-2-pyrrolidinyl]methyl]-L-valyl]-3-isoquinolinyl]carbonyl]-L-methionine;
N²-[[(S)-1,2,3,4-Tetrahydro-2-[N-[[(2S-*cis*)-4-mercapto-2-pyrrolidinyl]methyl]-3-methyl-L-valyl]-3-isoquinolinyl]carbonyl]-L-glutamine;
2-[2(S)-[[4(S)-Mercapto-2(S)-pyrrolidinyl)-methyl]amino]-3(S)-methyl-1-oxopentyl]-1,2,3,4-tetrahydroisoquinoline;
[S-(R*,R*)]-N-[(2,3-Dichlorophenyl)methyl]-N²-[(4-mercapto-2-pyrrolidinyl)methyl]-L-isoleucinamide;
N²-[[(S)-1,2,3,4-Tetrahydro-2-[N-[[(*cis*)-3-mercaptocyclopentyl]methyl]-3-methyl-L-valyl]-3-isoquinolinyl]carbonyl]-L-glutamine;
[S-(R*,R*)]-N-(2,3-Dihydro-1H-inden-5-yl)-N²-[(4-mercapto-2-pyrrolidinyl)methyl]-L-isoleucinamide;
[S-(R*,R*)]-N-(4-Cyclohexylphenyl)-N²-[(4-mercapto-2-pyrrolidinyl)methyl]-L-isoleucinamide;
N²[[(S)-2-[N-[[(2S-cis)-1-(2-Aminoethyl)-4-mercapto-2-pyrrolidinyl]methyl]-3-methyl-L-valyl]-1,2,3,4-tetrahydro-3-isoquinolinyl]carbonyl]-L-glutamine;
N-2-[[(S)-1,2,3,4-Tetrahydro-2-[N-[(2-cis)-4-hydroxy-2-pyrrolidinyl]methyl]-3-methyl-L-valyl]-3-isoquinolinyl]carbonyl]-L-glutamine;
N²-[[(S)-2-[N-[[(2S-trans)-4-Amino-2-pyrrolidinyl]methyl]-3-methyl-L-valyl]-1,2,3,4-tetrahydro-3-isoquinolinyl]carbonyl]-L-glutamine trifluoroacetate;
(2S-cis)-N²-[(4-mercapto-2-pyrrolidinyl)methyl]-N-[3-(1-methylethoxy)propyl]-L-isoleucinamide;
(2S-cis)-N-(2,3-Dihydro-1H-inden-2-yl)-N²-[(4-mercapto-2-pyrrolidinyl)methyl]-L-isoleucinamide;
(2S-cis)-N²-[(4-Mercapto-2-pyrrolidinyl)methyl]-N-[[4-(trifluoromethyl)phenyl]methyl]-L-isoleucinamide;
(2S-cis)-N-[[4-(Aminosulfonyl)phenyl]methyl]-N²-[(4-mercapto-2-pyrrolidinyl)methyl]-L-isoleucinamide;
(2S-cis)-N²-[(4-Mercapto-2-pyrrolidinyl)methyl]-N-[2-(2-pyridinyl)ethyl]-L-isoleucinamide;
(2S-cis)-N-(3,3-Diphenylpropyl)-N²-[(4-mercapto-2-pyrrolidinyl)methyl]-L-isoleucinamide;
(2S-cis)-N-[4-(1,1-Dimethylethyl)phenyl]-N²-[(4-mercapto-2-pyrrolidinyl)methyl]-L-isoleucinamide;
(2S-cis)-N²-[(4-Mercapto-2-pyrrolidinyl)methyl]-N-(1,2,3,4-tetrahydro-1-naphthalenyl)-L-isoleucinamide;
(2S-cis)-N²-[(4-Mercapto-2-pyrrolidinyl)methyl]-N-[[3-(trifluoromethyl)phenyl]methyl]-L-isoleucinamide;
(2S-cis)-N-Ethyl-N²-[(4-mercapto-2-pyrrolidinyl)methyl]-N-(phenylmethyl)-L-isoleucinamide;
[2S-[2α(R*),4α]]-1,2,3,4-Tetrahydro-2-[2-[[(4-mercapto-2-pyrrolidinyl)methyl]amino]-3,3-dimethyl-1-oxobutyl]isoquinoline;
(2S-cis-)-N²-[(4-Mercapto-2-pyrrolidinyl)methyl]-N-(1-phenylethyl)-L-isoleucinamide;
(2S-cis)-N-(2,3-Dihydro-1H-inden-1-yl)-N²-[(4-mercapto-2-pyrrolidinyl)methyl]-L-isoleucinamide;
(2S-cis)-N-[(2-Ethoxyphenyl)methyl]-N²-[(4-mercapto-2-pyrrolidinyl)methyl]-L-isoleucinamide;
(2S-cis)-N-[2-(4-Hydroxyphenyl)ethyl]-N²-[(4-mercapto-2-pyrrolidinyl)methyl]-L-isoleucinamide;
(2S-cis)-N-[(2,6-Difluorophenyl)methyl]-N²-[(4-mercapto-2-pyrrolidinyl)methyl]-L-isoleucinamide;
N-[[(S)-1,2,3,4-Tetrahydro-2-[N-[[(2S-cis)-4-mercapto-2-pyrrolidinyl]methyl]-L-isoleucyl]-3-isoquinolinyl]carbonyl]glycine, methyl ester;
(2S-cis)-N²-[(4-Mercapto-2-pyrrolidinyl)methyl]-N-(phenylmethyl)-L-isoleucinamide;
[2S-[2α(2R*,3R*),4α]]-1,2,3,4-Tetrahydro-2-[2-[[(4-mercapto-2-pyrrolidinyl)methyl]amino]-3-methylpentyl]isoquinoline;
(2S-cis)-N²-[(4-Mercapto-2-pyrrolidinyl)methyl]-N,N-bis(phenylmehyl)-L-isoleucinamide;
(2S-cis)-N²-[(4-Mercapto-2-pyrrolidinyl)methyl]-N-(1-methylbutyl)-L-isoleucineamide;
(2S-cis)-N-(Cyclohexylmethyl)-N²-[(4-mercapto-2-pyrrolidinyl)methyl]-L-isoleucinamide;
(2S-cis)-N-(1,3-Dimethylbutyl)-N²-[(4-mercapto-2-pyrrolidinyl)methyl]-L-isoleucinamide;
(2S-cis)-N-Hexyl-N²-[(4-mercapto-2-pyrrolidinyl)methyl]-N-methyl-L-isoleucinamide;
(2S-cis)-N²-[(4-Mercapto-2-pyrrolidinyl)methyl]-N-(2-methylpropyl)-L-isoleucinamide;
(2S-cis)-N²-[(4-Mercapto-2-pyrrolidinyl)methyl]-N-(2-phenylethyl)-L-isoleucinamide;
(2S-cis)-N-[(3-Hydroxyphenyl)methyl]-N²-[(4-mercapto-2-pyrrolidinyl)methyl]-L-isoleucinamide;
(2S-cis)-N-[(2,3-Dimethoxyphenyl)methyl]-N²-[(4-mercapto-2-pyrrolidinyl)methyl]-L-isoleucinamde;
(2S-cis)-N²-[(4-Mercapto-2-pyrrolidinyl)methyl]-N,N-bis(2-methylpropyl)-L-isoleucinamide;
(2S-cis)-N-[(4-Hydroxyphenyl)methyl]-N²[(4-mercapto-2-pyrrolidinyl)methyl]-L-isoleucinamide;
(2S-cis)-N,N-Bis[(2-ethoxyphenyl)methyl]-N²-[(4-mercapto-2-pyrrolidinyl)methyl]-L-isoleucinamide;
(2S-cis)-N,N-Bis[(2,3-dimethoxyphenyl)methyl]-N²-[(4-mercapto-2-pyrrolidinyl)methyl]-L-isoleucinamide;
(2S-cis)-N-[(2-Hydroxyphenyl)methyl]-N²-[(4-mercapto-2-pyrrolidinyl)methyl]-L-isoleucinamide;
cis-N-[(2,3-Dichlorophenyl)methyl]-N²-[(3-mercaptocyclopentyl)methyl]-L-isoleucinamide;
cis-N²-[(3-Mercaptocyclopentyl)methyl]-N,N-bis(phenylmethyl)-L-isoleucinamide;
[2S-[2α(R*),4α]]-N-Butyl-1,2,3,4-tetrahydro-2-[N-[[4-mercapto-2-pyrrolidinyl]-methyl]-L-isoleucyl]-3-isoquinolinecarboxamide; and
(2S-cis)-N²-[(4-Mercapto-2-pyrrolidinyl)methyl]-N-(1-methylethyl)-N-(phenylmethyl)-L-isoleucinamide.

12. A compound as defined in any one of claims 1-11 for use as an pharmaceutically active ingredient.

13. Use of a compound as defined in any one of claims 1-11 for the preparation of a pharmaceutical composition for inhibiting farnesyl protein transferase.

14. Use of a compound as defined in any one of claims 1-11 for the preparation of a pharmaceutical composition for inhibiting prenyl transferase.

15. Use of a compound as defined in any one of claims 1-11 for the preparation of a pharmaceutical composition for inhibiting tumors.

16. Use of a compound as defined in any one of claims 1-11 for the preparation of a pharmaceutical composition for treating diseases associated with signal transduction pathways operating through Ras.

17. Use of a compound as defined in any one of claims 1-11 for the preparation of a pharmaceutical composition for treating diseases associated with proteins that are post-translationally modified by the enzyme farnesyl protein transferase.

18. Use of a compound as defined in any one of claims 1-11 for the preparation of a pharmaceutical composition for treating diseases associated with proteins that are post-translationally modified by the enzymes geranylgeranyl protein transferase.

19. A compound of the formula 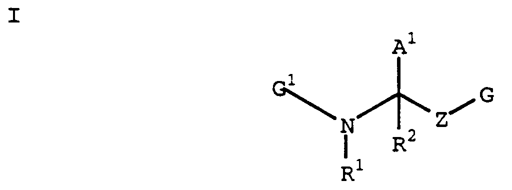 or an enantiomer, diastereomer, pharmaceutically acceptable salt or solvate thereof, wherein:
G is 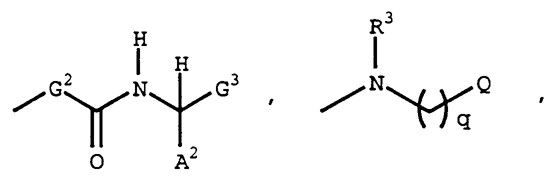 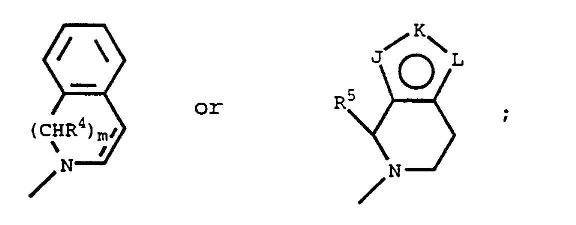 when G is 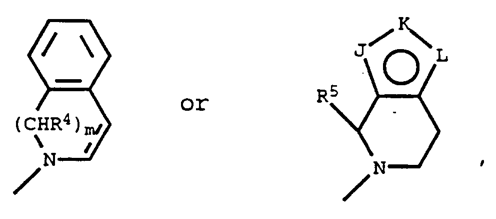 it is optionally substituted, at any available position or positions, with halo, alkyl or substituted alkyl having 1 to 20 carbon atoms, alkoxy, or a combination of these groups;
G¹ is 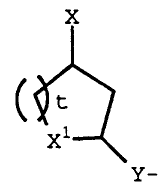 optionally substituted, at any of the available position or positions on the ring, with halo, alkyl or substituted alkyl having 1 to 20 carbon atoms, alkoxy, aryl, aralkyl, hydroxy, alkanoyl, alkanoyloxy, amino, alkylamino, dialkylamino, alkanoylamino, thiol, alkylthio, alkylthiono, alkylsulfonyl, sulfonamido, nitro, cyano, carboxy, carbamyl, N-hydroxycarbamyl, N-alkylcarbamyl, N-dialkylcarbamyl, alkoxycarbonyl, phenyl, substituted phenyl, or a combination of these groups;
G² is 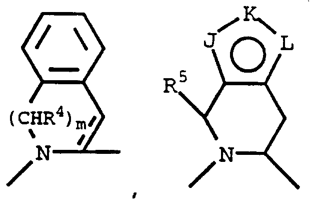 or -NR⁶-CH(Q¹)-;
J, K and L are each, independently, N, NR⁷, O, S or CR⁶ with the provisos that only one of the groups J, K and L can be O or S, and at least one of the groups J or L must be N, NR⁷, O or S to form a fused five-membered heteroring; the bond between J and K or K and L may also form one side of a phenyl ring fused to the fused five-membered heteroring;
Q is aryl;
Q¹, A¹ and A² are each, independently, H, alkyl, substituted alkyl, phenyl or substituted phenyl;
G³ is R⁸, -C(O)OR⁸, -C(O)NR⁸R⁹, -C(O)N(R¹⁰)OR⁸, -C(O)NHSO₂R¹¹ or -CH₂OR¹¹;
X is -SH, -OH or NHR¹²;
X¹ is -NR¹³-, -CH₂- or -CH(NHR¹⁴)-;
Y and Z are each, independently, -CH₂- or -C(O)-;
R¹ - R¹⁴ are each, independently, H or alkyl having 1 to 20 carbon atoms;
R⁴, R⁵ and R¹¹ may also be aryl or aralkyl; R⁷, R⁸, R⁹ and R¹⁰ may also be aralkyl; and R¹², R¹³ and R¹⁴ may also be substituted alkyl or aralkyl;
m is 0 or an integer from 1 to 2;
q is 0 or an integer from 1 to 3;
t is an integer from 1 to 2; and
the dotted line represents an optional double bond.
